# EUROPEAN PATENT APPLICATION

(11) **EP 2 199 284 A1**
(43) Date of publication of application: **23.06.2010**
(21) Application number: 08829342.8
(22) Date of filing: 05.09.2008
(51) Int. Cl.: C07D 215/38, A61K 31/4704, A61P 3/10, A61P 7/04, A61P 9/00, A61P 9/04, A61P 11/00, A61P 17/00, A61P 19/00, A61P 19/08, A61P 25/00, A61P 25/28, A61P 29/00, A61P 31/06, A61P 31/08, A61P 35/00, A61P 37/00, A61P 43/00

(54) **PHARMACEUTICAL COMPOSITION FOR INHIBITING ACCUMULATION OF AMYLOID- BETA PROTEIN**

(30) Priority: 06.09.2007 JP 2007231195
(71) Applicant: Sumitomo Chemical Company, Limited, Tokyo 104-8260 (JP)
(72) Inventor: MOTONAGA, Kozo, Ibaraki-shi Osaka 567-0841 (JP); SAITO, Koichi, Takarazuka-shi Hyogo 665-0847 (JP)
(74) Representative: Duckworth, Timothy John
(86) International application number: PCT/JP2008/066483
(87) International publication number: WO 2009/031705

(57) **Abstract**

The present invention provides: a pharmaceutical composition for inhibiting amyloid-β protein accumulation comprising a compound represented by the formulas including (I) or a pharmaceutically acceptable salt thereof; a method for inhibiting amyloid-β protein accumulation, comprising a step of administering an effective amount of the compound represented by the formulas including (I) or a pharmaceutically acceptable salt thereof to a mammal which may be diagnosed with an amyloid-β protein-related disease; and so on.

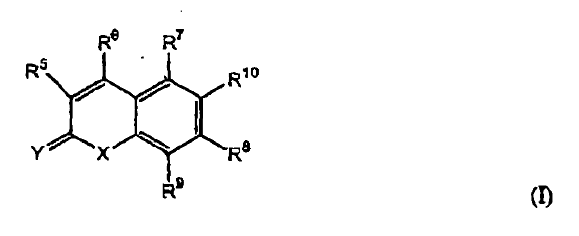

## Description

### Technical Field

The present invention relates to a pharmaceutical composition that inhibits amyloid-β protein accumulation, and so on.

### Background Art

An amyloid-β protein (hereinafter, sometimes referred to as Aβ) is an insoluble protein consisting of approximately 40 amino acid residues, and is generated by cleavage of an amyloid-β precursor protein by β-secretase and γ-secretase. The β-secretase has been identified as an aspartic protease (Neuron, 27, 419-422, 2000), and it has been revealed that a gene responsible for familial Alzheimer's disease (FAD) and presenilin or a complex containing presenilin are involved in expression of a γ-secretase activity (Neuron, 27, 419-422, 2000).

It has been known that accumulation of the Aβ in each organ and tissue in a living body causes functional impairment in the organ and tissue. Diseases in which each organ and tissue is impaired by accumulation of the Aβ are collectively called Aβ-related diseases (amyloidosis).

### Disclosure of the Invention

Based on the foregoing findings, a compound that inhibits Aβ accumulation is useful as a medicine capable of treating or preventing an Aβ-related disease. An object of the present invention is to provide a compound that inhibits Aβ accumulation.

The present invention includes the following inventions.

### [Invention 1]

1. A pharmaceutical composition for inhibiting amylold-β protein accumulation comprising a compound represented by the following formulas: 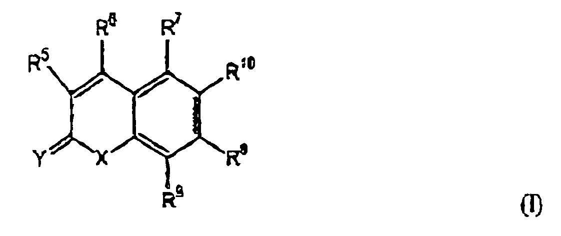 or 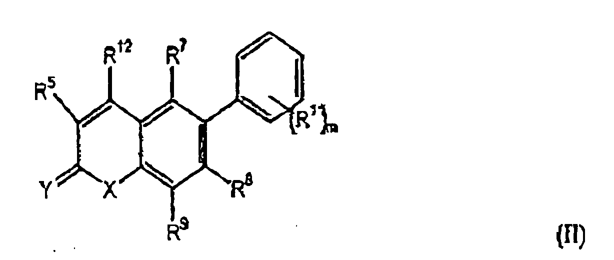 or 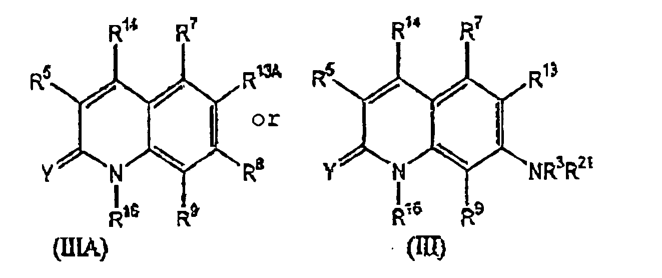 or 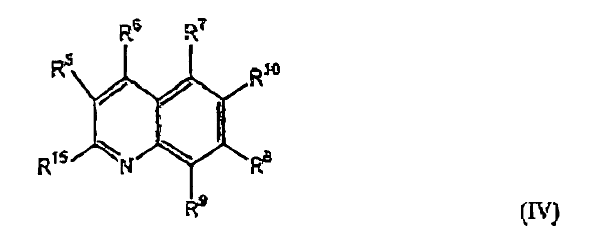 or 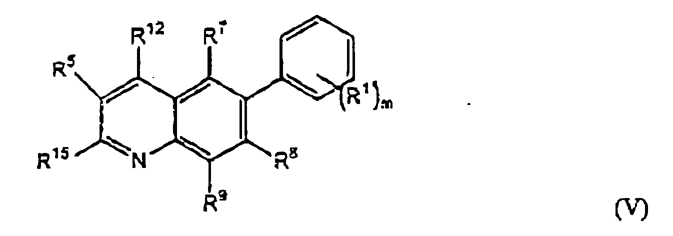 or 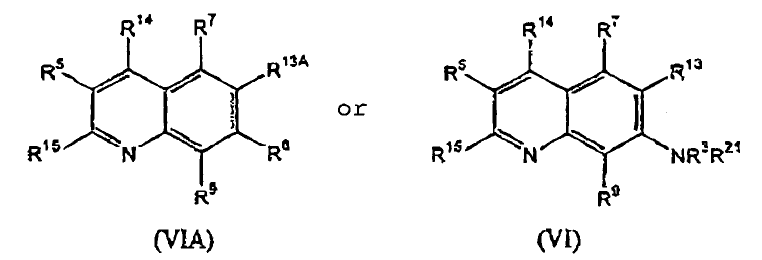 or 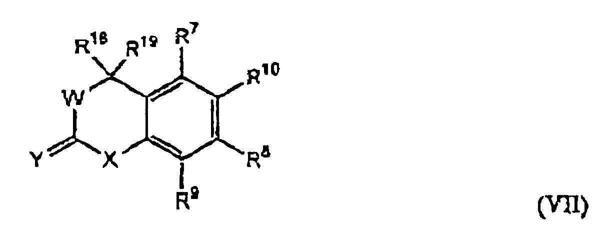 or 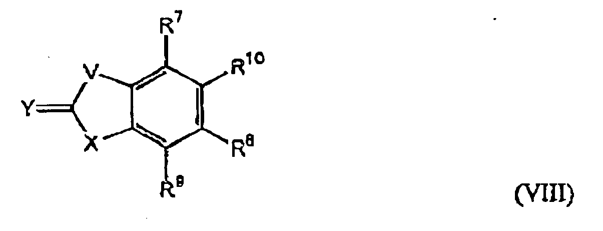 or 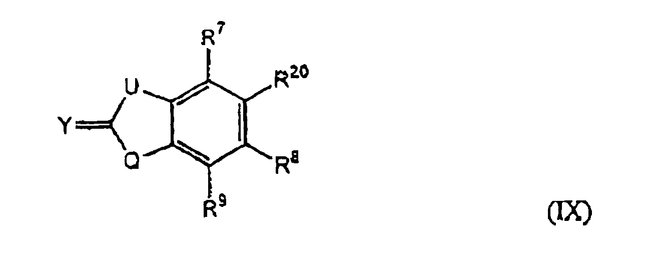 or 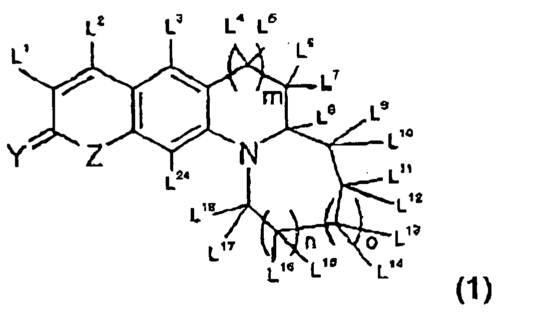 or 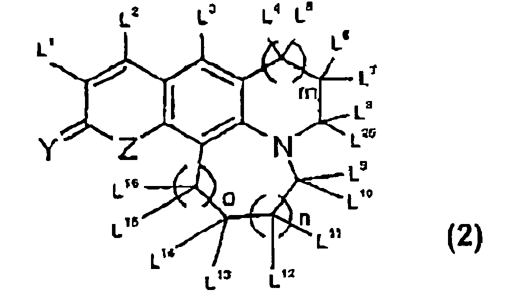 or 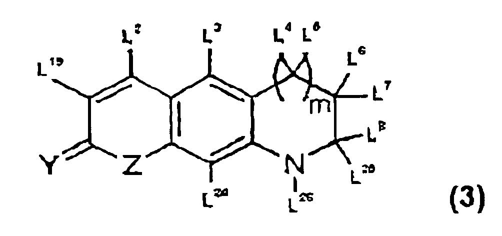 or 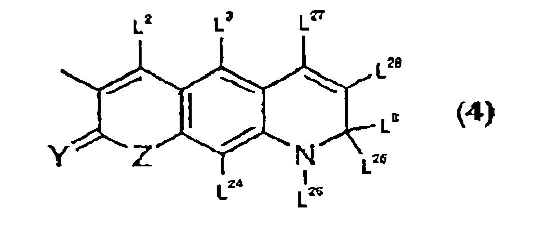 or 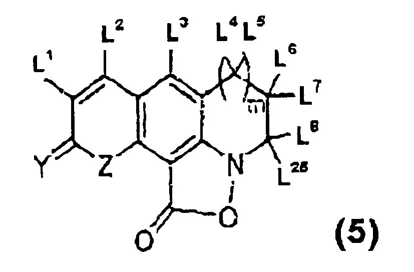 or 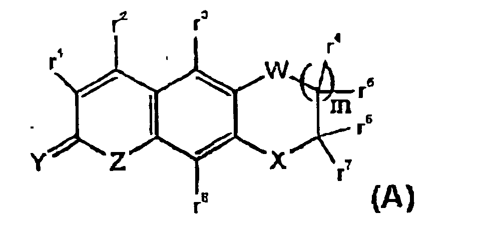 or 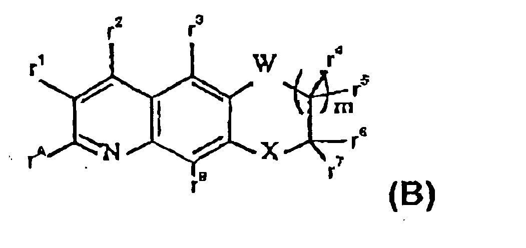 or 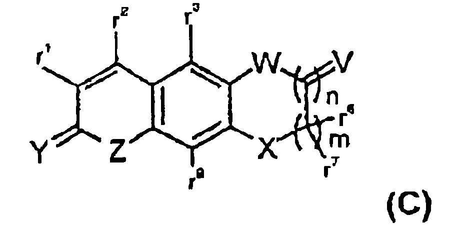 or 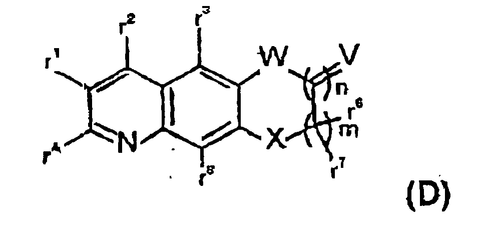 or 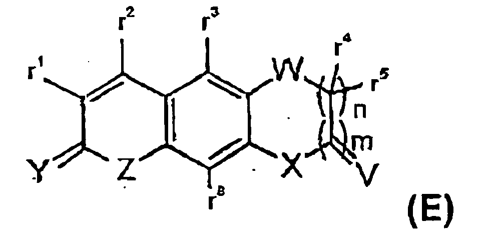 or 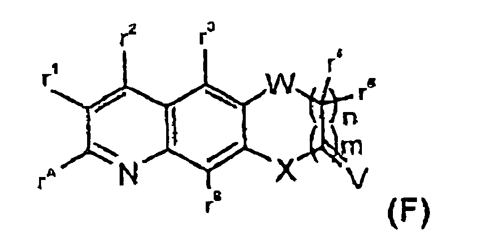 or 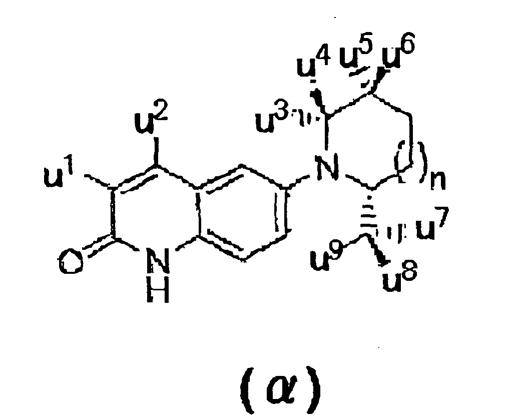
or a pharmaceutically acceptable salt thereof,
wherein, in the formulas (I) to (IX),
R¹ and R² are each independently selected from the group of COR³, CSR³, SO₂R³, NO, NR³R⁴, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, (CH₂)ₙR^{3A}, aryl, and heteroaryl, wherein the alkyl, alkenyl, alkynyl, haloalkyl, haloalkenyl, haloalkynyl, heteroalkyl, heteroalkenyl, heteroalkynyl, aryl, and heteroaryl are optionally substituted with F, Cl, Br, I, OR³, NR³R⁴, CN, NO₂, SR³, SOR³, SO₂R³, C₁-C₄ alkyl, C₁-C₄ haloalkyl or C₁-C₄ heteroalkyl; or
R¹ and R² are taken together to form a three- to nine-membered alkyl, alkenyl, heteroalkyl, or heteroalkenyl ring, wherein the alkyl, alkenyl, heteroalkyl, or heteroalkenyl ring are optionally substituted with F, Cl, Br, I, OR³, NR³R⁴, C₁-C₄ alkyl, C₁-C₄ haloalkyl or C₁-C₄ heteroalkyl; or R¹ and R² are taken together to form one of: 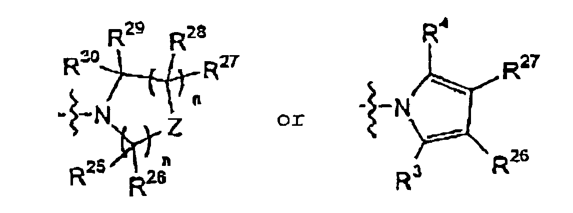 R³ and R⁴ are each independently selected from the group of hydrogen, C₁-C₈ alkyl, C₂-C₈ alkenyl, C₂-C₈ alkynyl, C₁-C₈ haloalkyl, C₁-C₈ heteroalkyl, heteroaryl, and aryl, wherein the alkyl, alkenyl, alkynyl, haloalkyl, heteroalkyl, heteroaryl, and aryl are optionally substituted with halogen, C₁-C₄ alkyl, C₁-C₄ haloalkyl or C₁-C₄ heteroalkyl;
R^{3A} is aryl or heteroaryl, wherein the aryl and heteroaryl is optionally substituted with halogen, CN, NO₂, C₁-C₄ alkyl, C₁-C₄ haloalkyl or C₁-C₄ heteroalkyl;
R⁵ is selected from the group of hydrogen, F, Cl, Br, I, OR³, SR³, NR³R⁴, C₁-C₄ alkyl, C₁-C₄ haloalkyl or C₁-C₄ heteroalkyl;
R⁶ is selected from the group of F, Cl, Br, I, CH₃, CF₃, CHF₂, CFH₂, CN, CF₂Cl, CF₂OR³, OR³, SR³, SOR³, SO₂R³, CO₂R³, NR³R⁴, C₁-C₄ alkyl, C₂-C₄ alkenyl, C₂-C₄ alkynyl, C₁-C₄ haloalkyl, C₂-C₄ haloalkenyl, C₂-C₄ haloalkynyl, C₁-C₄ heteroalkyl, C₂-C₄ heteroalkenyl, and C₂-C₄ heteroalkynyl, wherein the alkyl, alkenyl, alkynyl, haloalkyl, haloalkenyl, haloalkynyl, heteroalkyl, heteroalkenyl, and heteroalkynyl are optionally substituted with F, Cl, Br, C₁-C₄ alkyl, C₁-C₄ haloalkyl or C₁-C₄ heteroalkyl;
R⁷ and R⁸ are each independently selected from the group of hydrogen, F, Cl, Br, I, CN, OR³, NR³R⁴, NR³CR³R⁹CONR³R⁴, Cₙ(R³)₂ₙOR³, SR³, SOR³, SO₂R³, NR³COR⁴, C₁-C₈ alkyl, C₁-C₈ haloalkyl, and C₁-C₈ heteroalkyl ;
R⁹ is selected from the group of hydrogen, F, Br, Cl, I, OR³, NR³R⁴, SR³, SOR³, SO₂R³ C₁-C₄ alkyl, C₁-C₄ haloalkyl and C₁-C₄ heteroalkyl;,
R¹⁰ is selected from the group represented by the following formulas: 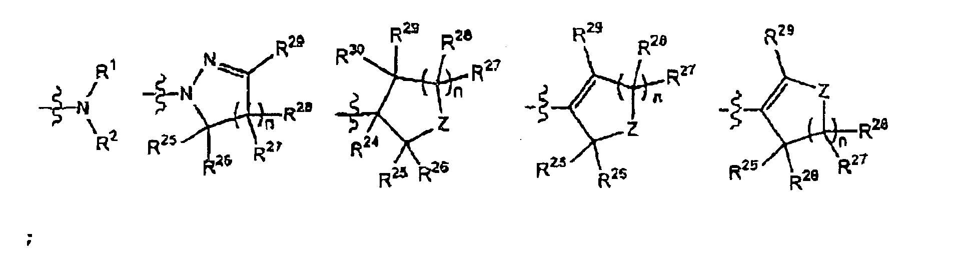 R¹¹ is selected from the group of F, Br, Cl, I, C₁-C₆ alkyl, C₁-C₆ haloalkyl, C₁-C₆ heteroalkyl, NO₂, CN, CF₃, OR³, NR³R⁴, SR³, SOR³, and SO₂R³;
R¹² is selected from the group of F, Br, Cl, I, CN, OR³, SR³, SOR³, SO₂R³, NR³R⁴, and C₁-C₄ haloalkyl;
R¹³ is selected from the group of hydrogen, F, Cl, Br, I, CN, OR³, NR³R⁴, COR³, CO₂R³, SR³, SOR³, SO₂R³, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, and (CH₂)ₙR^{3A}, wherein the alkyl, alkenyl, alkynyl, haloalkyl, haloalkenyl, haloalkynyl, heteroalkyl, heteroalkenyl and heteroalkynyl are optionally substituted with F, Cl, Br, I, CN, NO₂, NR³R⁴, SR³, C₁-C₄ alkyl, C₁-C₄ haloalkyl or C₁-C₄ heteroalkyl;
R^{13A} is NHR¹ or heteroaryl, wherein the heteroaryl is optionally substituted with F, Cl, Br, I, CN, NO₂, NR³R⁴, SR³, SOR³, SO₂R³, C₁-C₄ alkyl, C₁-C₄ haloalkyl or C₁-C₄ heteroalkyl;
R¹⁴ is selected from the group of F, Br, Cl, I, CF₃, CHF₂, CH₂F, CF₂Cl, and CF₂OR³
R¹⁵ is selected from the group of F, Br, Cl, I, CN, C₁-C₄ alkyl, C₁-C₄ haloalkyl, C₁-C₄ heteroalkyl, OR¹⁶, NR¹⁶R⁴, SR¹⁶, CH₂R¹⁶, COR³, CO₂R³, CONR³R⁴, SOR³, and SO₂R³
R¹⁶ is selected from the group of hydrogen, C₁-C₈ alkyl, C₁-C₈ haloalkyl, C₁-C₈ heteroalkyl, CH₃R^{3A}, aryl, heteroaryl ,COR¹⁷, CO₂R¹⁷, and CONR¹⁷R¹⁷;
R¹⁷ is selected from the group of hydrogen, C₁-C₄ alkyl, C₁-C₄ haloalkyl and C₁-C₄ heteroalkyl;
R¹⁸ and R¹⁹ are each independently selected from the group of hydrogen, C₁-C₆ alkyl, C₁-C₆ haloalkyl and C₁-C₆ heteroalkyl; or
R¹⁸ and R¹⁹ are taken together to form a three- to seven-membered ring;
R²⁰ is aryl or heteroaryl, wherein the aryl or heteroaryl are optionally substituted with F, Cl, Br, CN, OR³, SR³, SOR³, SO₂R³, NO₂, NR³R³, C₁-C₄ alkyl, C₁-C₄ haloalkyl or C₁-C₄ heteroalkyl;
R²¹ is selected from the group of CR³R⁴CONR³R⁴, Cₙ(R³)₂ₙOR³, SOR³, SO₂R³, C₂-C₈ alkyl, C₂-C₈ haloalkyl, and C₂-C₈ heteroalkyl;
R²² and R²³ are each independently selected from the group of hydrogen, C₁-C₆ alkyl, C₁-C₆ haloalkyl and C₁-C₆ heteroalkyl; or
R²² and R²³ are taken together to form a three- to seven-membered ring;
R²⁴ is hydrogen or OR³;
R²⁵ through R³⁰ are each independently selected from the group of hydrogen, F, Cl, Br, I, OR³, NR³R⁴, SR³, SOR³, SO₂R³, C₁-C₆ alkyl, C₁-C₆ haloalkyl, C₁-C₆ heteroalkyl, C₂-C₆ alkynyl and C₂-C₆ alkenyl, wherein the alkyl, haloalkyl, heteroalkyl, alkynyl, and alkenyl are optionally substituted with F, Cl, Br, I, OR³, NR³R⁴, C₁-C₄ alkyl, C₁-C₄ haloalkyl, C₁-C₄ heteroalkyl, aryl or heteroaryl, and wherein the aryl and heteroaryl are optionally substituted with F, Cl, Br, I, CN, NO₂, OH, OCH₃, CF₃ or C₁-C₆ alkyl; or
Any two of R²⁵ R²⁶, R²⁷, R²⁸, R²⁹ and R³⁰ are taken together to form a three to seven-membered alkyl or alkenyl or heteroalkyl ring; or
any four of R²⁵ R²⁶, R²⁷, R²⁸, R²⁹ and R³⁰ are taken together to form a fused aromatic ring;
Q is O or S;
U is selected from the group ot V, OCR²²R²³, SCR²²R²³, NR³CR²²R²³, and CR³R⁴CR²²R²³;
V is selected from the group of 0, NR³, CR²²R²³, CR³R⁴O, and CR³R⁴S;
W is selected from the group of O, S, NR³, and CR³R⁴;
X is selected from the group of O, S and NR¹⁶;
Y is selected from the group of O, S, NR³, NOR³ and CR³R⁴;
Z is selected from the group of O, S, NR³, C=O, and CR²⁵R²⁶; or
Z is two hydrogens;
n is 1, 2 or 3; and
m is 1 to 5,
and wherein, in the formulas (1) to (5),
L¹ is hydrogen, F, Cl, Br, I, NO₂, OL²⁰, NL²¹L²², SL²⁰, a C₁-C₄ alkyl or perhaloalkyl, or is an optionally substituted allyl, arylmethyl, alkynyl, alkenyl, aryl or heteroaryl, wherein L²¹ is hydrogen, a C₁-C₆ alkyl or perfluoroalkyl, aryl, heteroaryl, optionally substituted allyl or arylmethyl, SO₂L²³ or S(O)L²³, wherein L²³ is hydrogen, a C₁-C₆ alkyl or perfluoroalkyl, aryl, heteroaryl, or optionally substituted allyl or arylmethyl, L²⁰ is hydrogen, a C₁-C₆ alkyl or perfluoroalkyl, aryl, heteroaryl, optionally substituted allyl or arylmethyl, and L²² is hydrogen, a C₁-C₄ alkyl or perfluoroalkyl, aryl, heteroaryl, optionally substituted allyl or arylmethyl, OL²⁰ or NHL²¹;
L² is hydrogen, F, Br, Cl, a C₁-C₄ alkyl or perhaloalkyl, aryl, heteroaryl, CF₃, CF₂H, CFH₂, CF₂OL²⁰, CH₂OL²⁰, or OL²⁰, wherein L²⁰ has the same definition given above;
L³ is hydrogen, a C₁-C₄ alkyl, F, Cl, Br, I, OL²⁰, NL²¹L²² or SL²⁰, wherein L²⁰ through L²² have the definition given above;
L⁴ and L⁵ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁴ and L⁵ taken together can form a three- to seven-membered ring optionally substituted with hydrogen, F, Cl, Br, OL²⁰ or NL²¹L²², wherein L²⁰ through L²² have the definitions given above;
L⁶ and L⁷ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁶ and L⁷ taken together can form a three- to seven-membered ring optionally substituted with hydrogen, F, Cl, Br, OL²⁰ or NL²¹L²², wherein L²⁰ through L²² have the definitions given above;
L⁸ is hydrogen, a C₁-C₁₂ alkyl or perfluoroalkyl, hydroxymethyl, aryl, heteroaryl or optionally substituted allyl, arylmethyl, alkynyl or alkenyl;
L⁹ through L¹⁸ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or any two of L⁹ through L¹⁸ taken together can form a three- to seven-membered ring optionally substituted with hydrogen, F, Cl, Br, OL²⁰ or NL²¹L²²,wherein L²⁰ through L²² have the definitions given above;
L¹⁹ is F, NO₂ or SL²⁰, wherein L²⁰ has the definition given above;
L²⁴ is hydrogen, a C₁-C₄ alkyl, F, Cl, Br, I, NO₂, OL²⁰, NL²¹L²² or SL²⁰, wherein L²⁰ through L²² have the definitions given above;
L²⁵ is hydrogen, a C₁-C₁₂ alkyl or perfluoroalkyl, hydroxymethyl, aryl, heteroaryl or optionally substituted allyl, arylmethyl, alkynyl or alkenyl, or L²⁵ and L⁸ taken together can form a three- to seven-membered ring optionally substituted with hydrogen, F, Cl, Br, OL²⁰ or NL²¹L²², wherein L²⁰ through L²² have the definitions given above;
L²⁶ is hydrogen, a C₁-C₆ alkyl or perfluoroalkyl, NO₂, OL²⁰, C(O)L²⁰, C(O)OL²⁰, C(O)NL²¹, L²², or an optionally substituted aryl, heteroaryl, allyl or arylmethyl, wherein L²⁰ through L²² have the definitions given above;
L²⁷ and L²⁸ each independently are hydrogen, F, Cl, Br, I, OL²⁰, NL²¹L²², a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L²¹ and L²⁰ taken together can form a three- to seven-membered ring optionally substituted with hydrogen, F, Cl, Br, OL²⁰ or NL²¹L²², wherein L²⁰ through L²² have the definitions given above;
m is 0 or 1;
n is 0 or 1;
o is 0 or 1;
Y is 0 or S;
Z is O, S, NH, NL²² or NCOL²², wherein L²² has the definition given above; and
any two of L⁴ through L⁸, L²⁵ and L²⁸ taken together can form a three- to seven-membered ring optionally substituted with hydrogen, F, Cl, Br, OL²⁰ or NL²¹L²², wherein L²⁰ through L²² have the definitions given above,
and wherein, in the formulas (A) to (F),
r¹ is hydrogen, F, Cl, Br, I, NO₂, Or⁹, Nr¹⁰r¹¹, S(O)mr⁹, C₁-C₈ alkyl, C₃-C₈ cycloalkyl, C₁-C₈ heteroalkyl, C₁-C₈ haloalkyl, C₁-C₈ aryl, C₁-C₈ arylalkyl, C₁-C₈ heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r² is hydrogen, F, Cl, Br, I, CF₃, CF₂Cl, CF₂H, CFH₂, CF₂Or⁹, CH₂Or⁹, Or⁹, S(O)mr⁹, Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r³ is hydrogen, F, Cl, Br, I, Or⁹, S(O)mr⁹, Nr¹⁰r¹¹, or C₁-C₆ alkyl, C₁-C₆ heteroalkyl, or C₁-C₆ haloalkyl, wherein the alkyl, heteroalkyl, and haloalkyl groups may be optionally substituted;
r⁹ and r⁵ each independently are hydrogen, Or⁹, S(O)mr⁹, Nr¹⁰r¹¹, C(Y)Or¹¹, C(Y)Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁴ and r⁵ taken together can form a saturated or unsaturated three- to seven-membered ring that may be optionally substituted;
r⁶ and r⁷ each independently are hydrogen, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁶ and r⁷ taken together can form a saturated or unsaturated three- to seven-membered ring that may be optionally substituted; or
r⁶ and r⁵ taken together can form a saturated or unsaturated three- to seven-membered ring that may be optionally substituted;
r⁸ is hydrogen, C₁-C₄ alkyl, C₁-C₄ heteroalkyl, C₁-C₄ haloalkyl, F, Cl, Br, I, NO₂, Or⁹, Nr¹⁰r¹¹ or S(O)mr⁹, wherein the alkyl, heteroalkyl, and haloalkyl groups may be optionally substituted;
r⁹ is hydrogen, C₁-C₆ alkyl, C₁-C₆ heteroalkyl, C₁-C₆ haloalkyl, aryl, heteroaryl, arylalkyl, C₂-C₄ alkynyl or C₂-C₈ alkenyl, wherein the alkyl, heteroalkyl, haloalkyl, aryl, arylalkyl, heteroaryl, alkenyl and alkynyl groups may be optionally substituted;
r¹⁰ is hydrogen, C₁-C₆ alkyl, C₁-C₆ heteroalkyl, C₁-C₆ haloalkyl, C (Y) r¹², C(Y)Or¹², aryl, heteroaryl, C₂-C₄ alkynyl, C₂-C₄ alkenyl, arylalkyl, SO₂r¹² or S(O)r¹², wherein the alkyl, heteroalkyl, haloalkyl, aryl, arylalkyl, heteroaryl, alkynyl, and alkenyl groups may be optionally substituted;
r¹¹ is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, arylalkyl, heteroaryl, alkynyl and alkenyl groups may be optionally substituted;
r¹² is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, heteroaryl, arylalkyl, alkynyl and alkenyl groups may be optionally substituted;
r¹³ is hydrogen, C₁-C₈ alkyl, C₃-C₈ cycloalkyl, C₁-C₈ heteroalkyl, C₁-C₈ haloalkyl, aryl, heteroaryl, arylalkyl, heteroarylalkyl, C₂-C₄ alkynyl, or C₂-C₈ alkenyl, wherein the alkyl, cycloalkyl, heteroalkyl, haloalkyl, aryl, arylalkyl, heteroaryl, heteroarylalkyl, alkynyl and alkenyl groups may be optionally substituted; or
r¹³ and r⁴ taken together can form a saturated or unsaturated three- to seven-membered ring that may be optionally substituted;
r¹³ and any two of r⁴ through r⁷ taken together can form a saturated or unsaturated three- to seven-membered ring that may be optionally substituted;
r¹⁴ and r¹⁵ each independently are hydrogen, C₁-C₈ alkyl, C₃-C₈ cycloalkyl, C₁-C₈ heteroalkyl, C₁-C₈ haloalkyl, aryl, heteroaryl, arylalkyl, C₂-C₈ alkynyl or C₂-C₈ alkenyl, wherein the alkyl, cycloalkyl, heteroalkyl, haloalkyl, aryl, heteroaryl, arylalkyl, alkynyl and alkenyl groups may be optionally substituted;
r^{A} is hydrogen, F, Br, Cl, I, CN, C₁-C₆ alkyl, C₁-C₆ haloalkyl, C₁-C₆ heteroalkyl, Or¹⁶, Nr¹⁶r¹¹, Sr¹⁶, CH₂r¹⁶, COr¹⁷, CO₂r¹⁷, CONr¹⁷r¹¹, SOr¹⁷ or SO₂r¹⁷, wherein the alkyl, haloalkyl, and heteroalkyl groups may be optionally substituted;
r¹⁶ is hydrogen, C₁-C₈ alkyl, C₁-C₈ haloalkyl, C₁-C8 heteroalkyl, COr¹⁷, CO₂r¹⁷, CONr¹⁷r¹⁷, C₂-C₈ alkynyl, C₂-C₈ alkenyl, aryl or heteroaryl, wherein the alkyl, heteroalkyl, haloalkyl, aryl, heteroaryl, alkynyl and alkenyl groups may be optionally substituted;
r¹⁷ is hydrogen, C₁-C₄ alkyl, C₁-C₄ haloalkyl or C₁-C₄ heteroalkyl, wherein the alkyl, heteroalkyl, and haloalkyl groups may be optionally substituted;
m is 0, 1 or 2;
n is 1 or 2;
V is 0, S or Cr¹⁴r¹⁵;
W is 0, S, NH, Nr¹³, NC(Y)r¹¹, or NSO₂r¹¹;
X and Z each independently are 0, S(O)m, NH, Nr¹¹, NC(Y)r¹¹, NSO₂r¹² or NS(O)r¹²; and
Y is 0 or S, and
wherein, in the formula (α),
u¹ is hydrogen, F, Cl, or C₁-C₃ aliphatic;
u² is selected from the group of hydrogen, F, Cl, Br, C₁-C₄ aliphatic, C₁-C₄ haloaliphatic, and C₁-C₄ heteroaliphatic;
u³ and u⁴ each independently is selected from the group of hydrogen, C₁-C₄ aliphatic, C₁-C₄ haloaliphatic, C₁-C₄ heteroaliphatic, optionally substituted aryl and heteroaryl;
u⁵ and u⁶ each independently is selected from the group of hydrogen, F, Cl, Ou¹⁰, C₁-C₄ aliphatic, C₁-C₄ haloaliphatic, and C₁-C₄ heteroaliphatic;
u⁷ and u⁸ each independently is selected from the group of hydrogen, F, Cl, C₁-C₄ aliphatic, C₁-C₄ haloaliphatic, and C₁-C₄ heteroaliphatic; or
u⁷ and u⁸ taken together form a carbonyl group;
u⁹ is selected from the group of halogen, ou¹⁰, Su¹⁰, Nu¹⁰, u¹¹, C₁-C₄ haloaliphatic, C₁-C₄ heteroaliphatic, and C₁-C₄ heterohaloaliphatic; and
u¹⁰ and u¹¹ each independently is selected from the group of hydrogen, C₁-C₄ aliphatic, phenyl, and benzyl; and
n is 0 or 1.

### [Invention 2]

An amyloid-β protein accumulation-inhibitory agent for inhibiting amyloid-β protein accumulation comprising a compound represented by the following formulas: 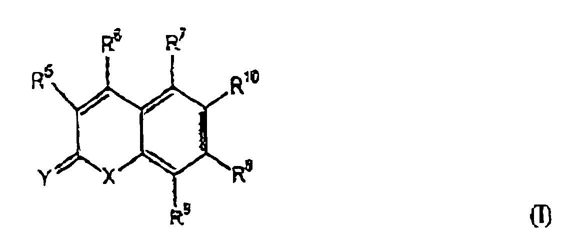 or 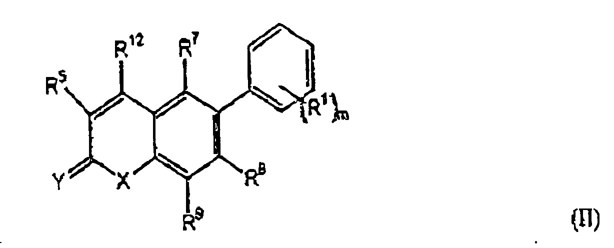 or 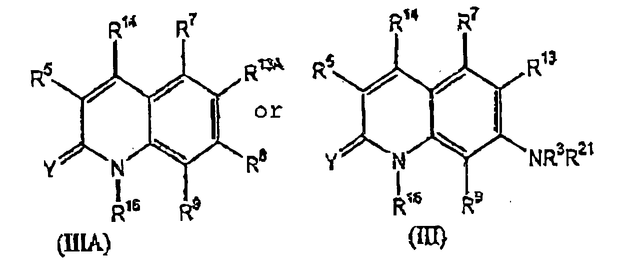 or 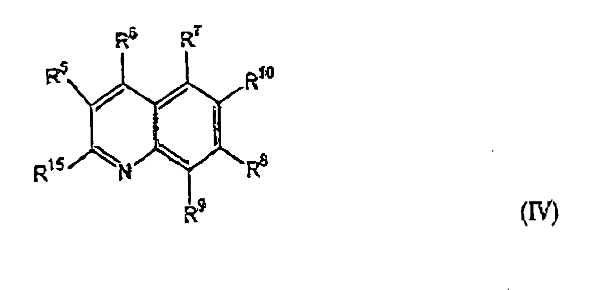 or 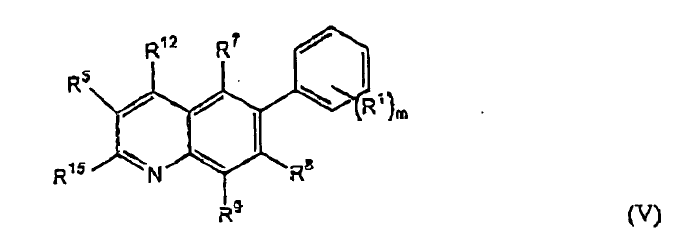 or 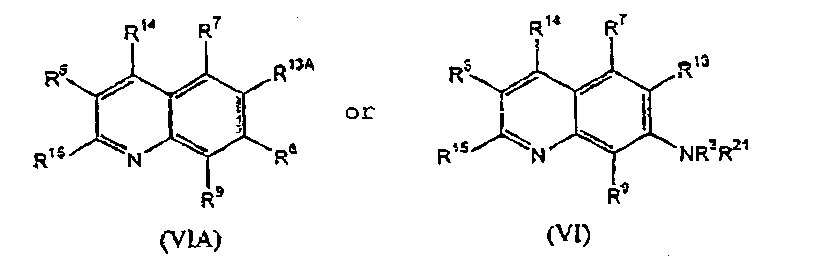 or 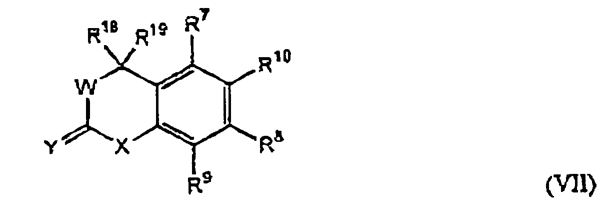 or 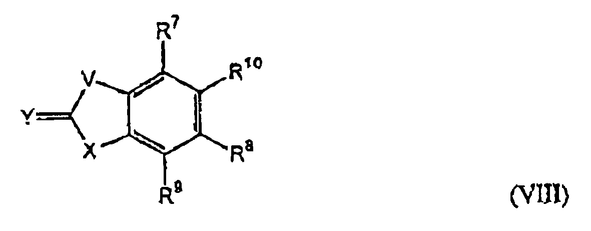 or 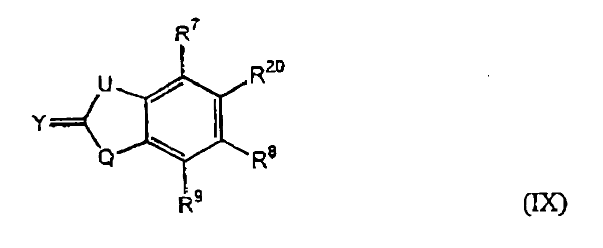 or 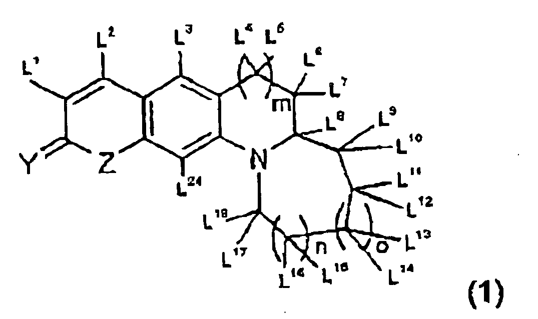 or 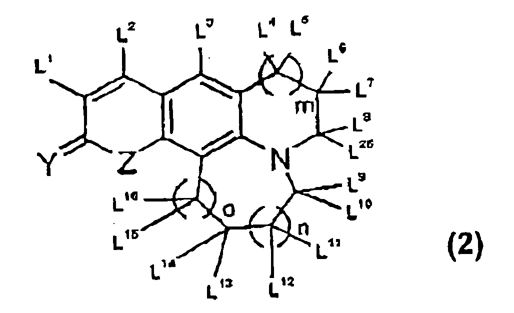 or 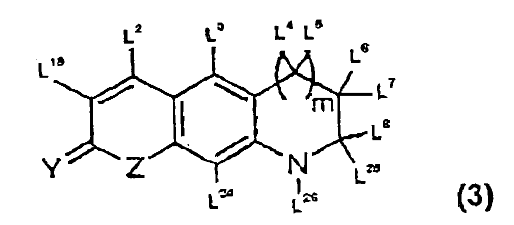 or 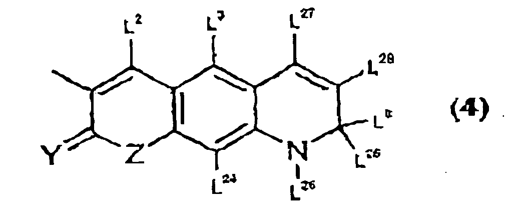 or 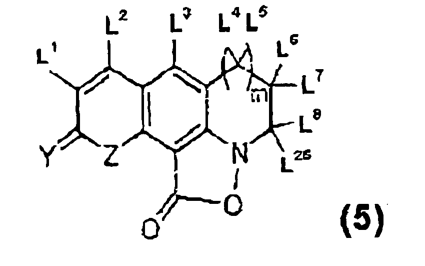 or 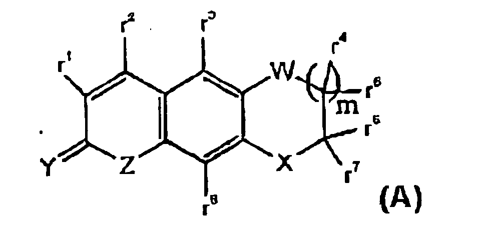 or 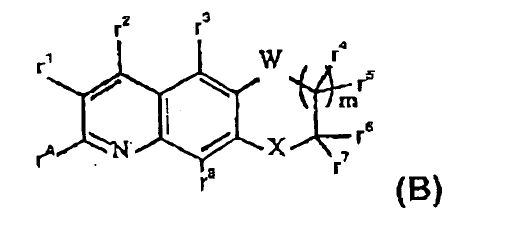 or 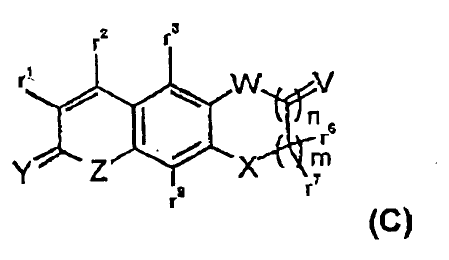 or 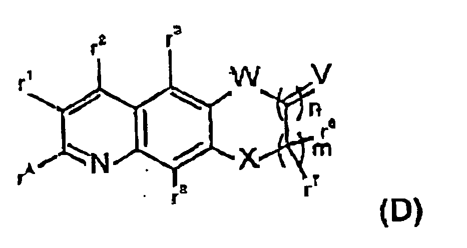 or 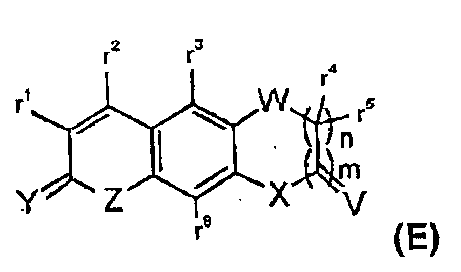 or 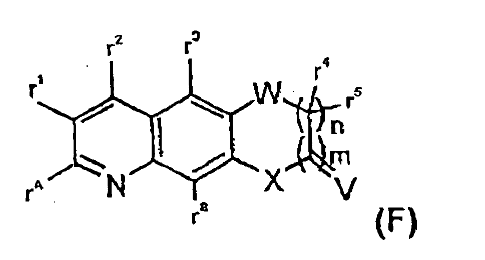 or 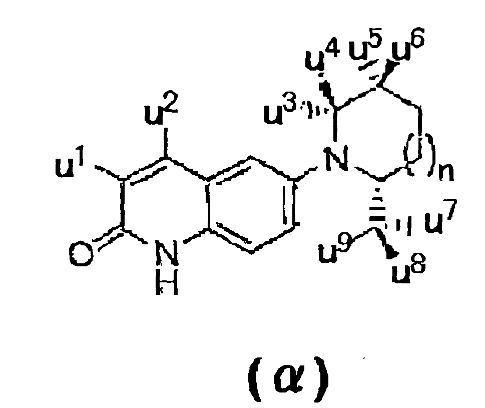 or a pharmaceutically acceptable salt thereof,
wherein, in the formulas (I) to (IX),
R¹ and R² are each independently selected from the group of COR³, CSR³, SO₂R³, NO, NR³R⁴, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, (CH₂)ₙR^{3A}, aryl, and heteroaryl, wherein the alkyl, alkenyl, alkynyl, haloalkyl, haloalkenyl, haloalkynyl, heteroalkyl, heteroalkenyl, heteroalkynyl, aryl, and heteroaryl are optionally substituted with F, Cl, Br, I, OR³, NR³R⁴, CN, NO₂, SR³, SOR³, SO₂R³, C₁-C₄ alkyl, C₁-C₄ haloalkyl or C₁-C₄ heteroalkyl; or
R¹ and R² are taken together to form a three- to nine-membered alkyl, alkenyl, heteroalkyl, or heteroalkenyl ring, wherein the alkyl, alkenyl, heteroalkyl, or heteroalkenyl ring are optionally substituted with F, Cl, Br, I, OR³, NR³R⁴, C₁-C₄ alkyl, C₁-C₄ haloalkyl or C₁-C₄ heteroalkyl; or R¹ and R² are taken together to form one of: 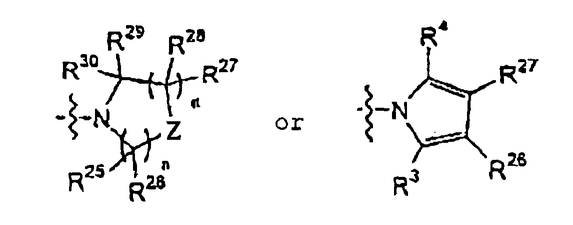 R³ and R⁴ are each independently selected from the group of hydrogen, C₁-C₈ alkyl, C₂-C₈ alkenyl, C₂-C₈ alkynyl, C₁-C₈ haloalkyl, C₁-C₈ heteroalkyl, heteroaryl, and aryl, wherein the alkyl, alkenyl, alkynyl, haloalkyl, heteroalkyl, heteroaryl, and aryl are optionally substituted with halogen, C₁-C₄ alkyl, C₁-C₄ haloalkyl or C₁-C₄ heteroalkyl;
R^{3A} is aryl or heteroaryl, wherein the aryl and heteroaryl is optionally substituted with halogen, CN, NO₂, C₁-C₄ alkyl, C₁-C₄ haloalkyl or C₁-C₄ heteroalkyl;
R⁵ is selected from the group of hydrogen, F, Cl, Br, I, OR³, SR³, NR³R⁴, C₁-C₄ alkyl, C₁-C₄ haloalkyl or C₁-C₄ heteroalkyl;
R⁶ is selected from the group of F, Cl, Br, I, CH₃, CE₃, CHF₂, CFH₂, CN, CF₂Cl, CF₂OR³, OR³, SR³, SOR³, SO₂R³, CO₂R³, NR³R⁴, C₁-C₄ alkyl, C₂-C₄ alkenyl, C₂-C₄ alkynyl, C₁-C₄ haloalkyl, C₂-C₄ haloalkenyl, C₂-C₄ haloalkynyl, C₁-C₄ heteroalkyl, C₂-C₄ heteroalkenyl, and C₂-C₄ heteroalkynyl, wherein the alkyl, alkenyl, alkynyl, haloalkyl, haloalkenyl, haloalkynyl, heteroalkyl, heteroalkenyl, and heteroalkynyl are optionally substituted with F, Cl, Br, C₁-C₄ alkyl, C₁-C₄ haloalkyl or C₁-C₄ heteroalkyl;
R⁷ and R⁶ are each independently selected from the group of hydrogen, F, Cl, Br, I, CN, OR³, NR³R⁴, NR³CR³R⁴CONR³R⁴, Cₙ(R³)₂ₙOR³, SR³, SOR³, SO₂R³, NR³COR⁴, C₁-C₈ alkyl, C₁-C₈ haloalkyl, and C₁-C₈ heteroalkyl ;
R⁹ is selected from the group of hydrogen, F, Br, Cl, I, OR³, NR³R⁴, SR³, SOR³, SO₂R³, C₁-C₄ alkyl, C₁-C₄ haloalkyl and C₁-C₄ heteroalkyl;,
R¹⁰ is selected from the group represented by the following formulas: 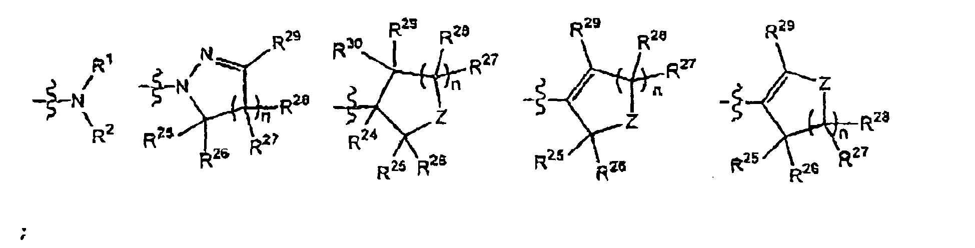 R¹¹ is selected from the group of F, Br, Cl, I, C₁-C₆ alkyl, C₁-C₆ haloalkyl, C₁-C₆ heteroalkyl, NO₂, CN, CF₃, OR³, NR³R⁴, SR³, SOR³, and SO₂R³;
R¹² is selected from the group of F, Br, Cl, I, CN, OR³, SR³, SOR³, SO₂R³, NR³R⁴, and C₁-C₄ haloalkyl;
R¹³ is selected from the group of hydrogen, F, Cl, Br, I, CN, OR³, NR³R⁴, COR³, CO₂R³, SR³, SOR³, SO₂R³, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, and (CHₐ)ₙR^{3A}, wherein the alkyl, alkenyl, alkynyl, haloalkyl, haloalkenyl, haloalkynyl, heteroalkyl, heteroalkenyl and heteroalkynyl are optionally substituted with F, Cl, Br, I, CN, NO₂, NR³R⁴, SR³, C₁-C₄ alkyl, C₁-C₄ haloalkyl or C₁-C₄ heteroalkyl;
R^{13A} is NHR¹ or heteroaryl, wherein the heteroaryl is optionally substituted with F, Cl, Br, I, CN, NO₂, NR³R⁴, SR³, SOR³, SO₂R³, C₁-C₄ alkyl, C₁-C₄ haloalkyl or C₁-C₄ heteroalkyl;
R¹⁴ is selected from the group of F, Br, Cl, I, CF₃, CHF₂, CH₂F, CF₂Cl, and CF₂OR³;
R¹⁵ is selected from the group of F, Br, Cl, I, CN, C₁-C₄ alkyl, C₁-C₄ haloalkyl, C₁-C₄ heteroalkyl, OR¹⁶, NR¹⁶R⁴, SR¹⁶, CH₂R¹⁶, COR³, CO₂R³, CONR³R⁴, SOR³, and SO₂R³;
R¹⁶ is selected from the group of hydrogen, C₁-C₈ alkyl, C₁-C₈ haloalkyl, C₁-C₈ heteroalkyl, CH₃R^{3A}, aryl, heteroaryl COR¹⁷, CO₂R¹⁷, and CONR¹⁷R¹⁷;
R¹⁷ is selected from the group of hydrogen, C₂-C₄ alkyl, C₁-C₄ haloalkyl and C₁-C₄ heteroalkyl;
R¹⁸ and R¹⁹ are each independently selected from the group of hydrogen, C₁-C₆ alkyl, C₁-C₆ haloalkyl and C₁-C₆ heteroalkyl; or
R¹⁸ and R¹⁹ are taken together to form a three- to seven-membered ring;
R²⁰ is aryl or heteroaryl, wherein the aryl or heteroaryl are optionally substituted with F, Cl, Br, CN, OR³, SR³, SOR³, SO₂R³, NO₂, NR³R³, C₁-C₄ alkyl, C₁-C₄ haloalkyl or C₁-C₄ heteroalkyl;
R²¹ is selected from the group of CR³R⁴CONR³R⁴, Cₙ(R³)₂ₙOR³, SOR³, SO₂R³, C₂-C₈ alkyl, C₂-C₈ haloalkyl, and C₂-C₈ heteroalkyl;
R²² and R²³ are each independently selected from the group of hydrogen, C₁-C₆ alkyl, C₁-C₆ haloalkyl and C₁-C₆ heteroalkyl; or
R²² and R²³ are taken together to form a three- to seven-membered ring;
R²⁴ is hydrogen or OR³;
R²⁵ through R³⁰ are each independently selected from the group of hydrogen, F, Cl, Br, I, OR³, NR³R⁴, SR³, SOR³, SO₂R³, C₁-C₆ alkyl, C₁-C₆ haloalkyl, C₁-C₆ heteroalkyl, C₂-C₆ alkynyl and C₂-C₆ alkenyl, wherein the alkyl, haloalkyl, heteroalkyl, alkynyl, and alkenyl are optionally substituted with F, Cl, Br, I, OR³, NR³R⁴, C₁-C₄ alkyl, C₁-C₄ haloalkyl, C₁-C₄ heteroalkyl, aryl or heteroaryl, and wherein the aryl and heteroaryl are optionally substituted with F, Cl, Br, I, CN, NO₂, OH, OCH₃, CF₃ or C₁-C₆ alkyl; or
Any two of R²⁵ R²⁶, R²⁷, R²⁸, R²⁹ and R³⁰ are taken together to form a three to seven-membered alkyl or alkenyl or heteroalkyl ring; or
any four of R²⁵ R²⁶, R²⁷, R²⁸, R²⁹ and R³⁰ are taken together to form a fused aromatic ring;
Q is O or S;
U is selected from the group of V, OCR²²R²³, SCR²²R²³, NR³CR²²R²³, and CR³R⁴CR²²R²³;
V is selected from the group of O, NR³, CR²²R²³, CR³R⁴O, and CR³R⁴S ;
W is selected from the group of 0, S, NR³, and CR³R⁴;
X is selected from the group of 0, S and NR¹⁶;
Y is selected from the group of 0, S, NR³, NOR³ and CR³R⁴;
Z is selected from the group of 0, S, NR³, C=O, and CR²⁵R²⁶; or
Z is two hydrogens;
n is 1, 2 or 3; and
m is 1 to 5,
and wherein, in the formulas (1) to (5),
L¹ is hydrogen, F, Cl, Br, I, NO₂, OL²⁰, NL²¹L²², SL²⁰, a C₁-C₄ alkyl or perhaloalkyl, or is an optionally substituted allyl, arylmethyl, alkynyl, alkenyl, aryl or heteroaryl, wherein L²¹ is hydrogen, a C₁-C₆ alkyl or perfluoroalkyl, aryl, heteroaryl, optionally substituted allyl or arylmethyl, SC₂L²³ or S(O)L²³, wherein L²³ is hydrogen, a C₁-C₆ alkyl or perfluoroalkyl, aryl, heteroaryl, or optionally substituted allyl or arylmethyl, L²⁰ is hydrogen, a C₁-C₆ alkyl or perfluoroalkyl, aryl, heteroaryl, optionally substituted allyl or arylmethyl, and L²² is hydrogen, a C₁-C₄ alkyl or perfluoroalkyl, aryl, heteroaryl, optionally substituted allyl or arylmethyl, OL²⁰ or NHL²¹;
L² is hydrogen, F, Br, Cl, a C₁-C₄ alkyl or perhaloalkyl, aryl, heteroaryl, CF₃, CF₂H, CFH₂, CF₂OL²⁰, CH₂OL²⁰, or OL²⁰, wherein L²⁰ has the same definition given above;
L³ is hydrogen, a C₁-C₄ alkyl, F, Cl, Br, I , OL²⁰, NL²¹L²² or SL²⁰, wherein L²⁰ through L²² have the definition given above;
L⁴ and L⁵ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁴ and L⁵ taken together can form a three- to seven-membered ring optionally substituted with hydrogen, F, Cl, Br, OL²⁰ or NL²¹L²², wherein L²⁰ through L²² have the definitions given above;
L⁶ and L⁷ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁶ and L⁷ taken together can form a three- to seven-membered ring optionally substituted with hydrogen, F, Cl, Br, OL²⁰ or NL^{2l}L²², wherein L²⁰ through L²² have the definitions given above;
L⁸ is hydrogen, a C₁-C₁₂ alkyl or perfluoroalkyl, hydroxymethyl, aryl, heteroaryl or optionally substituted allyl, arylmethyl, alkynyl or alkenyl;
L⁹ through L¹⁸ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or any two of L⁹ through L¹⁸ taken together can form a three- to seven-membered ring optionally substituted with hydrogen, F, Cl, Br, OL²⁰ or NL²¹L²², wherein L²⁰ through L²² have the definitions given above;
L¹⁹ is F, NO₂ or SL²⁰, wherein L²⁰ has the definition given above;
L²⁴ is hydrogen, a C₁-C₄ alkyl, F, Cl, Br, I, NO₂, OL²⁰, NL²¹L²² or SL²⁰, wherein L²⁰ through L²² have the definitions given above;
L²⁵ is hydrogen, a C₁-C₁₂ alkyl or perfluoroalkyl, hydroxymethyl, aryl, heteroaryl or optionally substituted allyl, arylmethyl, alkynyl or alkenyl, or L²⁵ and L⁸ taken together can form a three- to seven-membered ring optionally substituted with hydrogen, F, Cl, Br, OL²⁰ or NL²¹L²², wherein L²⁰ through L²² have the definitions given above;
L²⁶ is hydrogen, a C₁-C₆ alkyl or perfluoroalkyl, NO₂, OL²⁰, C(O)L²⁰, C(O)OL²⁰, C(O)NL²¹, L²², or an optionally substituted aryl, heteroaryl, allyl or arylmethyl, wherein L²⁰ through L²² have the definitions given above;
L²⁷ and L²⁸ each independently are hydrogen, F, Cl, Br, I, Oh²⁰, NL²¹L²², a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L²⁷ and L²⁸ taken together can form a three- to seven-membered ring optionally substituted with hydrogen, F, Cl, Br, OL²⁰ or NL²¹L²², wherein L²⁰ through L²² have the definitions given above;
m is 0 or 1;
n is 0 or 1;
o is 0 or 1;
Y is 0 or S;
Z is O, S, NH, NL²² or NCOL²², wherein L²² has the definition given above; and
any two of L⁴ through L⁸, L²⁵ and L²⁸ taken together can form a three- to seven-membered ring optionally substituted with hydrogen, F, Cl, Br, OL²⁰ or NL²¹L²², wherein L²⁰ through L²² have the definitions given above,
and wherein, in the formulas (A) to (F),
r¹ is hydrogen, F, Cl, Br, I, NO₂, Or⁹, Nr¹⁰r¹¹, S(O)mr⁹, C₁-C₈ alkyl, C₃-C₈ cycloalkyl, C₁-C₈ heteroalkyl, C₁-C₈ haloalkyl, C₁-C₈ aryl, C₁-C₈ arylalkyl, C₁-C₈ heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r² is hydrogen, F, Cl, Br, I, CF₃, CF₂Cl, CF₂H, CFH₂, CF₂Or⁹, CH₂Or⁹, Or⁹, S(O)mr⁹, Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r³ is hydrogen, F, Cl, Br, I, Or⁹, S(O)mr⁹, Nr¹⁰r¹¹, or C₁-C₆ alkyl, C₁-C₆ heteroalkyl, or C₁-C₆ haloalkyl, wherein the alkyl, heteroalkyl, and haloalkyl groups may be optionally substituted;
r⁴ and r⁵ each independently are hydrogen, Or⁹, S(O)mr⁹, Nr¹⁰r¹¹, C(Y)Or¹¹, C(Y)Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁴ and r⁵ taken together can form a saturated or unsaturated three- to seven-membered ring that may be optionally substituted;
r⁶ and r⁷ each independently are hydrogen, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁶ and r⁷ taken together can form a saturated or unsaturated three- to seven-membered ring that may be optionally substituted; or
r⁶ and r⁵ taken together can form a saturated or unsaturated three- to seven-membered ring that may be optionally substituted;
r⁸ is hydrogen, C₁-C₄ alkyl, C₁-C₄ heteroalkyl, C₁-C₄ haloalkyl, F, Cl, Br, I, NO₂, Or⁹, Nr¹⁰r¹¹ or S(O)mr⁹, wherein the alkyl, heteroalkyl, and haloalkyl groups may be optionally substituted;
r⁹ is hydrogen, C₁-C₆ alkyl, C₁-C₆ heteroalkyl, C₁-C₆ haloalkyl, aryl, heteroaryl, arylalkyl, C₂-C₄ alkynyl or C₂-C₈ alkenyl, wherein the alkyl, heteroalkyl, haloalkyl, aryl, arylalkyl, heteroaryl, alkenyl and alkynyl groups may be optionally substituted;
r¹⁰ is hydrogen, C₁-C₆ alkyl, C₁-C₆ heteroalkyl, C₁-C₆ haloalkyl, C(Y)r¹², C(Y)Or¹², aryl, heteroaryl, C₂-C₄ alkynyl, C₂-C₄ alkenyl, arylalkyl, SO₂r¹² or S(O)r¹², wherein the alkyl, heteroalkyl, haloalkyl, aryl, arylalkyl, heteroaryl, alkynyl, and alkenyl groups may be optionally substituted;
r¹¹ is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, arylalkyl, heteroaryl, alkynyl and alkenyl groups may be optionally substituted;
r¹² is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, heteroaryl, arylalkyl, alkynyl and alkenyl groups may be optionally substituted;
r¹³ is hydrogen, C₁-C₈ alkyl, C₃-C₈ cycloalkyl, C₁-C₈ heteroalkyl, C₁-C₈ haloalkyl, aryl, heteroaryl, arylalkyl, heteroarylalkyl, C₂-C₄ alkynyl, or C₂-C₈ alkenyl, wherein the alkyl, cycloalkyl, heteroalkyl, haloalkyl, aryl, arylalkyl, heteroaryl, heteroarylalkyl, alkynyl and alkenyl groups may be optionally substituted; or
r¹³ and r⁴ taken together can form a saturated or unsaturated three- to seven-membered ring that may be optionally substituted;
r¹³ and any two of r⁴ through r⁷ taken together can form a saturated or unsaturated three- to seven-membered ring that may be optionally substituted;
r¹⁴ and r¹⁵ each independently are hydrogen, C₁-C₈ alkyl, C₃-C₈ cycloalkyl, C₁-C₈ heteroalkyl, C₁-C₈ haloalkyl, aryl, heteroaryl, arylalkyl, C₂-C₈ alkynyl or C₂-C₈ alkenyl, wherein the alkyl, cycloalkyl, heteroalkyl, haloalkyl, aryl, heteroaryl, arylalkyl, alkynyl and alkenyl groups may be optionally substituted;
r^{A} is hydrogen, F, Br, Cl, I, CN, C₁-C₆ alkyl, C₁-C₆ haloalkyl, C₁-C₆ heteroalkyl, Or¹⁶, Nr¹⁶r¹⁷, Sr¹⁶, CH₂r¹⁶, COr¹⁷, CO₂r¹⁷, CONr¹⁷r¹⁷, SOr¹⁷ or SO₂r¹⁷, wherein the alkyl, haloalkyl, and heteroalkyl groups may be optionally substituted;
r¹⁶ is hydrogen, C₁-C₈ alkyl, C₁-C₈ haloalkyl, C₁-C₈ heteroalkyl, COr¹⁷, CO₂r¹⁷, CONr¹⁷r¹⁷, C₂-C₈ alkynyl, C₂-C₈ alkenyl, aryl or heteroaryl, wherein the alkyl, heteroalkyl, haloalkyl, aryl, heteroaryl, alkynyl and alkenyl groups may be optionally substituted;
r¹⁷ is hydrogen, C₁-C₄ alkyl, C₁-C₄ haloalkyl or C₁-C₄ heteroalkyl, wherein the alkyl, heteroalkyl, and haloalkyl groups may be optionally substituted;
m is 0, 1 or 2;
n is 1 or 2;
V is 0, S or Cr¹⁴r¹⁵;
W is O, S, NH, Nr¹³, NC(Y)r¹¹, or NSO₂r¹¹;
X and Z each independently are 0, S(O)m, NH, Nr¹¹, NC(Y)r¹¹, NSO₂r¹² or NS(O)R¹²; and
Y is O or S, and
wherein, in the formula (α),
u¹ is hydrogen, F, Cl, or C₁-C₃ aliphatic;
u² is selected from the group of hydrogen, F, Cl, Br, C₁-C₄ aliphatic, C₁-C₄ haloaliphatic, and C₁-C₄ heteroaliphatic;
u³ and u⁴ each independently is selected from the group of hydrogen, C₁-C₄ aliphatic, C₁-C₄ haloaliphatic, C₁-C₄ heteroaliphatic, optionally substituted aryl and heteroaryl;
u⁵ and u⁶ each independently is selected from the group of hydrogen, F, Cl, Ou¹⁰, C₁-C₄ aliphatic, C₁-C₄ haloaliphatic, and C₁-C₄ heteroaliphatic;
u⁷ and u⁸ each independently is selected from the group of hydrogen, F, Cl, C₁-C₄ aliphatic, C₁-C₄ haloaliphatic, and C₁-C₄ heteroaliphatic; or
u⁷ and u⁸ taken together form a carbonyl group;
u⁹ is selected from the group of halogen, Ou¹⁰, Su¹⁰, Nu¹⁰, u¹¹, C₁-C₄ haloaliphatic, C₁-C₄ heteroaliphatic, and C₁-C₄ heterohaloaliphatic; and
u¹⁰ and u¹¹ each independently is selected from the group of hydrogen, C₁-C₄ aliphatic, phenyl, and benzyl; and
n is 0 or 1.

### [Invention 3]

Use for inhibiting amyloid-β protein accumulation of a compound represented by the following formulas: 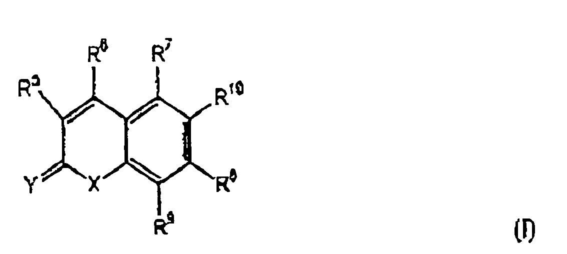 or 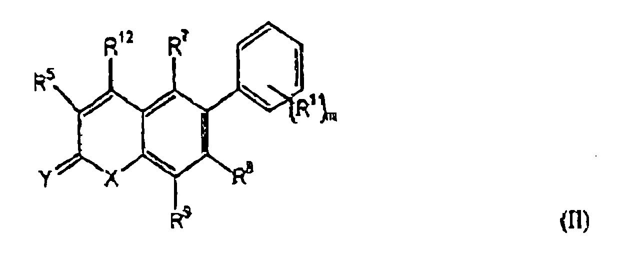 or 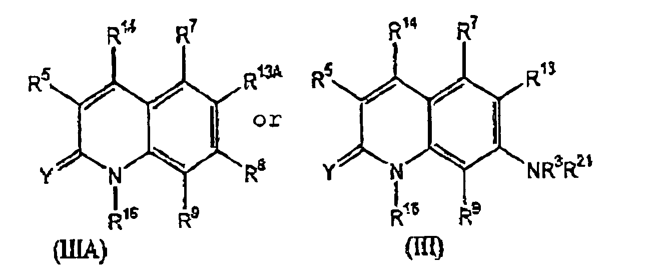 or 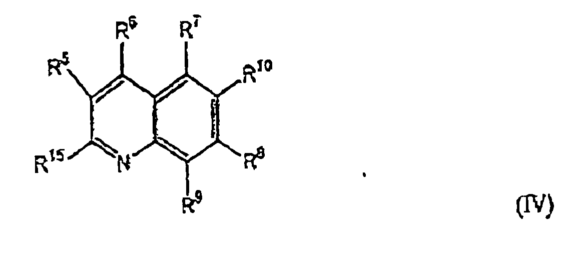 or 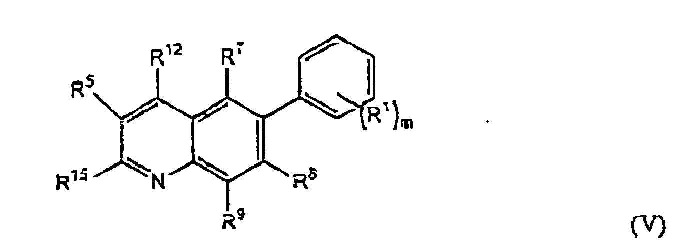 or 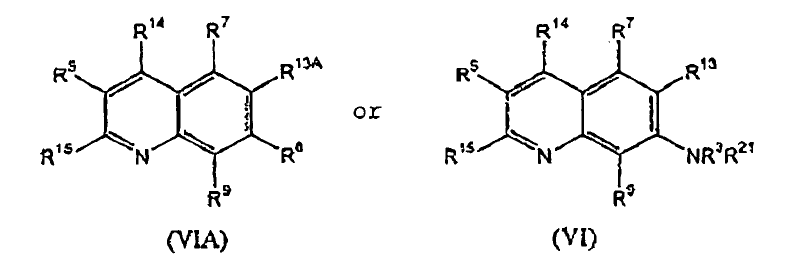 or 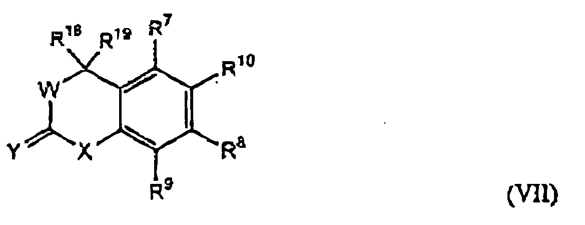 or 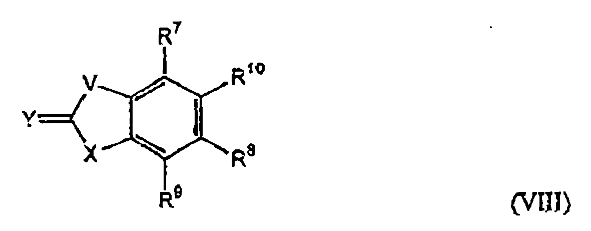 or 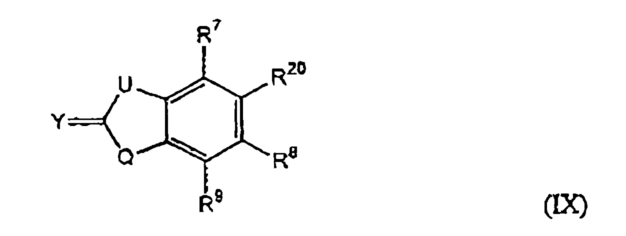 or 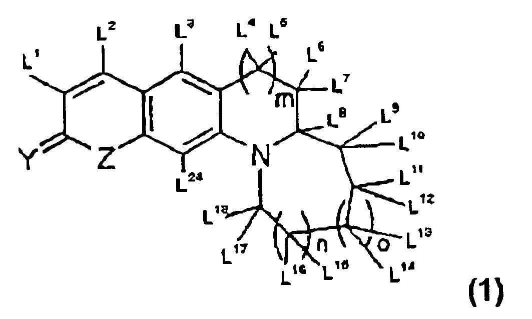 or 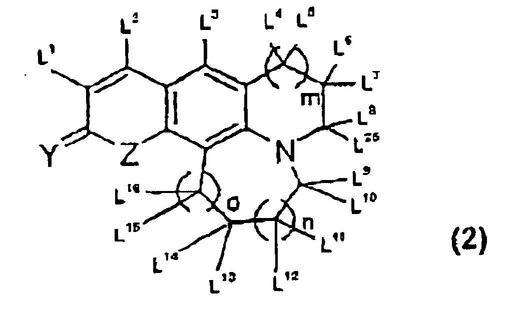 or 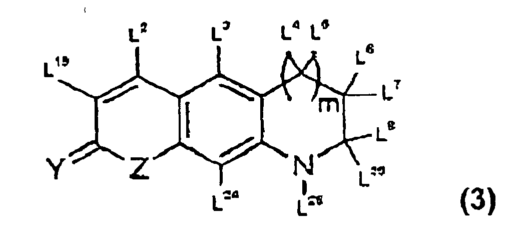 or 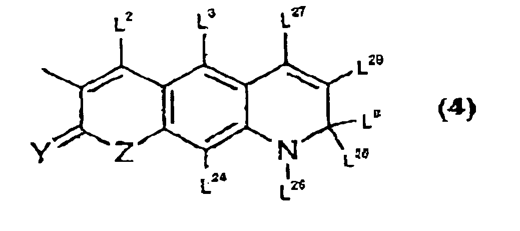 or 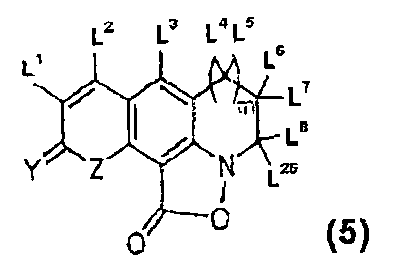 or 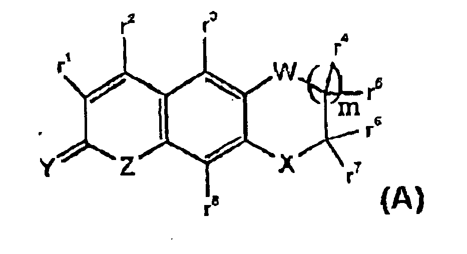 or 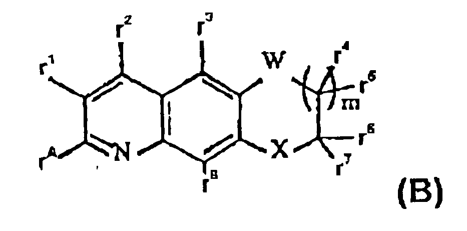 or 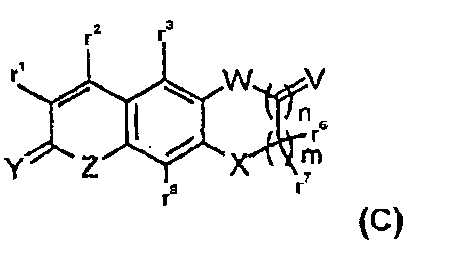 or 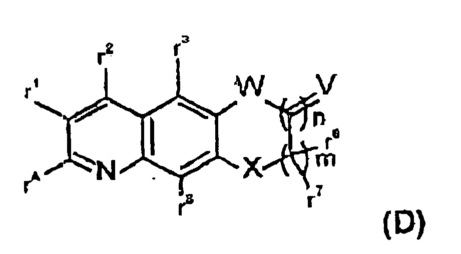 or 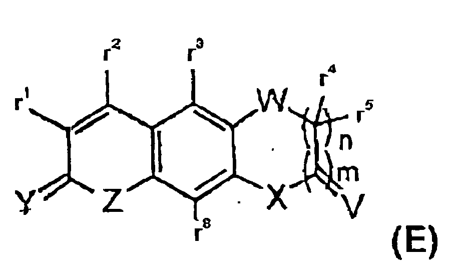 or 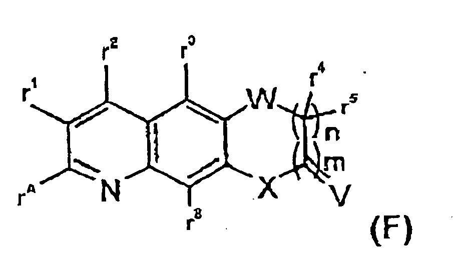 or 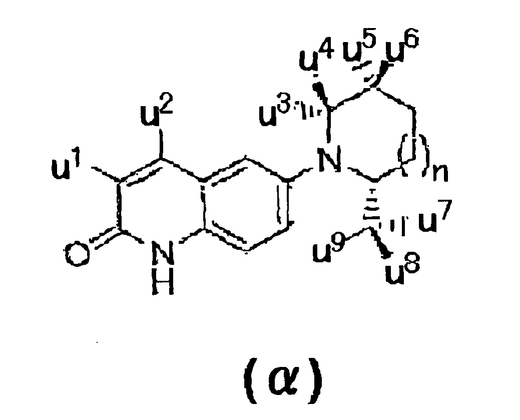 or a pharmaceutically acceptable salt thereof,
wherein, in the formulas (I) to (IX),
R¹ and R² are each independently selected from the group of COR³, CSR³, SO₂R³, NO, NR³R⁴, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, (CH₂)ₙR^{3A}, aryl, and heteroaryl, wherein the alkyl, alkenyl, alkynyl, haloalkyl, haloalkenyl, haloalkynyl, heteroalkyl, heteroalkenyl, heteroalkynyl, aryl, and heteroaryl are optionally substituted with F, Cl, Br, I, OR³, NR³R⁴, CN, NO₂, SR³, SOR³, SO₂R³, C₁-C₄ alkyl, C₁-C₄ haloalkyl or C₁-C₄ heteroalkyl; or
R¹ and R² are taken together to form a three- to nine-membered alkyl, alkenyl, heteroalkyl, or heteroalkenyl ring, wherein the alkyl, alkenyl, heteroalkyl, or heteroalkenyl ring are optionally substituted with F, Cl, Br, I, OR³, NR³R⁴, C₁-C₄ alkyl, C₁-C₄ haloalkyl or C₁-C₄ heteroalkyl; or R¹ and R² are taken together to form one of: 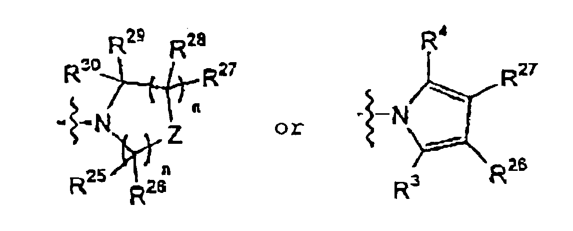 R³ and R⁴ are each independently selected from the group of hydrogen, C₁-C₈ alkyl, C₂-C₈ alkenyl, C₂-C₅ alkynyl, C₁-C₈ haloalkyl, C₁-C₈ heteroalkyl, heteroaryl, and aryl, wherein the alkyl, alkenyl, alkynyl, haloalkyl, heteroalkyl, heteroaryl, and aryl are optionally substituted with halogen, C₁-C₄ alkyl, C₁-C₄ haloalkyl or C₁-C₄ heteroalkyl;
R^{3A} is aryl or heteroaryl, wherein the aryl and heteroaryl is optionally substituted with halogen, CN, NO₂, C₁-C₄ alkyl, C₁-C₄ haloalkyl or C₁-C₄ heteroalkyl;
R⁵ is selected from the group of hydrogen, F, Cl, Br, I, OR³, SR³, NR³R⁴, C₁-C₄ alkyl, C₁-C₄ haloalkyl or C₁-C₄ heteroalkyl;
R⁶ is selected from the group of F, Cl, Br, I, CH₃, CF₃, CHF₂, CFH₂, CN, CF₂Cl, CF₂OR³, OR³, SR³, SOR³, SO₂R³, CO₂R³, NR³R⁴, C₁-C₄ alkyl, C₂-C₄ alkenyl, C₂-C₄ alkynyl, C₁-C₄ haloalkyl, C₂-C₄ haloalkenyl, C₂-C₄ haloalkynyl, C₁-C₄ heteroalkyl, C₂-C₄ heteroalkenyl, and C₂-C₄ heteroalkynyl, wherein the alkyl, alkenyl, alkynyl, haloalkyl, haloalkenyl, haloalkynyl, heteroalkyl, heteroalkenyl, and heteroalkynyl are optionally substituted with F, Cl, Br, C₁-C₄ alkyl, C₁-C₄ haloalkyl or C₁-C₄ heteroalkyl;
R⁷ and R⁸ are each independently selected from the group of hydrogen, F, Cl, Br, I, CN, OR³, NR₃R⁴, NR³CR³R⁴CONR³R⁴, Cₙ(R³)₂ₙOR³, SR³, SOR³, SO₂R³, NR³COR⁴, C₁-C₈ alkyl, C₁-C₈ haloalkyl, and C₁-C₈ heteroalkyl ;
R⁹ is selected from the group of hydrogen, F, Br, Cl, I, OR³, NR³R⁴, SR³, SOR³, SO₂R³, C₁-C₄ alkyl, C₁-C₄haloalkyl and C₁-C₄ heteroalkyl;,
R¹⁰ is selected from the group represented by the following formulas: 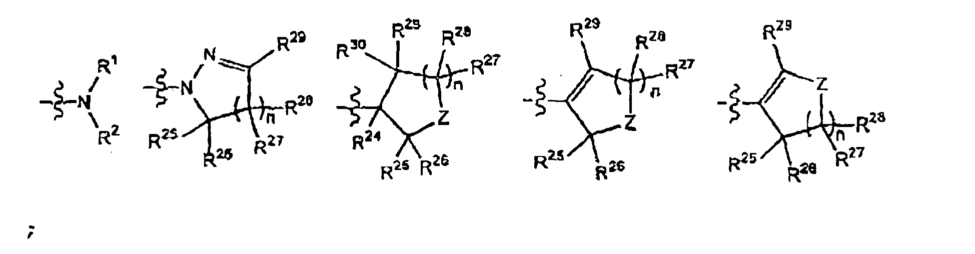 R¹¹ is selected from the group of F, Br, Cl, I, C₁-C₆ alkyl, C₁-C₆ haloalkyl, C₁-C₆ heteroalkyl, NO₂, CN, CF₃, OR³, NR³R⁴, SR³, SOR³, and SO₂R³:
R¹² is selected from the group of F, Br, Cl, I, CN, OR³, SR³, SOR³, SO₂R³, NR³R⁴, and C₁-C₄ haloalkyl;
R¹³ is selected from the group of hydrogen, F, Cl, Br, I, CN, OR³, NR³R⁴, COR³, CO₂R³, SR³, SOR³, SO₂R³, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, and (CH₂)ₙR^{3A}, wherein the alkyl, alkenyl, alkynyl, haloalkyl, haloalkenyl, haloalkynyl, heteroalkyl, heteroalkenyl and heteroalkynyl are optionally substituted with F, Cl, Br, I, CN, NO₂, NR³R⁴, SR³, C₁-C₉ alkyl, C₁-C₄ haloalkyl or C₁-C₄ heteroalkyl;
R^{13A} is NHR¹ or heteroaryl, wherein the heteroaryl is optionally substituted with F, Cl, Br, I, CN, NO₂, NR³R⁴, SR³, SOR³, SO₂R³, C₁-C₄ alkyl, C₁-C₄ haloalkyl or C₁-C₄ heteroalkyl;
R¹⁴ is selected from the group of F, Br, Cl, I, CF₃, CHF₂, CH₂F, CF₂Cl, and CF₂OR³;
R¹⁵ is selected from the group of F, Br, Cl, I, CN, C₁-C₄ alkyl, C₁-C₄ haloalkyl, C₁-C₄ heteroalkyl, OR¹⁶, NR¹⁶R⁴, SR₁₆, CH₂R¹⁶, COR³, CO₂R³, CONR³R⁴, SOR³, and SO₂R³;
R¹⁶ is selected from the group of hydrogen, C₁-C₈ alkyl, C₁-C₈ haloalkyl, C₁-C₈ heteroalkyl, CH₃R^{3A}, aryl, heteroaryl COR¹⁷ CO₂R¹⁷, and CONR¹⁷R¹⁷;
R¹⁷ is selected from the group of hydrogen, C₁-C₄ alkyl, C₁-C₄ haloalkyl and C₁-C₄ heteroalkyl;
R¹⁸ and R¹⁹ are each independently selected from the group of hydrogen, C₁-C₆ alkyl, C₁-C₆ haloalkyl and C₁-C₆ heteroalkyl; or
R¹⁸ and R¹⁹ are taken together to form a three- to seven-membered ring;
R²⁰ is aryl or heteroaryl, wherein the aryl or heteroaryl are optionally substituted with F, Cl, Br, CN, OR³, SR³, SOR³, SO₂R³, NO₂, NR³R³, C₁-C₄ alkyl, C₁-C₄ haloalkyl or C₁-C₄ heteroalkyl;
R²¹ is selected from the group of CR³R⁴CONR³R⁴, Cₙ(R³)₂ₙOR³, SOR³, SO₂R³, C₂-C₈ alkyl, C₂-C₈ haloalkyl, and C₂-C₈ heteroalkyl;
R²² and R²³ are each independently selected from the group of hydrogen, C₁-C₆ alkyl, C₁-C₆ haloalkyl and C₁-C₆ heteroalkyl; or
R²² and R²³ are taken together to form a three- to seven-membered ring;
R²⁴ is hydrogen or OR³;
R²⁵ through R³⁰ are each independently selected from the group of hydrogen, F, Cl, Br, I, OR³, NR³R⁴, SR³, SOR³, SO₂R³, C₁-C₆ alkyl, C₁-C₆ haloalkyl, C₁-C₆ heteroalkyl, C₂-C₆ alkynyl and C₂-C₆ alkenyl, wherein the alkyl, haloalkyl, heteroalkyl, alkynyl, and alkenyl are optionally substituted with F, Cl, Br, I, OR³, NR³R⁴, C₁-C₄ alkyl, C₁-C₄ haloalkyl, C₁-C₄ heteroalkyl, aryl or heteroaryl, and wherein the aryl and heteroaryl are optionally substituted with F, Cl, Br, I, CN, NO₂, OH, OCH₃, CF₃ or C₁-C₆ alkyl; or
Any two of R²⁵ R²⁶, R²⁷ R²⁸, R²⁹ and R³⁰ are taken together to form a three to seven-membered alkyl or alkenyl or heteroalkyl ring; or
any four of R²⁵ R²⁶, R²⁷, R²⁸, R²⁹ and R³⁰ are taken together to form a fused aromatic ring;
Q is 0 or S;
U is selected from the group of V, OCR²²R²³, SCR²²R²³, NR³CR²²R²³, and CR³R⁴CR²²R²³;
V is selected from the group of O, NR³, CR²²R²³, CR³R⁴⁰, and CR³R⁴S ;
W is selected from the group of 0, S, NR³, and CR³R⁴;
X is selected from the group of 0, S and NR¹⁶;
Y is selected from the group of O, S, NR³, NOR³ and CR³R⁴;
Z is selected from the group of 0, S, NR³, C=O, and CR²⁵R²⁶; or
Z is two hydrogens;
n is 1, 2 or 3; and
m is 1 to 5,
and wherein, in the formulas (1) to (5),
L¹ is hydrogen, F, Cl, Br, I, NO₂, OL²⁰, NL²¹L²², SL²⁰, a C₁-C₄ alkyl or perhaloalkyl, or is an optionally substituted allyl, arylmethyl, alkynyl, alkenyl, aryl or heteroaryl, wherein L²¹ is hydrogen, a C₁-C₆ alkyl or perfluoroalkyl, aryl, heteroaryl, optionally substituted allyl or arylmethyl, SO₂L²³ or S(O)L²³, wherein L²³ is hydrogen, a C₁-C₆ alkyl or perfluoroalkyl, aryl, heteroaryl, or optionally substituted allyl or arylmethyl, L²⁰ is hydrogen, a C₁-C₆ alkyl or perfluoroalkyl, aryl, heteroaryl, optionally substituted allyl or arylmethyl, and L²² is hydrogen, a C₁-C₄ alkyl or perfluoroalkyl, aryl, heteroaryl, optionally substituted allyl or arylmethyl, OL²⁰ or NHL²¹;
L² is hydrogen, F, Br, Cl, a C₁-C₄ alkyl or perhaloalkyl, aryl, heteroaryl, CF₃, CF₂H, CFH₂, CF₂OL²⁰, CH₂₀OL²⁰, or OL²⁰, wherein L²⁰ has the same definition given above;
L³ is hydrogen, a C₁-C₄ alkyl, F, Cl, Br, I, OL²⁰, NL²¹L²² or SL²⁰, wherein L²⁰ through L²² have the definition given above;
L⁴ and L⁵ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁴ and L⁵ taken together can form a three- to seven-membered ring optionally substituted with hydrogen, F, Cl, Br, OL²⁰ or NL²¹L²², wherein L²⁰ through L²² have the definitions given above;
L⁶ and L⁷ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, Oh²⁰ or NL²¹L²², or L⁶ and L⁷ taken together can form a three- to seven-membered ring optionally substituted with hydrogen, F, Cl, Br, OL²⁰ or NL²¹L²², wherein L²⁰ through L²² have the definitions given above;
L⁸ is hydrogen, a C₁-C₁₂ alkyl or perfluoroalkyl, hydroxymethyl, aryl, heteroaryl or optionally substituted allyl, arylmethyl, alkynyl or alkenyl;
L⁹ through L¹⁸ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or any two of L⁹ through L¹⁸ taken together can form a three- to seven-membered ring optionally substituted with hydrogen, F, Cl, Br, OL²⁰ or NL²¹L²², wherein L²⁰ through L²² have the definitions given above;
L¹⁹ is F, NO₂ or SL²⁰, wherein L²⁰ has the definition given above;
L²⁴ is hydrogen, a C₁-C₄ alkyl, F, Cl, Br, I, NO₂, OL²⁰, NL²¹L²² or SL²⁰, wherein L²⁰ through L²² have the definitions given above;
L²⁵ is hydrogen, a C₁-C₁₂ alkyl or perfluoroalkyl, hydroxymethyl, aryl, heteroaryl or optionally substituted allyl, arylmethyl, alkynyl or alkenyl, or L²⁵ and L⁸ taken together can form a three- to seven-membered ring optionally substituted with hydrogen, F, Cl, Br, OL²⁰ or NL²¹L²², wherein L²⁰ through L²² have the definitions given above;
L²⁶ is hydrogen, a C₁-C₆ alkyl or perfluoroalkyl, NO₂, OL²⁰, C(O)L²⁰, C(O)OL²⁰, C(O)NL²¹, L²², or an optionally substituted aryl, heteroaryl, allyl or arylmethyl, wherein L²⁰ through L²² have the definitions given above;
L²⁷ and L²⁸ each independently are hydrogen, F, Cl, Br, I, OL²⁰, NL²¹L²², a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L²⁷ and L²⁸ taken together can form a three- to seven-membered ring optionally substituted with hydrogen, F, Cl, Br, OL²⁰ or NL²¹L²², wherein L²⁰ through L²² have the definitions given above;
m is 0 or 1;
n is 0 or 1;
o is 0 or 1;
Y is O or S;
Z is O, S, NH, NL²² or NCOL²², wherein L²² has the definition given above; and
any two of L⁴ through L⁸ L²⁵ and L²⁸ taken together can form a three- to seven-membered ring optionally substituted with hydrogen, F, Cl, Br, OL²⁰ or NL²¹L²², wherein L²⁰ through L²² have the definitions given above,
and wherein, in the formulas (A) to (F),
r¹ is hydrogen, F, Cl, Br, I, NO₂, Or⁹, Nr¹⁰r¹¹, S(O)mr⁹, C₁-C₈ alkyl, C₃-C₈ cycloalkyl, C₁-C₈ heteroalkyl, C₁-C₈ haloalkyl, C₁-C₈ aryl, C₁-C₈ arylalkyl, C₁-C₈ heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r² is hydrogen, F, Cl, Br, I, CF₃, CF₂Cl, CF₂H, CFH₂, CF₂Or⁹, CH₂Or⁹, Or⁹, S(O)mr⁹, Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r³ is hydrogen, F, Cl, Br, I, Or⁹, S(O)mr⁹, Nr¹⁰r¹¹, or C₁-C₆ alkyl, C₁-C₆ heteroalkyl, or C₁-C₆ haloalkyl, wherein the alkyl, heteroalkyl, and haloalkyl groups may be optionally substituted;
r⁴ and r⁵ each independently are hydrogen, Or⁹, S(O)mr⁹, Nr¹⁰r¹¹, C(Y)or¹¹, C(Y)Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁴ and r⁵ taken together can form a saturated or unsaturated three- to seven-membered ring that may be optionally substituted;
r⁶ and r⁷ each independently are hydrogen, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁶ and r⁷ taken together can form a saturated or unsaturated three- to seven-membered ring that may be optionally substituted; or
r⁶ and r⁵ taken together can form a saturated or unsaturated three- to seven-membered ring that may be optionally substituted;
r⁸ is hydrogen, C₁-C₄ alkyl, C₁-C₄ heteroalkyl, C₁-C₄ haloalkyl, F, Cl, Br, I, NO₂, or⁹, Nr¹⁰r¹¹ or S(O)mr⁹, wherein the alkyl, heteroalkyl, and haloalkyl groups may be optionally substituted;
r⁹ is hydrogen, C₁-C₆ alkyl, C₁-C₆ heteroalkyl, C₁-C₆ haloalkyl, aryl, heteroaryl, arylalkyl, C₂-C₄ alkynyl or C₂-C₈ alkenyl, wherein the alkyl, heteroalkyl, haloalkyl, aryl, arylalkyl, heteroaryl, alkenyl and alkynyl groups may be optionally substituted;
r¹⁰ is hydrogen, C₁-C₆ alkyl, C₁-C₆ heteroalkyl, C₁-C₆ haloalkyl, C(Y)r¹², C(Y)Or¹², aryl, heteroaryl, C₂-C₄ alkynyl, C₂-C₄ alkenyl, arylalkyl, SO₂r¹² or S(O)r¹², wherein the alkyl, heteroalkyl, haloalkyl, aryl, arylalkyl, heteroaryl, alkynyl and alkenyl groups may be optionally substituted;
r¹¹ is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, arylalkyl, heteroaryl, alkynyl and alkenyl groups may be optionally substituted;
r¹² is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, heteroaryl, arylalkyl, alkynyl and alkenyl groups may be optionally substituted;
r¹³ is hydrogen, C₁-C₈ alkyl, C₃-C₈ cycloalkyl, C₁-C₈ heteroalkyl, C₁-C₈ haloalkyl, aryl, heteroaryl, arylalkyl, heteroarylalkyl, C₂-C₄ alkynyl, or C₂-C₈ alkenyl, wherein the alkyl, cycloalkyl, heteroalkyl, haloalkyl, aryl, arylalkyl, heteroaryl, heteroarylalkyl, alkynyl and alkenyl groups may be optionally substituted; or
r¹³ and r⁴ taken together can form a saturated or unsaturated three- to seven-membered ring that may be optionally substituted;
r¹³ and any two of r⁴ through r⁷ taken together can form a saturated or unsaturated three- to seven-membered ring that may be optionally substituted;
r¹⁴ and r¹⁵ each independently are hydrogen, C₁-C₈ alkyl, C₃-C₈ cycloalkyl, C₁-C₈ heteroalkyl, C₁-C₈ haloalkyl, aryl, heteroaryl, arylalkyl, C₂-C₈ alkynyl or C₂-C₈ alkenyl, wherein the alkyl, cycloalkyl, heteroalkyl, haloalkyl, aryl, heteroaryl, arylalkyl, alkynyl and alkenyl groups may be optionally substituted;
r^{A} is hydrogen, F, Br, Cl, I, CN, C₁-C₆ alkyl, C₁-C₆ haloalkyl, C₁-C₆ heteroalkyl, or¹⁶, Nr¹⁶r¹⁷, Sr¹⁶, CH₂r¹⁶, Cor¹⁷, CO₂r¹⁷, CONr¹⁷r¹⁷, sor¹⁷ or SO₂r¹⁷, wherein the alkyl, haloalkyl, and heteroalkyl groups may be optionally substituted;
r¹⁶ is hydrogen, C₁-C₈ alkyl, C₁-C₈ haloalkyl, C₁-C₈ heteroalkyl, Cor¹⁷,CO₂r¹⁷, CONr¹⁷r¹⁷, C₂-C₈ alkynyl, C₂-C₈ alkenyl, aryl or heteroaryl, wherein the alkyl, heteroalkyl, haloalkyl, aryl, heteroaryl, alkynyl and alkenyl groups may be optionally substituted;
r¹⁷ is hydrogen, C₁-C₄ alkyl, C₁-C₄ haloalkyl or C₁-C₄ heteroalkyl, wherein the alkyl, heteroalkyl, and haloalkyl groups may be optionally substituted;
m is 0, 1 or 2;
n is 1 or 2;
V is 0, S or Cr¹⁴r¹⁵;
W is 0, S, NH, Nr¹³, NC(Y)r¹¹, or NSO₂r¹¹;
X and Z each independently are O, S (O)m, NH, Nr¹¹, NC(Y)r¹¹, NSO₂r¹² or NS(O)r¹²; and
Y is O or S, and
wherein, in the formula (α),
u¹ is hydrogen, F, Cl, or C₁-C₃ aliphatic;
u² is selected from the group of hydrogen, F, Cl, Br, C₁-C₄ aliphatic, C₁-C₄ haloaliphatic, and C₁-C₄ heteroaliphatic;
u³ and u⁴ each independently is selected from the group of hydrogen, C₁-C₄ aliphatic, C₁-C₄ haloaliphatic, C₁-C₄ heteroaliphatic, optionally substituted aryl and heteroaryl;
u⁵ and u⁶ each independently is selected from the group of hydrogen, F, Cl, ou¹⁰, C₁-C₄ aliphatic, C₁-C₄ haloaliphatic, and C₁-C₄ heteroaliphatic;
u⁷ and u⁸ each independently is selected from the group of hydrogen, F, Cl, C₁-C₄ aliphatic, C₁-C₄ haloaliphatic, and C₁-C₄ heteroaliphatic; or
u⁷ and u⁸ taken together form a carbonyl group;
u⁹ is selected from the group of halogen, Ou¹⁰, Su¹⁰, Nu¹⁰, u¹¹, C₁-C₄ haloaliphatic, C₁-C₄ heteroaliphatic, and C₁-C₄ heterohaloaliphatic; and
u¹⁰ and u¹¹ each independently is selected from the group of hydrogen, C₁-C₄ aliphatic, phenyl, and benzyl; and
n is 0 or 1.

### [Invention 4]

A method for inhibiting amyloid-β protein accumulation, comprising a step of administering to a mammal which may be diagnosed with an amyloid-β protein-related disease an effective amount of a compound represented by the following formulas: 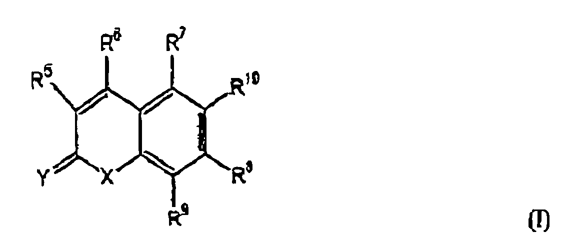 or 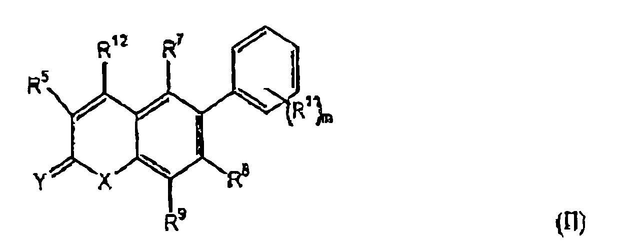 or 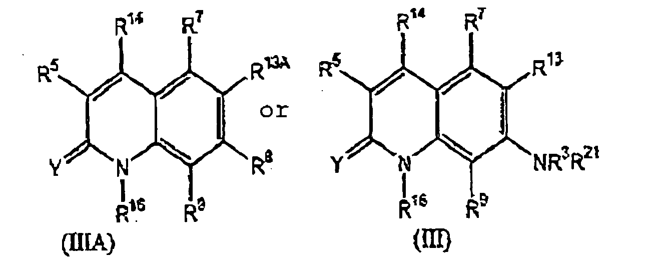 or 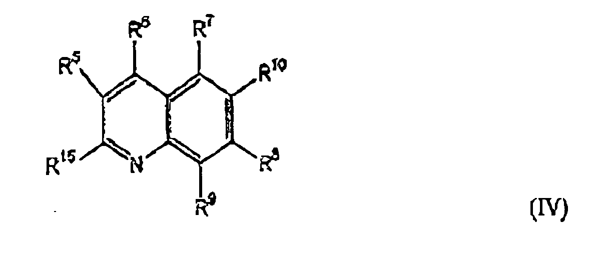 or 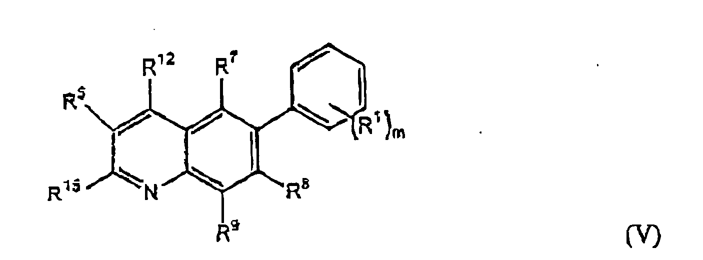 or 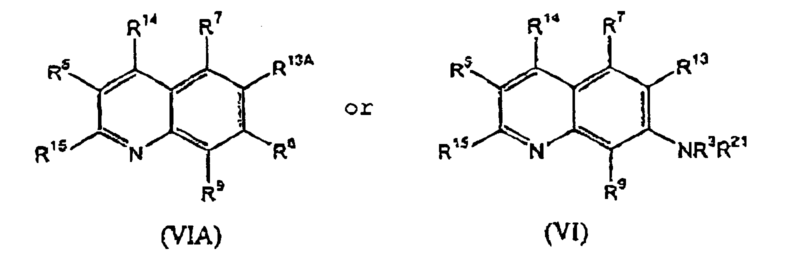 or 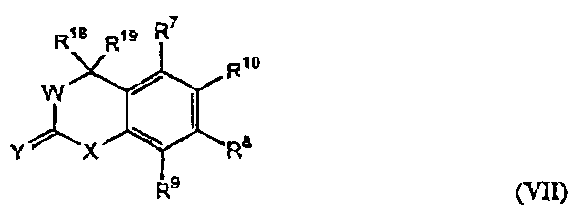 or 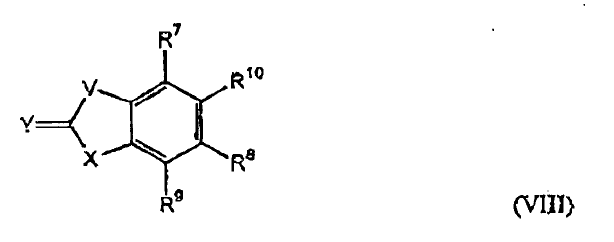 or 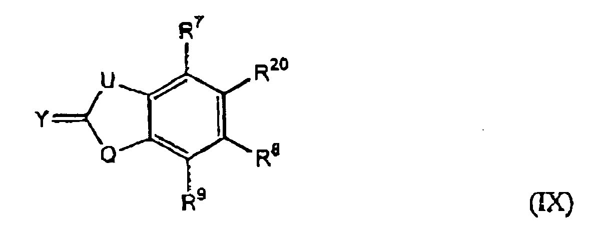 or 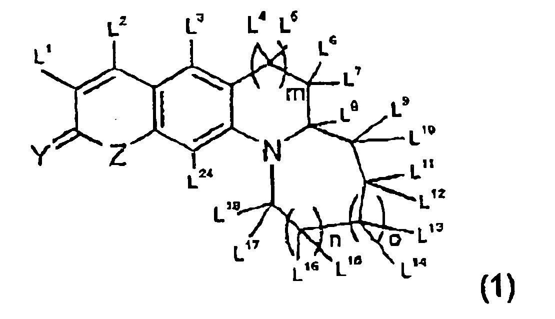 or 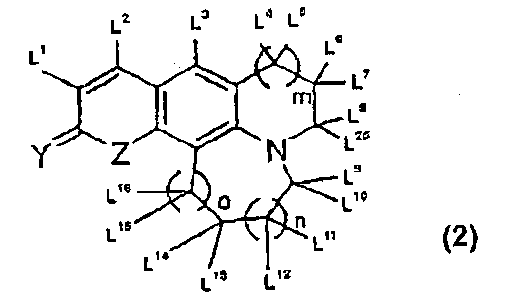 or 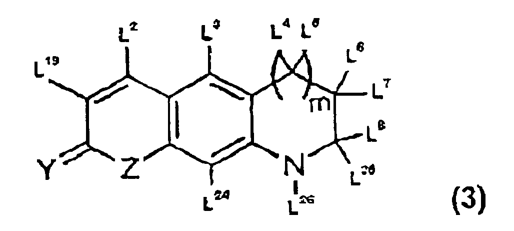 or 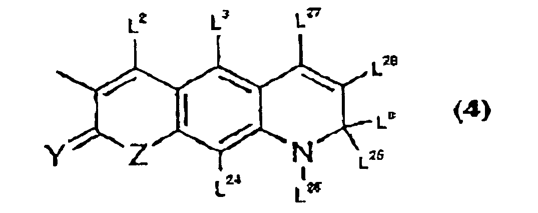 or 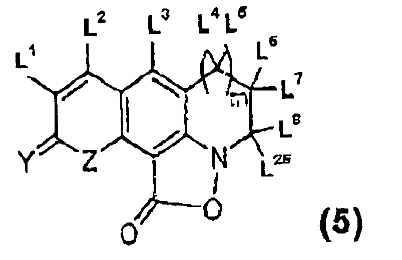 or 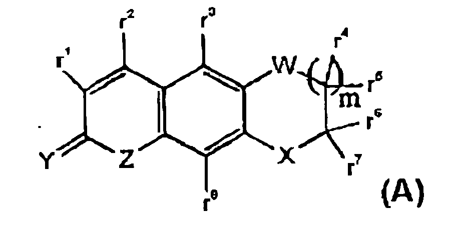 or 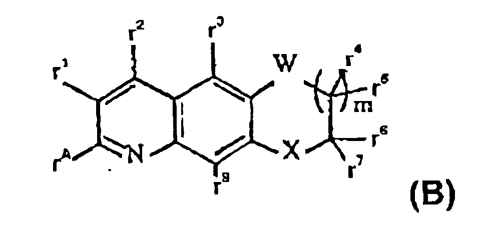 or 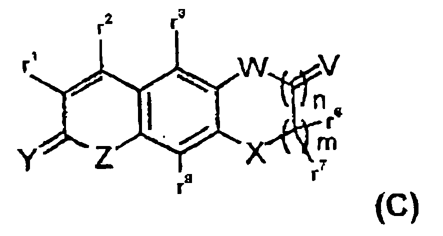 or 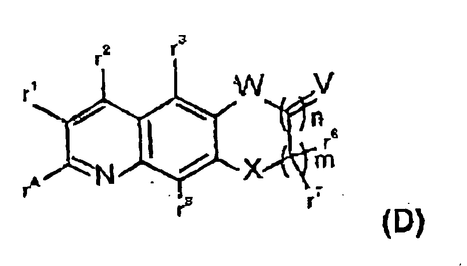 or 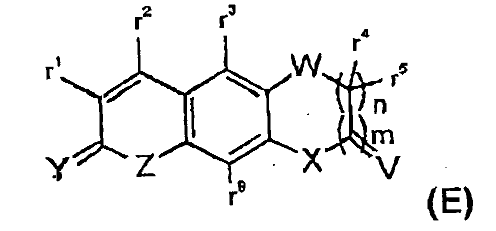 or 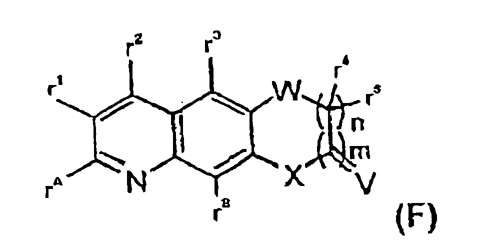 or 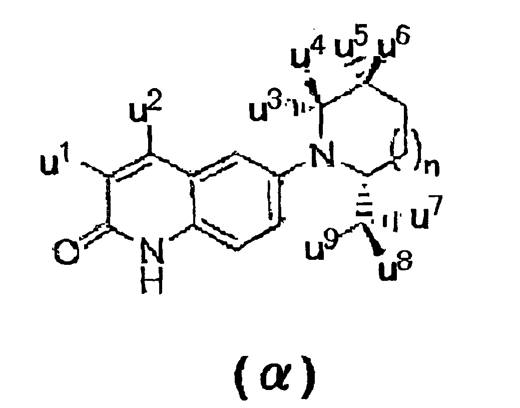 or a pharmaceutically acceptable salt thereof,
wherein, in the formulas (I) to (IX),
R¹ and R² are each independently selected from the group of COR³, CSR³, SO₂R³, NO, NR³R⁹, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, (CH₂)ₙR^{3A}, aryl, and heteroaryl, wherein the alkyl, alkenyl, alkynyl, haloalkyl, haloalkenyl, haloalkynyl, hetexoalkyl, heteroalkenyl, heteroalkynyl, aryl, and heteroaryl are optionally substituted with F, Cl, Br, I, OR³, NR³R⁴, CN, NO₂, SR³, SOR³, SOR³, C₁-C₄ alkyl, C₁-C₄ haloalkyl or C₁-C₄ heteroalkyl; or
R¹ and R² are taken together to form a three- to nine-membered alkyl, alkenyl, heteroalkyl, or heteroalkenyl ring, wherein the alkyl, alkenyl, heteroalkyl, or heteroalkenyl ring are optionally substituted with F, Cl, Br, I, OR³, NR³R⁴, C₁-C₄ alkyl, C₁-C₄ haloalkyl or C₁-C₄ heteroalkyl; or R¹ and R² are taken together to form one of: 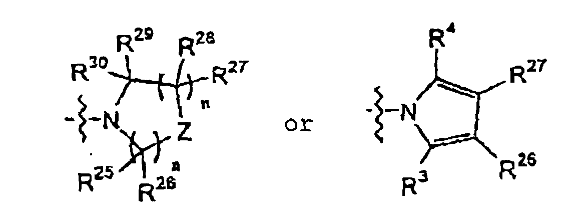 R³ and R⁴ are each independently selected from the group of hydrogen, C₁-C₈ alkyl, C₂-C₈ alkenyl, C₂-C₈ alkynyl, C₁-C₈ haloalkyl, C₁-C₈ heteroalkyl, heteroaryl, and aryl, wherein the alkyl, alkenyl, alkynyl, haloalkyl, heteroalkyl, heteroaryl, and aryl are optionally substituted with halogen, C₁-C₄ alkyl, C₁-C₄ haloalkyl or C₁-C₄ heteroalkyl;
R^{3A} is aryl or heteroaryl, wherein the aryl and heteroaryl is optionally substituted with halogen, CN, NO₂, C₁-C₄ alkyl, C₁-C₄ haloalkyl or C₁-C₄ heteroalkyl;
R⁵ is selected from the group of hydrogen, F, Cl, Br, I, OR³, SR³, NR³R⁴, C₁-C₄ alkyl, C₁-C₄ haloalkyl or C₁-C₄ heteroalkyl;
R⁶ is selected from the group of F, Cl, Br, I, CH₃, CF₃, CHF₂, CFH₂, CN, CF₂Cl, CF₂OR³, OR³, SR³, SOR³, SO₂R³, CO₂R³, NR³R⁴, C₁-C₄ alkyl, C₂-C₄ alkenyl, C₂-C₄ alkynyl, C₁-C₄ haloalkyl, C₂-C₄ haloalkenyl, C₂-C₄ haloalkynyl, C₁-C₄ heteroalkyl, C₂-C₄ heteroalkenyl, and C₂-C₄ heteroalkynyl, wherein the alkyl, alkenyl, alkynyl, haloalkyl, haloalkenyl, haloalkynyl, heteroalkyl, heteroalkenyl, and heteroalkynyl are optionally substituted with F, Cl, Br, C₁-C₄ alkyl, C₁-C₄ haloalkyl or C₁-C₄ heteroalkyl;
R⁷ and R⁶ are each independently selected from the group of hydrogen, F, Cl, Br, I, CN, OR³, NR³R⁴, NR³CR³R⁴CONR³R⁴, Cₙ(R³)₂ₙOR³. SR³, SOR³, SOR³, NR³COR⁴, C₁-C₈ alkyl, C₁-C₈ haloalkyl, and C₁-C₈ heteroalkyl;
R⁹ is selected from the group of hydrogen, F, Br, Cl, I, OR³, NR³R⁴, SR³, SOR³, SO₂R³ C₁-C₄ alkyl, C₁-C₄ haloalkyl and C₁-C₄ heteroalkyl;,
R¹⁰ is selected from the group represented by the following formulas: 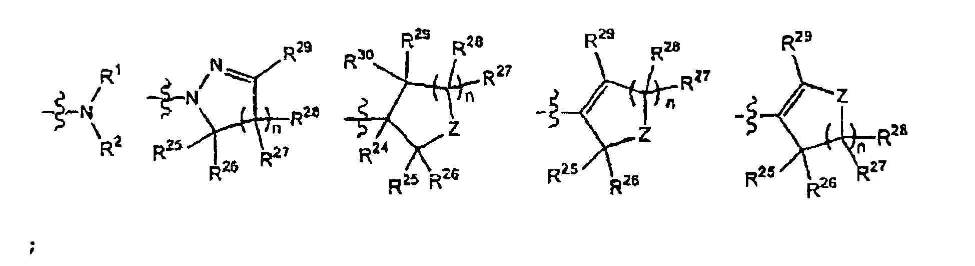 R¹¹ is selected from the group of F, Br, Cl, I, C₁-C₆ alkyl, C₁-C₆ haloalkyl, C₁-C₆ heteroalkyl, NO₂, CN, CF₃, OR³, NR³R⁴, SR³, SOR³, and SO₂R³;
R¹² is selected from the group of F, Br, Cl, I, CN, OR³, SR³, SOR³, SO₂R³, NR³R⁴, and C₁-C₄ haloalkyl;
R¹³ is selected from the group of hydrogen, F, Cl, Br, I, CN, OR³, NR³R⁴, COR³, CO₂R³, SR³, SOR³, SO₂R³. C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, and (CH₂)ₙR^{3A}, wherein the alkyl, alkenyl, alkynyl, haloalkyl, haloalkenyl, haloalkynyl, heteroalkyl, heteroalkenyl and heteroalkynyl, are optionally substituted with F, Cl, Br, I, CN, NO₂, NR³R⁴, SR³, C₁-C₄ alkyl, C₁-C₄ haloalkyl or C₁-C₄ heteroalkyl;
R^{13A} is NHR¹ or heteroaryl, wherein the heteroaryl is optionally substituted with F, Cl, Br, I, CN, NO₂, NR³R⁴, SR³, SOR³, SO₂R³, C₁-C₄ alkyl, C₁-C₄ haloalkyl or C₁-C₄ heteroalkyl;
R¹⁴ is selected from the group of F, Br, Cl, I, CF₃, CHF₂, CH₂F, CF₂Cl, and CF₂OR³;
R¹⁵ is selected from the group of F, Br, Cl, I, CN, C₁-C₄ alkyl, C₁-C₄ haloalkyl, C₁-C₄ heteroalkyl, OR¹⁶, NR¹⁶R⁴, SR¹⁶, CH₂R¹⁶, COR³, CO₂R³, CONR³R⁴, SOR³, and SO₂R³;
R¹⁶ is selected from the group of hydrogen, C₁-C₈ alkyl, C₁-C₈ haloalkyl, C₁-C₈ heteroalkyl, CH₃R^{3A}, aryl, heteroaryl COR¹⁷, CO₂R¹⁷, and CONR¹⁷R¹⁷;
R¹⁷ is selected from the group of hydrogen, C₁-C₄ alkyl, C₁-C₄ haloalkyl and C₁-C₄ heteroalkyl;
R¹⁸ and R¹⁹ are each independently selected from the group of hydrogen, C₁-C₆ alkyl, C₁-C₆ haloalkyl and C₁-C₆ heteroalkyl; or
R¹⁸ and R¹⁹ are taken together to form a three- to seven-membered ring;
R²⁰ is aryl or heteroaryl, wherein the aryl or heteroaryl are optionally substituted with F, Cl, Br, CN, OR³, SR³, SOR³, SO₂R³, NO₂, NR³R³, C₁-C₄ alkyl, C₁-C₄ haloalkyl or C₁-C₄ heteroalkyl;
R²¹ is selected from the group of CR³R⁴CONR³R⁴, Cₙ(R³)₂ₙOR³. SOR³, SO₂R³, C₂-C₈ alkyl, C₂-C₈ haloalkyl, and C₂-C₈ heteroalkyl;
R²² and R²³ are each independently selected from the group of hydrogen, C₁-C₆ alkyl, C₁-C₆ haloalkyl, and C₁-C₆ heteroalkyl; or
- R²² and R²³ are taken together to form a three- to seven-membered ring;
R²⁴ is hydrogen or OR³;
R²⁵ through R³⁰ are each independently selected from the group of hydrogen, F, Cl, Br, I, OR³, NR³R⁴, SR³, SOR³, SO₂R³, C₁-C₆ alkyl, C₁-C₆ haloalkyl, C₁-C₆ heteroalkyl, C₂-C₆ alkynyl and C₂-C₆ alkenyl, wherein the alkyl, haloalkyl, heteroalkyl, alkynyl, and alkenyl are optionally substituted with F, Cl, Br, I, OR³, NR³R⁴, C₁-C₄ alkyl, C₁-C₄ haloalkyl, C₁-C₄ heteroalkyl, aryl or heteroaryl, and wherein the aryl and heteroaryl are optionally substituted with F, Cl, Br, I, CN, NO₂, OH, OCH₃, CF₃ or C₁-C₆ alkyl; or
Any two of R²⁵ R²⁶, R²⁷, R²⁸, R²⁹ and R³⁰ are taken together to form a three to seven-membered alkyl or alkenyl or heteroalkyl ring; or
any four of R²⁵ R²⁶, R²¹, R²⁸, R²⁹ and R³⁰ are taken together to form a fused aromatic ring;
Q is 0 or S;
U is selected from the group of V, OCR²²R²³, SCR²²R²³, NR³CR²²R²³, and CR³R⁴CR²²R²³;
V is selected from the group of O, NR³, CR²²R²³, CR³R⁴⁰, and CR³R⁴S;
W is selected from the group of 0, S, NR³, and CR³R⁴;
X is selected from the group of O, S and NR¹⁶;
Y is selected from the group of O, S, NR³, NOR³ and CR³R⁴;
Z is selected from the group of 0, S, NR³, C=O, and CR²⁵R²⁶; or
Z is two hydrogens;
n is 1, 2 or 3; and
m is 1 to 5,
and wherein, in the formulas (1) to (5),
L¹ is hydrogen, F, Cl, Br, I, NO₂, OL²⁰, NL²¹L²², SL²⁰, a C₁-C₄ alkyl or perhaloalkyl, or is an optionally substituted allyl, arylmethyl, alkynyl, alkenyl, aryl or heteroaryl, wherein L²¹ is hydrogen, a C₁-C₆ alkyl or perfluoroalkyl, aryl, heteroaryl, optionally substituted allyl or arylmethyl, SO₂L²³ or S(O)L²³, wherein L²³ is hydrogen, a C₁-C₆ alkyl or perfluoroalkyl, aryl, heteroaryl, or optionally substituted allyl or arylmethyl, L²⁰ is hydrogen, a C₁-C₆ alkyl or perfluoroalkyl, aryl, heteroaryl, optionally substituted allyl or arylmethyl, and L²² is hydrogen, a C₁-C₄ alkyl or perfluoroalkyl, aryl, heteroaryl, optionally substituted allyl or arylmethyl, OL²⁰ or NHL²¹;
L² is hydrogen, F, Br, Cl, a C₁-C₄ alkyl or perhaloalkyl, aryl, heteroaryl, CF₃, CF₂H, CFH₂, CF₂OL²⁰, CH₂OL²⁰, or OL²⁰, wherein L²⁰ has the same definition given above;
L³ is hydrogen, a C₁-C₄ alkyl, F, Cl, Br, I, OL²⁰, NL²¹L²² or SL²⁰, wherein L²⁰ through L²² have the definition given above;
L⁴ and L⁵ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁴ and L⁵ taken together can form a three- to seven-membered ring optionally substituted with hydrogen, F, Cl, Br, OL²⁰ or NL²¹L²², wherein L²⁰ through L²² have the definitions given above;
L⁶ and L⁷ each independently are hydrogen, a C₁-C₄ alkyl or perfluoroalkyl, heteroaryl, optionally substituted allyl, arylmethyl, alkynyl or alkenyl, or an aryl optionally, substituted with hydrogen, F, Cl, Br, OL²⁰ or NL²¹L2², or L⁶ and L⁷ taken together can form a three- to seven-membered ring optionally substituted with hydrogen, F, Cl, Br, OL²⁰ or NL²¹L²², wherein L²⁰ through L²² have the definitions given above;
L⁸ is hydrogen, a C₁-C₁₂ alkyl or perfluoroalkyl, hydroxymethyl, aryl, heteroaryl or optionally substituted allyl, arylmethyl, alkynyl or alkenyl;
L⁹ through L¹⁸ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or any two of L⁹ through L¹⁸ taken together can form a three- to seven-membered ring optionally substituted with hydrogen, F, Cl, Br, OL²⁰ or NL²¹L²², wherein L²⁰ through L²² have the definitions given above;
L¹⁹ is F, NO₂ or SL²⁰, wherein L²⁰ has the definition given above;
L²⁴ is hydrogen, a C₁-C₄ alkyl, F, Cl, Br, I, NO₂, OL²⁰, NL²¹L²² or SL²⁰, wherein L²⁰ through L²² have the definitions given above;
L²⁵ is hydrogen, a C₁-C₁₂ alkyl or perfluoroalkyl, hydroxymethyl, aryl, heteroaryl or optionally substituted allyl, arylmethyl, alkynyl or alkenyl, or L²⁵ and L⁶ taken together can form a three- to seven-membered ring optionally substituted with hydrogen, F, Cl, Br, OL²⁰ or NL²¹L²², wherein L²⁰ through L²² have the definitions given above;
L²⁶ is hydrogen, a C₁-C₆ alkyl or perfluoroalkyl, NO₂, OL²⁰, C(O)L²⁰, C(O)OL²⁰, C(O)NL²¹, L²², or an optionally substituted aryl, heteroaryl, allyl or arylmethyl, wherein L²⁰ through L²² have the definitions given above;
L²⁷ and L²⁸ each independently are hydrogen, F, Cl, Br, I, OL²⁰, NL²¹L²², a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L²⁷ and L²⁸ taken together can form a three- to seven-membered ring optionally substituted with hydrogen, F, Cl, Br, OL²⁰ or NL²¹L²², wherein L²⁰ through L²² have the definitions given above;
m is 0 or 1;
n is 0 or 1;
o is 0 or 1;
Y is 0 or S;
Z is O, S, NH, NL²² or NCOL²², wherein L²² has the definition given above; and
any two of L⁴ through L⁸, L²⁵ and L²⁸ taken together can form a three- to seven-membered ring optionally substituted with hydrogen, F, Cl, Br, OL²⁰ or NL²¹L²², wherein L²⁰ through L²² have the definitions given above,
and wherein, in the formulas (A) to (F),
r¹ is hydrogen, F, Cl, Br, I, NO₂, Or⁹, Nr¹⁰r¹¹, S(O)ₘᵣ⁹, C₁-C₈ alkyl, C₃-C₈ cycloalkyl, C₁-C₈ heteroalkyl, C₁-C₈ haloalkyl, C₁-C₈ aryl, C₁-C₈ arylalkyl, C₁-C₈ heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r² is hydrogen, F,Cl, Br, I, CF₃, CF₂Cl, CF₂H, CFH₂, CF₂Or⁹, CH₂Or⁹, Or⁹, S(O)mr⁹, Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r³ is hydrogen, F, Cl, Br, I, or⁹, S(O)mr⁹, Nr¹⁰r¹¹, or C₁-C₆ alkyl, C₁-C₆ heteroalkyl, or C₁-C₆ haloalkyl, wherein the alkyl, heteroalkyl, and haloalkyl groups may be optionally substituted;
r⁴ and r⁵ each independently are hydrogen, Or⁹, S(O)mr⁹, Nr¹⁰r¹¹, C(Y)Or¹¹, C(Y)Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁴ and r⁵ taken together can form a saturated or unsaturated three- to seven-membered ring that may be optionally substituted;
r⁶ and r⁷ each independently are hydrogen, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁶ and r⁷ taken together can form a saturated or unsaturated three- to seven-membered ring that may be optionally substituted; or
r⁶ and r⁵ taken together can form a saturated or unsaturated three- to seven-membered ring that may be optionally substituted;
r⁹ is hydrogen, C₁-C₄ alkyl, C₁-C₄ heteroalkyl, C₁-C₄ haloalkyl, F, Cl, Br, I, NO₂, Or⁹, Nr¹⁰r¹¹ or S(O)mr⁹, wherein the alkyl, heteroalkyl, and haloalkyl groups may be optionally substituted;
r⁹ is hydrogen, C₁-C₆ alkyl, C₁-C₆ heteroalkyl, C₁-C₆ haloalkyl, aryl, heteroaryl, arylalkyl, C₂-C₄ alkynyl or C₂-C₈ alkenyl, wherein the alkyl, heteroalkyl, haloalkyl, aryl, arylalkyl, heteroaryl, alkenyl and alkynyl groups may be optionally substituted;
r¹⁰ is hydrogen, C₁-C₆ alkyl, C₁-C₆ heteroalkyl, C₁-C₆ haloalkyl, C(Y)r¹², C(Y)Or¹², aryl, heteroaryl, C₂-C₄ alkynyl, C₂-C₄ alkenyl, arylalkyl, SO₂r¹² or S(O)r¹², wherein the alkyl, heteroalkyl, haloalkyl, aryl, arylalkyl, heteroaryl, alkynyl, and alkenyl groups may be optionally substituted;
r¹¹ is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, arylalkyl, heteroaryl, alkynyl and alkenyl groups may be optionally substituted;
r¹² is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, heteroaryl, arylalkyl, alkynyl and alkenyl groups may be optionally substituted;
r¹³ is hydrogen, C₁-C₈ alkyl, C₃-C₈ cycloalkyl, C₁-C₈ heteroalkyl, C₁-C₈ haloalkyl, aryl, heteroaryl, arylalkyl, heteroarylalkyl, C₂-C₄ alkynyl, or C₂-C₈ alkenyl, wherein the alkyl, cycloalkyl, heteroalkyl, haloalkyl, aryl, arylalkyl, heteroaryl, heteroarylalkyl, alkynyl and alkenyl groups may be optionally substituted; or
r¹³ and r⁴ taken together can form a saturated or unsaturated three- to seven-membered ring that may be optionally substituted;
r¹³ and any two of r⁴ through r⁷ taken together can form a saturated or unsaturated three- to seven-membered ring that may be optionally substituted;
r¹⁴ and r¹⁵ each independently are hydrogen, C₁-C₄ alkyl, C₃-C₈ Cycloalkyl, C₁-C₈ heteroalkyl, C₁-C₈ haloalkyl, aryl, heteroaryl, arylalkyl, C₂-C₈ alkynyl or C₂-C₈ alkenyl, wherein the alkyl, cycloalkyl, heteroalkyl, haloalkyl, aryl, heteroaryl, arylalkyl, alkynyl and alkenyl groups may be optionally substituted;
r^{A} is hydrogen, F, Br, Cl, I, CN, C₁-C₆ alkyl, C₁-C₆ haloalkyl, C₁-C₆ heteroalkyl, Or¹⁶, Nr¹⁶r¹⁷, Sr¹⁶, CH₂r¹⁶, COr¹⁷, -CO₂r¹⁷, CONr¹⁷r¹⁷, SOr¹⁷ or SO₂r¹⁷, wherein the alkyl, haloalkyl, and heteroalkyl groups may be optionally substituted;
r¹⁶ is hydrogen, C₁-C₈ alkyl, C₁-C₈ haloalkyl, C₁-C₈ heteroalkyl, COr¹⁷,CO₂r¹⁷, CONr¹⁷r¹⁷, C₂-C₈ alkynyl, C₂-C₈ alkenyl, aryl or heteroaryl, wherein the alkyl, heteroalkyl, haloalkyl, aryl, heteroaryl, alkynyl and alkenyl groups may be optionally substituted;
r¹⁷ is hydrogen, C₁-C₄ alkyl, C₁-C₄ haloalkyl or C₁-C₄ heteroalkyl, wherein the alkyl, heteroalkyl, and haloalkyl groups may be optionally substituted;
m is 0, 1 or 2;
n is 1 or 2;
V is 0, S or Cr¹⁴r¹⁵;
W is 0, S, NH, Nr¹³, NC(Y)r¹¹, or NSO₂r¹¹;
X and Z each independently are 0, S(O)m, NH, Nr¹¹, NC(Y)r¹¹, NSO₂r¹² or NS(O)r¹²; and
Y is 0 or S, and
wherein, in the formula (α),
u¹ is hydrogen, F, Cl, or C₁-C₃ aliphatic;
u² is selected from the group of hydrogen, F, Cl, Br, C₁-C₄ aliphatic, C₁-C₄ haloaliphatic, and C₁-C₄ heteroaliphatic;
u³ and u⁴ each independently is selected from the group of hydrogen, C₁-C₄ aliphatic, C₁-C₄ haloaliphatic, C₁-C₄ heteroaliphatic, optionally substituted aryl and heteroaryl;
u⁵ and u⁶ each independently is selected from the group of hydrogen, F, Cl, Ou¹⁰, C₁-C₄ aliphatic, C₁-C₄ haloaliphatic, and C₁-C₄ heteroaliphatic;
u⁷ and u⁸ each independently is selected from the group of hydrogen, F, Cl, C₁-C₄ aliphatic, C₁-C₄ haloaliphatic, and C₁-C₄ heteroaliphatic; or
u⁷ and u⁵ taken together form a carbonyl group;
u⁹ is selected from the group of halogen, Ou¹⁰, Su¹⁰, Nu¹⁰, u¹¹, C₁-C₄ haloaliphatic, C₁-C₄ heteroaliphatic, and C₁-C₄ heterohaloaliphatic; and
u¹⁰ and u¹¹ each independently is selected from the group of hydrogen, C₁-C₄ aliphatic, phenyl, and benzyl; and
n is 0 or 1.

### Brief Description of the Drawings

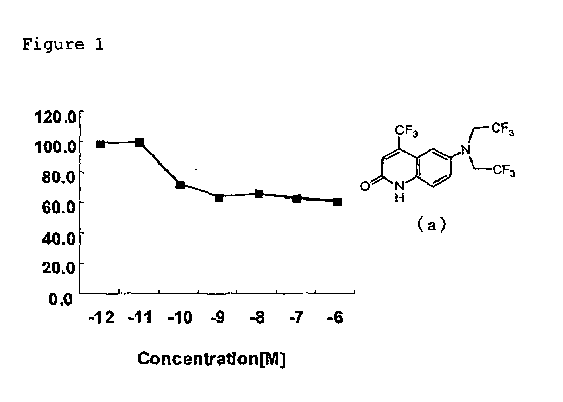

Figure 1 is a diagram showing results of measuring fluctuation caused by a test substance of the formula (a) in the amount of Aβ accumulation in cultured nerve cells (CHP212 cells).

In the diagram, each column represents, starting from the left, a result of measurement obtained in a well to which the test substance has been added so that its final concentration can be 10⁻¹² M (10⁻¹² M), in a well to which the test substance has been added so that its final concentration can be 10⁻¹¹ M (10-¹¹ M), in a well to which the test substance has been added so that its final concentration can be 10⁻¹⁰ M (10⁻¹⁰ M), in a well to which the test substance A has been added so that its final concentration can be 10⁻⁹ M (10⁻⁹ M), in a well to which the test substance has been added so that its final concentration can be 10⁻⁸ M (10⁻⁸ M), in a well to which the test substance has been added so that its final concentration can be 10⁻⁷ M (10⁻⁷ M), and in a well to which the test substance has been added so that its final concentration can be 10⁻⁶ M (10⁻⁶ M). The results are shown in % by setting a result obtained in a control well to which only a solvent (DMSO) has been added as 100%.

### Mode for Carrying Out the Invention

Among compounds contained in the pharmaceutical composition of the present invention (hereinafter, the compounds may be referred to as "the present compounds" and the composition may be referred to as "the present pharmaceutical composition"), compounds represented by the formulas (I) to (IX) (hereinafter, may be referred to as "the present compounds (I) to (IX) ") are described in, for example, J. Med. Chem. 2006, 49, 6143 or National Publication of International Patent Application No. 2003-508387, compounds represented by the formulas (1) to (5) (hereinafter, may be referred to as "the present compounds (1) to (5)") are described in, for example, J. Med. Chem. 2007, 50, 2486 or National Publication of International Patent Application No. 2000-513362, compounds represented by the formulas (A) to (F) (hereinafter, may be referred to as "the present compounds (A) to (F)"), are described in, for example, J. Med. Chem. 2007, 50, 2486 or National Publication of International Patent Application No. 2003-508402, and a compounds represented by the formula (α) (hereinafter, may be referred to as "the present compound (α)"), is described in, for example, J. Med. Chem. 2007, 50, 5049 or National Publication of International Patent Application No. 2007-503398, and thus structures, production methods, etc. of the present compounds are publicly known.

Preferred embodiments of the present compounds include:
as the present compounds (I) to (IX),
   6-propylamino-4-trifluoromethyl-2(1H)-quinolinone;
   6-isopropylamino-4-trifluoromethyl-2(1H)-quinolinone;
   6-isobutylamino-4-trifluoromethyl-2(1H)-quinolinone;
   6-(2,2-dimethylpropyl)amino-4-trifluoromethyl-2(1H)-quinoli none;
   6-cyclopentylamino-4-trifluoromethyl-2(1H)-quinolinone;
   6-(2,2,2-trifluoroethyl)amino-4-trifluoromethyl-2(1H)-quino linone;
   6-(2,2,3,3,3-pentafluoropropyl)amino-4-trifluoromethyl-2(1H )-quinolinone;
   6-(2,2-difluoroethyl)amino-4-trifluoromethyl-2(1H)-quinolin one;
   6-(2-chloro-2,2-difluoroethyl)amino-4-trifluoromethyl-2(1H) -quinolinone;
   6-acetylamino-4-trifluoromethyl-2(1H)-quinolinone;
   6-trifluoroacetylamino-4-trifluoromethyl-2(1H)-quinolinone;
   6-benzoylamino-4-trifluoromethyl-2(1H)-quinolinone;
   6-dimethylacetylamino-4-trifluoromethyl-2(1H)-quinolinone;
   6-dimethylamino-4-trifluoromethyl-2(1H)-quinolinone;
   6-diethylamino-4-trifluoromethyl-2(1H)-quinolinone;
   6-dipropylamino-4-trifluoromethyl-2(1-quinolinone;
   6-dibutylamino-4-trifluoromethyl-2(1H)-quinolinone;
   6-diisobutylamino-4-trifluoromethyl-2(1H)-quinolinone;
   6-(bis-cyclopropylmethyl)amino-4-trifluoromethyl-2(1H)-quin olinone;
   6-(bis-2,2,2-trifluoroethyl)amino-4-trifluoromethyl-2(1H)-q uinolinone;
   6-(bis-2,2,3,3,3-pentafluoropropyl)amino-4-trifluoromethyl-2(1H)-quinolinone;
   6-(bis-2-chloro-2,2-difluoroethyl)amino-4-trifluoromethyl-2 (1H)-quinolinone;
   6-(bis-2-bromoethyl)amino-4-trifluoromethyl-2(1H)-quinolino ne;
   6-(N-2,2,2-trichloroethyl)amino-4-trifluoromethyl-2(1H)-qui nolinone;
   6-(bis-N-2,2,2-trichloroethyl)amino-4-trifluoromethyl-2(1H) -quinolinone;
   6-(N-2,2,2-chlorodifluoroethyl-N-2,2,2-trichloroethyl)amino -4-trifluoromethyl-2(1H)-quinolinone;
   6-(bis-N-2,2-difluoroethyl)amino-4-trifluoromethyl-2(1H)-qu inolinone;
   6-(N-2,2-dichloroethyl-N-2,2,2-trichloroethyl)amino-4-trifl uoromethyl-2(1H)-quinolinone;
   6-(bis-N-2,2-dichloroethyl)amino-4-trifluoromethyl-2(1H)-qu inolinone;
   6-(N-2,2-dichloroethyl-N-2,2-difluoroethyl)amino-4-trifluor omethyl-2(1H)-quinolinone;
   6-(N-2,2-dichloroethyl-N-2,2,2-chlorodifluoroethyl)amino-4-trifluoromethyl-2(1H)-quinolinone;
   6-(N-isopropyl-N-methyl)amino-4-trifluoromethyl-2(1H)-quino linone;
   6-(N-methyl-N-cyclopentyl)amino-4-trifluoromethyl-2(1H)-qui nolinone;
   6-(N-methyl-N-isobutyl)amino-4-trifluoromethyl-2(1H)-quinol inone;
   6-(N-ethyl-N-propyl)amino-4-trifluoromethyl-2(1H)-quinolino ne;
   6-(N-ethyl-N-isopropyl)amino-4-trifluoromethyl-2(1H)-quinol inone;
   6-(N-ethyl-N-1-methylpropyl)amino-4-trifluoromethyl-2(1H)-q uinolinone;
   6-(N-ethyl-N-isobutyl)amino-4-trifluoromethyl-2(1H)-quinoli none;
   6-(N-ethyl-N-2,2-dimethylpropyl)amino-4-trifluoromethyl-2(1 H)-quinolinone;
   6-(N-ethyl-N-cyclopentyl)amino-4-trifluoromethyl-2(1H)-quin olinone;
   6-(N-ethyl-N-1-acetylethyl)amino-4-trifluoromethyl-2(1H)-qu inolinone;
   (±)-6-(N-ethyl-N-1-methyl-2-hydroxypropyl)amino-4-trifluoro methyl-2(1H)-quinolinone;
   6-(N-ethyl-N-2,2,2-trifluoroethyl)amino-4-trifluoromethyl-2 (1H)-quinolinone;
   6-(N-ethyl-N-3-furylmethyl)amino-4-trifluoromethyl-2(1H)-qu inolinone;
   (±)-6-(N-ethyl-N-2,2-dimethoxyisopropyl)amino-4-trifluorome thyl-2(1H)-quinolinone;
   6-(N-isopropyl-N-propyl)amino-4-trifluoromethyl-2(1H)-quino linone;
   6-(N-2-hydroxyethyl-N-propyl)amino-4-trifluoromethyl-2(1H)-quinolinone;
   (±)-6-(N-propyl-N-1-methylbutyl)amino-4-trifluoromethyl-2(1 H)-quinolinone;
   (±)-6-(N-propyl-N-1,2-dimethylpropyl)amino-4-trifluoromethy 1-2(1H)-quinolinone;
   6-(N-propyl-N-isobutyl)amino-4-trifluoromethyl-2(1H)-quinol inone;
   6-(N-propyl-N-cyclopropylmethyl)amino-4-trifluoromethyl-2(1 H)-quinolinone;
   (±)-6-(N-propyl-N-1-methylpropyl)amino-4-trifluoromethyl-2( 1H)-quinolinone;
   6-(N-2-hydroxyethyl-N-isopropyl)amino-4-trifluoromethyl-2(1 H)-quinolinone;
   6-(N-isopropyl-N-cyclopropylmethyl)amino-4-trifluoromethyl-2(1H)-quinolinone;
   6-(N-methyl-N-2,2,2-trifluoroethyl)amino-4-trifluoromethyl-2(1H)-quinolinone;
   6-(N-2,2,2-trifluoroethyl-N-isobutyl)amino-4-trifluoromethy 1-2(1H)-quinolinone;
   6-(N-2,2,2-trifluoroethyl-N-isopropyl)amino-4-trifluorometh yl-2(1H)-quinolinone;
   6-(N-2,2,2-trifluoroethyl-N-cyclopropylmethyl)amino-4-trifl uoromethyl-2(1H)-quinolinone;
   (±)-6-(N-2,2,2-trifluoroethyl-N-1-methylpropyl)amino-4-trif luoromethyl-2(1H)-quinolinone;
   (±)-6-(N-2,2,2-trifluoroethyl-N-2-chloroisopropyl)amino-4-t rifluoromethyl-2(1H)-quinolinone;
   (+)-6-(N-2,2,2-trifluoroethyl-N-2-chloroisopropyl)amino-4-t rifluoromethyl-2(1H)-quinolinone;

   (-)-6-(N-2,2,2-trifluoroethyl-N-2-chloroisopropyl)amino-4-t rifluoromethyl-2(1H)-quinolinone;
   6-(N-2,2,2-trifluoroethyl-N-3-furfuryl)amino-4-trifluoromet hyl-2(1H)-quinolinone;
   6-(N-2,2,2-trifluoroethyl-N-3-thiophenemethyl)amino-4-trifl uoromethyl-2(1H)-quinolinone;
   6-(N-2,2,2-trifluoroethyl-N-3,3-dimethylbutyl)amino-4-trifl uoromethyl-2(1H)-quinolinone;
   6-(N-2,2,2-trifluoroethyl-N-2-thiophenemethyl)amino-4-trifl uoromethyl-2(1H)-quinolinone;
   6-(N-2,2,2-trifluoroethyl-N-2-furfuryl)amino-4-trifluoromet hyl-2(1H)-quinolinone;
   6-(N-butyl-N-2,2,2-trifluoroethyl)amino-4-trifluoromethyl-2 (1H)-quinolinone;
   6-(bis-N,N-benzyl)amino-4-trifluoromethyl-2(1H)-quinolinone
   6-(N-2,2,2-trifluoroethyl-N-cyclobutyl)amino-4-trifluoromet hyl-2(1H)-quinolinone;
   6-(N-2,2,2-trifluoroethyl-N-2,2-dichloroethyl)amino-4-trifl uoromethyl-2(1H)-quinolinone;
   6-(N-2,2,2-trifluoroethyl-N-2-chloroethyl)amino-4-trifluoro methyl-2(1H)-quinolinone:
   6-(N-benzyl-N-2,2,2-trifluoroethyl)amino-4-trifluoromethyl-2(1H)-quinolinone;
   6-(N-4-fluorobenzyl-N-2,2,2-trifluoroethyl)amino-4-trifluor omethyl-2(1H)-quinollnone;
   6-(N-propyl-N-2,2,2-trifluoroethyl)amino-4-trifluoromethyl-2(1H)-quinolinone;
   6-(N-2,2,3,3,3-pentafluoropropyl-N-2,2,2-trifluoroethyl)ami no-4-trifluoromethyl-2(1H)-quinolinone;
   6-diallylamino-4-trifluoromethyl-2(1H)-quinolinone;
   6-(N-isobutyl-N-allyl)amino-4-trifluoromethyl-2(1H)-quinoli none;
   6-(N-isopropyl-N-allyl)amino-4-trifluoromethyl-2(1H)-quinol inone;
   6-(N-allyl-N-2,2,2-trifluoroethyl)amino-4-trifluoromethyl-2 (1H)-quinolinone;
   6-allylamino-4-trifluoromethyl-2(1H)-quinolinone;
   6-(N-allyl-N-cyclopropylmethyl)amino-4-trifluoromethyl-2(1H )-quinolinone;
   6-(N-allyl-N-2,2,2-trifluoroacethyl)amino-4-trifluoromethyl -2(1H)-quinolinone;
   6-(N-2,2,2-trifluoroethyl-N-2,2,2-trifluoroacetyl)amino-4-t rifluoromethyl-2(1H)-quinolinone;
   6-(N-allyl-N-propyl)amino-4-trifluoromethyl-2(1H)-quinolino ne;
   (±)-6-(N-2-hydroxyisopropyl-N-2,2,2-trifluoroethyl)amino-4-trifluoromethyl-2(1H)-quinolinone;
   (±)-6-(N-isobutyl-N-2,2,2-trifluoroisopropyl)amino-4-triflu oromethyl-2(1H)-quinolinone;
   6-(N-2,2-difluoroethyl-N-2,2,2-trifluoroethyl)amino-4-trifl uoromethyl-2(1H)-quinolinone;
   6-(N-2,2-dimethylpropyl-N-2,2,2-trifluoroethyl)amino-4-trif luoromethyl-2(1H)-quinolinone;
   6-(N-2,2-difluoro-2-chloroethyl-N-2,2,2-trifluoroethyl)amin o-4-trifluoromethyl-2(1H)-quinolinone;
   6-(N-2,2-difluoro-2-chloroethyl-N-2,2-difluoroethyl)amino-4 -trifluoromethyl-2(1H)-quinolinone;
   6-(N-2,2,2-trifluoroethyl-N-methylsufonyl)amino-4-trifluoro methyl-2(1H)-quinolinone;
   1-methyl-6-(N-propyl-N-isobutyl)amino-4-trifluoromethyl-2(1 H)-quinolinone;
   1-methyl-6-(bis-2,2,2-trifluoroethyl)amino-4-trifluoromethy 1-2(1H)-quinolinone;
   1-ethyl-6-(bis-2,2,2-trifluoroethyl)amino-4-trifluoromethyl -2(1H)-quinolinone;
   6-(N-2,2,2-trifluoroethyl)amino-4-trifluoromethyl-2(1H)-qui nolinone;
   6-(bis-N,N-2,2,2-trifluoroethyl)amino-4-trifluoromethyl-2(1 H)-thioquinolinone;
   (±)-6-(N-2,2,2-trifluoroethyl-N-2,2,2-trifluoroisopropyl)am ino-4-trifluoromethyl-2(1H)-quinolinone;
   (+)-6-(N-2,2,2-trifluoroethyl-N-2,2,2-trifluoroisopropyl)am ino-4-trifluoromethyl-2(1H)-quinolinone;
   (-)-6-(N-2,2,2-trifluoroethyl-N-2,2,2-trifluoroisopropyl)am ino-4-trifluoromethyl-2(1H)-quinolinone;
   6-methoxythiocarbonylmercapto-4-trifluoromethyl-2(1H)-quino linone;
   6-(1,1-dimethyl-2-propynyl)amino-4-trifluoromethyl-2(1H)-qu inolinone;
   6-tert-butylamino-4-trifluoromethyl-2(1H)-quinolinone;
   6-tert-butylamino-2-isopropyloxy-4-trifluoromethylquinoline ; 6-(1-Piperdinyl)-9-trifluoromethyl-2(1H)-quinolinone;
   6-(1-Pyrrolidinyl)-4-trifluoromethyl-2(1H)-quinolinone;
   6-(1-morpholino-4-trifluoromethyl-2(1H)-quinolinone;
   (±)-6-(2-methyl-1-piperidino)-4-trifluoromethyl-2(1H)-quino linone;
   (+)-6-(2-methyl-1-piperidino)-4-trifluoromethyl-2(1H)-quino linone;
   (-)-6-(2-methyl-1-piperidino)-4-trifluoromethyl-2(1H)-quino linone;
   6-(N-phenylamino)-4-trifluoromethyl-2(1H)-quinolinone;
   6-(N-phenyl-N-ethylamino)-4-trifluoromethyl-2(1H)-quinolino ne;
   6-(N-phenyl-N-ethylamino)-4-trifluoromethyl-2-isopropyloxyq uinoline;
   6-(N-phenyl-N-2,2,2-trifluoroethylamino)-4-trifluoromethyl-2(1H)-quinolinone;
   (±)-6-(3-methyl-1-piperidino)-4-trifluoromethyl-2(1H)-quino linone;
   6-(4-methyl-1-piperidino)-4-trifluoromethyl-2(1H)-quinolino ne;
   6-(cis-3,5-dimethyl-1-piperidino)-4-trifluoromethyl-2(1H)-q uinolinone;
   6-(2,6-cis-dimethyl-1-piperidino)-4-trifluoromethyl-2(1H)-q uinolinone;
   6-(2,6-trans-dimethyl-1-piperidino)-4-trifluoromethyl-2(1H) -quinolinone;
   (±)-6-(2-methyl-1-pyrrolidino)-4-trifluoromethyl-2(1H)-quin olinone;
   6-(2,5-cis-dimethyl-1-pyrrolidino)-4-trifluoromethyl-2(1H)-quinolinone;
   (±)-6-(2,5-trans-dimethyl-1-pyrrolidino)-4-trifluoromethyl-2(1H)-qui.nolinone;
   6-(1-azepano)-4-trifluoromethyl-2(1H)-quinolinone;
   (±)-6-(2-hydroxymethyl-1-piperidino)-4-trifluoromethyl-2(1H )-quinolinone;
   6-(2,5-cis-dimethyl-1-pyrrolino)-4-trifluoromethyl-2(1H)-qu inolinone;
   (±)-6-(2-propyl-1-piperidino)-4-trifluoromethyl-2(1H)-quino linone;
   (±)-6-(2-methoxymethyl-1-piperidino)-4-trifluoromethyl-2(1H )-quinolinone;
   (±)-6-(2-ethyl-1-piperidino)-4-trifluoromethyl-2(1H)-quinol inone;
   6-(1-cycloheptylamino)-4-trifluoromethyl-2(1H)-quinolinone;
   (±)-6-(2-ethoxycarbonyl-1-piperidino)-4-trifluoromethyl-2(1 H)-quinolinone;
   (±)-6-(2-isopropyl-1-pyrrolidino)-4-trifluoromethyl-2(1H)-q uinolinone;
   (±)-6-(2-hydroxycarbonyl-1-piperidino)-4-trifluoromethyl-2( 1H)-quinolinone;
   6-(3,5-cis-dimethyl-1-piperazino)-4-trifluoromethyl-2(1H)-q uinolinone;
   (±)-6-(2-benzyl-1-pyrrolidino)-4-trifluoromethyl-2(1H)-quin olinone;
   (±)-6-(2-methyl-2-oxo-1-pyrrolidino)-4-trifluoromethyl-2(1H )-quinolinone;
   (±)-6-(2-(2-hydroxyethyl)-1-piperidino)-4-trifluoromethyl-2 (1H)-quinolinone;
   (±)-6-(3-hydroxy-1-pyrrolidino)-4-trifluoromethyl-2(1H)-qui nolinone;
   (±)-6-(3-acetyloxy-1-pyrrolidino)-4-trifluoromethyl-2(1H)-q uinolinone;
   (±)-6-(3-hydroxy-1-pyrrolidino)-4-trifluoromethyl-2(1H)-qui nolinone;
   6-(1-indolino)-4-trifluoromethyl-2(1H)-quinolinone;
   6-(1-tetrahydroquinolino)-4-trifluoromethyl-2(1H)-quinolino ne;
   6-(2-tetrahydroisoquinolino)-4-trifluoromethyl-2(1H)-quinol inone;
   (±)-6-(1,3,3-trimethyl-6-azabicyclo[3.2.1]octanyl-6-)-4-tri fluoromethyl-2(1H)-quinolinone;
   (±)-6-(2-trifluoromethyl-5-cis-methyl-1oxazolidino)-4-trif luoromethyl-2(1H)-quinolinone;
   (±)-6-(2-trifluoromethyl-5-trans-methyl-1-oxazolidino)-4-tr ifluoromethyl-2(1H)-quinolinone;
   6-N-(1-hydroxyisopropyl)amino-4-trifluoromethyl-2(1H)-quino linone;
   (±)-6-(2-trifluoromethyl-5-cis-ethyl-1-oxazolidino)-4-trifl uoromethyl-2(1H)-quinolinone;
   (±)-6-(2-trifluoromethyl-5-trans-ethyl-1-oxazolidino)-4-tri fluoromethyl-2(1H)-quiriolinone;
   (±)-6-(5-methyl-1-oxazolidino)-4-trifluoromethyl-2(1H)-quin olinone;
   6-(2,5-dimethyl-1-pyrrolyl)-4-trifluoromethyl-2(1H)-quinoli none;
   6-(N-2,2,2-trifluoroethyl-N-3,3,3-trifluoropropyl)amino-4-t rifluoromethyl-2(1H)-quinolinone;
   6-(N-3,3,3-trifluoropropyl)amino-4-trifluoromethyl-2-isopro pyloxyquinoline;
   6-bis-N,N-thiomethoxymethylamino-4-trifluoromethyl-2(1H)-qu inolinone;
   6-bis-N,N-thiomethoxymethylamino-4-trifluoromethyl-2-thiome thoxymethyloxyquinoline;
   (±)-6-(2,5-trans-diethyl-1-pyrrolidino)-4-trifluoromethyl-2 (1H)-quinolinone;
   6-(2,5-cis-diethyl-1-pyrrolidino)-4-trifluoromethyl-2(1H)-q uinolinone;
   (±)-6-(2,5-trans-dipropyl-1-pyrrolidino)-4-trifluoromethyl-2(1H)-quinolinone;
   6-(2,5-cis-dipropyl-1-pyrrolidino)-4-trifluoromethyl-2(1H)-quinolinone;
   6-(2,5-dipropyl-1-pyrrolo)-4-trifluoromethyl-2(1H)-quinolin one;
   6-(2,5-cis-dibutyl-1-pyrrolidino)-4-trifluoromethyl-2(1H)-q uinolinone;
   (±)-6-(2,5-trans-dibutyl-1-pyrrolidino)-4-trifluoromethyl-2 (1H)-quinolinone;
   6-(2,6-cis-diethyl-1-piperidino)-4-trifluoromethyl-2(1H)-qu inolinone;
   (±)-6-(2,6-trans-diethyl-1-piperidino)-4-trifluoromethyl-2( 1H)-quinolinone;
   6-(2,6-cis-dimethyl-1-piperidino)-4-trifluoromethyl-2(1H)-q uinolinone;
   (±)-6-(2,6-trans-dimethyl-1-piperidino)-4-trifluoromethyl-2 (1H)-quinolinone;
   6-(N-propyl-N-2,2,2-trifluoroethyl)amino-4-methyl-2(1H)-qui nolinone;
   6-(bis-2,2,2-trifluoroethyl)amino-4-methyl-2(1H)-quinolinon e;
   6-(2,5-dimethyl-1-pyrrolyl)-4-methyl-2(1H)-quinolinone; (±)-6-(2,5-trans-dimethyl-1-pyrrolidino)-4-methyl-2(1H)-qui nolinone;
   6-(2,5-cis-dimethyl-1-pyrrolidino)-4-methyl-2(1H)-quinolino ne;
   6-(N-isobutyl-N-2,2,2-trifluoroethyl)amino-4-methyl-2(1H)-q uinolinone;
   6-(N-2,2,2-chlorodifluoroethyl)amino-4-methyl-2(1H)-quinoli none;
   6-(bis-N,N-2,2,2-chlorodifluoroethyl)amino-4-methyl-2(1H)-q uinolinone;
   6-(N-2,2,2-chlorodifluoroethyl-N-2,2,2-trifluoroethyl)amino -4-methyl-2(1H)-quinolinone;
   6-N-ethylamino-4-methyl-2(1H)-quinolinone;
   6-(N-ethyl-N-2,2,2-trifluoroethyl)amino-4-methyl-2(1H)-quin olinone;
   6-N,N-diethylamino-4-methyl-2(1H)-quinolinone;
   6-(bis-2,2,2-trifluoroethyl)amino-4-ethyl-2(1H)-quinolinone ;
   6-(bis-2,2,2-trifluoroethyl)amino-4-isopropyl-2(1H)-quinoli none;
   7-fluoro-6-(bis-trifluoroethyl)amino-4-trifluoromethyl-2(1H )-quinolinone;
   8-fluoro-6-(bis-2,2,2-trifluoroethyl)amino-4-trifluoromethy 1-2(1H)-quinolinone;
   8-fluoro-4-trifluoromethyl-2(1H)-quinolinone;
   6-amino-8-fluoro-4-trifluoromethyl-2(1H)-quinolinone;
   8-fluoro-6-(N-2,2,2-trifluoroethyl)amino-4-trifluoromethyl-2(1H)-quinolinone;
   8-fluoro-6-(N-2,2,2-trifluoroethyl-N-isopropyl)amino-4-trif luoromethyl-2(1H)-quinolinone;
   3-fluoro-6-(2,2,2-trifluoroethyl)amino-4-trifluoxomethyl-2( 1H)-quinolinone;
   3-fluoro-6-(bis-2,2,2-trifluorofluoroethyl)amino-4-trifluor omethyl-2(1H)-quinolinone;
   6-(bis-isobutylamino)-4-methyl-2(1H)-quinolinone;
   3-fluoro-6-(N-methyl-N-2,2,2-trifluorofluoroethyl)amino-4-t rifluoromethyl-2(1H)-quinolinone;
   7-bromo-6-isopropylamino-4-trifluoromethyl-2(1H)-quinolinon e;
   6-(bis-N,N-2,2,2-trifluoroethyl)amino-4-hydroxy-2(1H)-quino linone;
   6-(bis-N,N-2,2,2-trifluoroethyl)amino-4-methoxy-2(1H)-quino linone;
   6-(bis-N,N-2,2,2-trifluoroethyl)amino-4-difluoromethyl-2(1H )-quinolinone;
   6-(bis-N,N-2,2,2-trifluoroethyl)amino-2(1H)-quinolinone; 4-chloro-6-(bis-N,N-2,2,2-trifluoroethyl)amino-2(1H)-quinol inone;
   (R)-6-(2-hydroxymethyl-1-pyrrolidino)-4-trifluoromethyl-2(1 H)-quinolinone;
   (R)-6-(2-methoxycarbonyl-1-pyrrolidino)-4-trifluoromethyl-2 -isopropyloxyquinoline;
   (R)-6-(2-methoxymethyl-1-pyrrolidino)-4-trifluoromethyl-2(1 H)-quinolinone;
   (±)-6-(2-chloromethyl-1-piperidino)-4-trifluoromethyl-2(1H) -quinolinone;
   (±)-6-(2-cyanothiomethyl-1-piperidino)-4-trifluoromethyl-2( 1H)-quinolinone;
   (±)-6-(2-Thiomethoxymethyl-1-piperidino)-4-trifluoromethyl-2(1H)-quinolinone;
   (±)-6-(2-cyanomethyl-1-piperidino)-4-trifluoromethyl-2(1H)-quinolinone;
   (±)-6-(2-bromomethyl-1-piperidino)-4-trifluromethyl-2(1H)-quinolinone;
   (±)-6-(2-iodomethyl-1-piperidino)-4-trifluoromethyl-2(1H)-q uinolinone;
   (+)R-6-(2-iodomethyl-1-pyrrolidino)-4-trifluoromethyl-2(1H) -quinolinone;
   (±)-6-(2-fluoromethyl-1-piperidino)-4-trifluoromethyl-2(1H)
   -quinolinone; (+)S-6-(2-chloromethyl-1-piperidino)-4-trifluoromethyl-2(1H )-quinolinone;
   (-)R-6-(2-chloromethyl-1-piperidino)-4-trifluoromethyl-2(1H )-quinolinone;
   (+)R-6-(2-chloromethyl-1-pyrrolidino)-4-trifluoromethyl-2(1 H)-quinolinone;
   (-)S-6-(2-chloromethyl-1-pyrrolidino)-4-trifluoromethyl-2(1 H)-quinolinone;
   R-6-(2-difluoromethyl-1-pyrrolidino)-4-trifluoromethyl-2(1H )-quinolinone;
   (±)-6-(21-(11-hydroxy-2,2,2-trifluoroethyl)-1-piperidino)-4 -trifluoromethyl-2(1H)-quinolinone;
   (±)-6-(21-(lu-hydroxy-2,2,2-trifluoroethyl)-1-piperidino)-4 -trifluoromethyl-2(1H)-quinolinone;
   (±)-6-(2-formyl-1-piperidino)-4-trifluoromethyl-2(1H)-quino linone;
   (±)-6-(2-difluoromethyl-1-piperidino)-4-trifluoromethyl-2(1 H)-quinolinone;
   (±)-6-(2-aminomethyl-1-piperidino)-4-trifluoromethyl-2(1H)-quinolinone;
   (R)-6-(2-vinyl-l-pyrrolidino)₋4-trifluoromethyl-2(1H)-quino linone;
   (R)-6-(2-formyl-1-pyrrolidino)-4-trifluoromethyl-2(1H)-quin olinone;
   (±)-6-(2-vinyl-1-piperidino)-4-trifluoromethyl-2(1H)-quinol inone;
   (±)-6-(2-benzyloxyethyl-1-piperidino)-4-trifluoromethyl-2(1 H)-quinolinone;
   (±)-6-(2-(2,2-difluoroethyl)-l-piperidino)-4-trifluoromethy 1-2(1H)-quinolinone;
   (±)-6-(2-trifluoroacetamidomethyl-1-piperidino)-4-trifluoro methyl-2-(1H)-quinolinone;
   (±)-6-(2-(2-ethoxyethyl)-1-piperidino)-4-trifluoromethyl-2( 1H)-quinolinone;
   (±)-6-(2-(4-trifluoromethyl)benzyloxyethyl-1-piperidino)-4-trifluoromethyl-2(1H)-quinolinone;
   (+)-6-(2R-(1R-hydroxy-2,2,2-trifluoroethyl)-1-piperidino)-4 -trifluoromethyl-2(1H)-quinolinone;
   (-)6-(2R-(1S-hydroxy-2,2,2-trifluoroethyl)-1-pyrrolidino)-4 -trifluoromethyl-2(1H)-quinolinone;
   6-(2S-(1R-hydroxy-2,2,2-trifluoroethyl)-1-pyrrolidino)-4-tr ifluoromethyl-2(1H)-quinolione;
   6-(2S-(1S-hydroxy-2,2,2-trifluoroethyl)-1-pyrrolidino)-4-tr ifluoromethyl-2(1H)-quinolinone;
   (±)-6-(21-(11-hydroxyethyl)-1-piperidino)-4-trifluoromethyl -2(1H)-quinolinone;
   (±)-6-(21-(1u-hydroxyethyl)-1-piperidino)-4-trifluoromethyl -2(1H)-quinolinone:
   (-)-6-(2S-(15-hydroxy-2,2,2-trifluoroethyl)-1-piperidino)-4 -trifluoromethyl-2(1H)-quinolinone;
   (+)-6-(2R-(1R-hydroxy-2,2,2-trifluoroethyl)-1-piperidino)-4 -trifluoromethyl-2(1H)-quinolinone;
   (+)-6-(2R-(1S-hydroxy-2,2,2-trifluoroethyl)-1-piperidino)-4 -trifluoromethyl-2(1H)-quinolinone;
   (-)-6-(2S-(1R-hydroxy-2,2,2-trifluoroethyl)-1-piperidino)-4 -trifluoromethyl-2(1H)-quinolinone;
   (±)-6-(21-(11-acetyloxyethyl)-1-piperidino)-4-trifluorometh yl-2(1H)-quinolinone;
   (±)-6-(21-(1u-acetyloxyethyl)-1-piperidino)-4-trifluorometh yl-2(1H)-quinolinone;
   (±)-6-(21-(1u-methoxy-2,2,2-trifluoroethyl)-1-piperidino)-4 -trifluoromethyl-2(1H)-quinolinone;
   (±)-6-(21-(11--methoxy-2,2,2-trifluoroethyl)-1-piperidino)-4 -trifluoromethyl-2(1H)-quinolinone;
   7-methoxy-6-(N-methyl-N-2,2,2-trifluoroethyl)amino-4-triflu oromethyl-2(1H)-quinolinone;
   7-methoxy-6-(N-2,2,2-trifluoroethyl)amino-4-trifluoromethyl -2(1H)-quinolinone;
   7-methoxy-6-(N-ethyl-N-2,2,2-trifluoroethyl)amino-4-trifluo romethyl-2(1H)-quinolinone;
   7-hydroxy-6-(2,2,2-trifluoroethyl)amino-4-trifluoromethyl-2 (1H)-quinolinone;
   6-(N-cyclopropylmethyl-N-2,2,2-trifluoroethyl)amino-7-metho xy-4-trifluoromethyl-2(1H)-quinolinone;
   6-(N-cyclopropylmethyl-N-2,2,2-trifluoroethyl)amino-7-hydro xy-4-trifluoromethyl-2(1H)-quinolinone;
   6-(N-isobutyl-N-2,2,2-trifluoroethyl)amino-7-methoxy-4-trif luoromethyl-2(1H)-quinolinone;
   6-(N-isobutyl-N-2,2,2-trifluoroethyl)amino-7-hydroxy-4-trif luoromethyl-2(1H)-quinolinone;
   6-(bis-2,2,2-trifluoroethyl)amino-4-trifluoromethylcoumarin;
   (±)-3,4-dihydro-6-(bis-2,2,2-trifluoroethyl)amino-4-trifluo romethylcoumarin;
   6-(2,2,2-trifluoroethyl)amino-4-trifluoromethylcoumarin;
   6-(N-isopropyl-N-2,2,2-trifluoroethyl)amino-4-trifluorometh ylcoumarin;
   6-N-isobutylamino-4-trifluoromethylcoumarin;
   6-N,N-diethylamino-4-trifluoromethylcoumarin;
   6-N,N-dipropylamino-4-trifluoromethylcoumarin;
   6-N-propylamino-4-trifluoromethylcoumarin;
   6-(N-isobutyl-N-propylamino)-4-trifluoromethylcoumarin;
   6-(N-2,2,2-trifluoroethyl-N-propylamino)-4-trifluoromethylc oumarin;
   1,4-dihydro-4,4-dimethyl-6-methylamino-1,3-benzo[d]oxazin-2 -one;
   1,4-dihydro-4,4-dimethyl-6-dimethylamino-1,3-benzo[d]oxazin -2-one;
   1,4-dihydro-4,4-dimethyl-6-dipropylamino-1,3-benzo[d]oxazin -2-one;
   1,4-dihydro-4,4-dimethyl-6-(bis-N,N-2,2,2-trifluoroethyl)am ino-1,3-benzo[d]oxazin-2one;
   1,4-dihydro-4,4-dimethyl-6-(N-2,2,2-trifluoroethyl)amino-1, 3-benzo[d]oxazin-2-one;
   (±)-1,4-dihydro-4-methyl-6-diallylamino-1,3-benzo[d]oxazin-2-one;
   6-diallylamino-3,4-dihydro-4,4-dimethyl-2(1H)-quinolinone;
   3,4-dihydro-4,4-dimethyl-6-dipropylamino-2(1H)-quinolinone;
   3,4-dihydro-4,4-dimethyl-6-propylamino-2(1H)-quinolinone;
   3,4-dihydro-4,4-dimethyl-6-(N-2,2,2-trifluoroethyl)amino-2( 1H)-quinolinone;
   3,4-dihydro-4,4-dimethyl-6-(bis-N,N-2,2,2-trifluoroethyl)am ino-2(1H)-quinolinone;
   3,4-dihydro-6-(N-2,2,2-trifluoroethyl)amino-2(1H)-quinolino ne;
   3,4-dihydro-6-(bis-N,N-2,2,2-trifluoroethyl)amino-2(1H)-qui nolinone;
   5-(bis-N,N-2,2,2-trifluoroethyl)amino-3,3-spirocyclohexyl-2 -indolone;
   7-(bis-N,N-2,2,2-trifluoroethyl)amino-1,4-benzoxazin-3(4H)-one;
   6-(bis-N,N-2,2,2-trifluoroethyl)amino-2,4-dichloroquinoline;
   7-propylamino-4-trifluoromethyl-2(1H)-quinolinone;
   7-isopropylamino-4-trifluoromethyl-2(1H)-quinolinone;
   7-(2,2-dimethylpropyl)amino-4-trifluoromethyl-2(1H)-quinoli none;
   7-(2-methylpropyl)amino-4-trifluoromethyl-2(1H)-quinolinone ;
   7-benzylamino-9-trifluoromethyl-2(1H)-quinolinone;
   7-(2,2,3,3,3-pentafluoropropyl)amino-4-trifluoromethyl-2(1H )-quinolinone;
   7-butylamino-4-trifluoromethyl-2(1H)-quinolinone;
   7-ethylamino-4-trifluoromethyl-2(1H)-quinolinone;
   7-(N-2,2,2-trifluoroethyl)amino-9-trifluoromethyl-2(1H)qui nolinone;
   7-cyclohexylamino-4-trifluoromethyl-2(1H)-quinolinone;
   7-cyclopentylamino-4-trifluoromethyl-2(1H)-quinolinorie;
   7-cyclobutylamino-4-trifluoromethyl-2(1H)-quinolinone;
   7-(2-hydroxy-2-methylpropionyl)amino-4-trifluoromethyl-2(1H )-quinolinone;
   7-(trifluoroacetamido)-4-trifluoromethyl-2(1H)-quinolinone;
   1-methyl-7-methylamino-4-trifluoromethyl-2(1H)-quinolinorie;
   1-methyl-7-(N-methyl-N-isopropylamino)-4-trifluoromethyl-2( 1H)-quinolinone;
   1-methyl-7-(2,2,2-trifluoromethyl)amino-4-trifluoromethyl-2 (1H)-quinolinone;
   3-fluoro-7-(2,2,2-trifluoromethyl)amino-4-trifluoromethyl-2 (1H)-quinolinone;
   3-fluoro-7-isopropylamino-4-trifluoromethyl-2(1H)-quinolino ne;
   3-fluoro-7-cyclopentylamino-4-trifluoromethyl-2(1H)-quinoli none;
   3-fluoro-7-cyclohexylamino-4-trifluoromethyl-2(1H)-quinolin one;
   3-fluoro-7-cyclobutylamino-4-trifluoromethyl-2(1H)-quinolin one;
   3-fluoro-7-propylamino-4-trifluoromethyl-2(1H)-quinolinone;
   3-fluoro-1-methyl-7-(N-methyl-N-isopropyl)amino-4-trifluoro methyl-2(1H)-quinolinone;
   3-fluoro-1-methyl-7-propylamino-4-trifluoromethyl-2(1H)-qui nolinone;
   6-fluoro-7-propylamino-4-trifluoromethyl-2(1H)-quinolinone;
   6-fluoro-7-isobutylamino-4-trifluoromethyl-2(1H)-quinolinon e;
   6-fluoro-1-methyl-7-propylamino-4-trifluoromethyl-2(1H)-qui nolinone;
   6-fluoro-1-methyl-7-(N-methyl-N-propylamino)-4-trifluoromet hyl-2(1H)-quinolinone;
   7-isobutylamino-6-methyl-4-trifluocomethyl-2(1H)-quinolinon e;
   7-propylamino-6-methyl-4-trifluoromethyl-2(1H)-quinolinone;
   7-(1,1-dimethyl-3-oxobutyl)amino-4-trifluoromethyl-2(1H)-qu inolinone;
   7-(1,1,3-trimethyl-3-hydroxybutyl)amino-4-trifluoromethyl-2 (1H)-quinolinone;
   7-(1,1,3-trimethyl-3-butenylamino)-4-trifluoromethyl-2(1H)-quinolinone;
   7-(1-Phenylaminocarbonylisopropyl)amino-4-trifluoromethyl-2 (1H)-quinolinone;
   7-(2-hydroxy-1,1-dimethylethyl)amino-4-trifluoromethyl-2(1H )-quinolinone;
   7-(N-1-formylisopropyl)amino-4-trifluoromethyl-2(1H)-quinol inone;
   7-(1,1-dimethylallyl)amino-4-trifluoromethyl-2(1H)-quinolin one;
   7-(1,1-dimethylpropyl)amino-4-trifluoromethyl-2(1H)-quinoli none;
   7-(1-methyl-1-acetylenylpropyl)aminol-4-(trifluoromethyl)-2 (1H)-quinolinone;
   7-(1-ethyl-1-methylpropyl)amino-4-(trifluoromethyl)-2(1H)-q uinolinone;
   8-methyl-7-(3-methyl-2-butenyl)amino-4-trifluoromethyl-2(1H )-quinolinone;
   8-methyl-7-(3-methylbutyl)amino-4-trifluoromethyl-2(1H)-qui nolinone;
   8-methyl-7-propylamino-4-(trifluoromethyl)-2(1H)-quinolinon e;
   8-methyl-7-isobutylamino-4-(trifluoromethyl)-2(1H)-quinolin one;
   7-amino-6-(2,2,2-trifluoroethoxy)-4-trifluoromethyl-2(1H)-q uinolinone;
   7-isobutylmino-6-(2,2,2-trifluoroethoxy)-4-trifluoromethyl-2(1H)-quinolinone;
   7-(2-Picolylamino)-6-(2,2,2-trifluoroethoxy)-4-trifluoromet hyl-2(1H)-quinolinone;
   7-amino-6-cyclohexyl-4-trifluoromethyl-2(1H)-quinolinone;
   6-ethyl-7-(2,2,2-trifluoroethyl)amino-4-trifluoromethyl-2(1 H)-quinolinone;
   6-isobutyl-7-methylamino-4-trifluoromethyl-2(1H)-quinolinon e;
   7-(1-morpholino)-4-trifluoromethyl-2(1H)-quinolinone;
   6-(bis-N,N-2,2,2-trifluoroethyl)amino-5-methoxy-4-trifluoro methyl-2(1H)-quinolinone;
   6-(N-2,2,2-trifluoroethyl)amino-5-propyloxy-4-trifluorometh yl-2(1H)-quinolinone;
   6-(bis-N,N-2,2,2-trifluoroethyl)amino-5-propyloxy-4-trifluo romethyl-2(1H)-quinolinone;
   6-(N-2,2,2-trifluoroethyl)amino-5-ethoxy-4-trifluoromethyl-2(1H)-quinolinone;
   6-(bis-N,N-2,2,2-trifluoroethyl)amino-5-ethoxy-4-trifluorom ethyl-2(1H)-quinolinone;
   6-(N-2,2,2-trifluoroethyl)amino-5-(3,3,3-trifluoropropyloxy )-4-trifluoromethyl-2(1H)-quinolinone;
   6-(N-2,2,2-trifluoroethyl)amino-5-chloro-4-trifluoromethyl-2(1H)-quinolinone;
   6-(bis-N,N-2,2,2-trifluoroethyl)amino-5-chloro-4-trifluorom ethyl-2(1H)-quinolinone;
   6-cyclohexyl-4-trifluoromethyl-2(1H)-quinolinone;
   6-(1-trans-Propenyl)-4-trifluoromethyl-2(1H)-quinolinone;
   6-cyclohexyl-3-fluoro-4-trifluoromethyl-2(1H)-quinolinone;
   6-(1-hydroxy-3,3,5,5-tetramethyl)cyclohexyl-4-trifluorometh yl-2(1H)-quinolinone;
   6-(3,3,5,5-tetramethyl)cyclohexenyl-4-trifluoromethyl-2(1H) -quinolinone;
   6-(5,5-dimethylcyclopentenyl)-4-trifluoromethyl-2(1H)-quino linone;
   6-(2,2-dimethylcyclopentyl)-4-trifluoromethyl-2(1H)-quinoli none;
   6-(1-hydroxycyclohexyl)-4-trifluoromethyl-2(1H)-quinolinone ;
   6-cyclohexenyl-4-trifluoromethyl-2(1H)-quinolinone;
   6-cyclohexyl-4-trifluoromethyl-2(1H)-thioquinolinone;
   6-cyclopentenyl-4-trifluoromethyl-2(1H)-quinolinone;
   6-cycloheptenyl-4-trifluoromethyl-2(1H)-quinolinone;
   6-cyclohexenyl-3-fluoro-4-trifluoromethyl-2(1H)-quinolinone ;
   6-cyclohexyl-7-methoxy-4-trifluoromethyl-2(1H)-quinolinone;
   6-cyclopentyl-3-fluoro-4-trifluoromethyl-2(1H)-quinolinone;
   (Z)-6-(1-propyl-1)butenyl-4-trifluoromethyl-2(1H)-quinolino ne;
   (E)-6-(1-propyl-1)butenyl-4-trifluoromethyl-2(1H)-quinolino ne;
   6-(1-propyl)butyl-4-trifluoromethyl-2(1H)-quinolinone; (E)-6-(1-methyl-1-)butenyl-4-trifluoromethyl-2(1H)-quinolin one;
   (Z)-6-(1-methyl-1-)butenyl-4-trifluoromethyl-2(1H)-quinolin one;
   (±)-6-(1-methyl)butyl-4-trifluoromethyl-2(1H)-quinolinone;
   (E)-6-(1-ethyl-1-)propenyl-4-trifluoromethyl-2(1H)-quinolin one;
   (Z)-6-(1-ethyl-1-)propenyl-4-trifluoromethyl-2(1H)-quinolin one;
   6-(1-ethyl)propyl-4-trifluoromethyl-2(1H)-quinolinone;
   6-(1-isopropyl-2-methyl-1-)propenyl-4-trifluoromethyl-2(1H) -quinolinone;
   6-(1-isopropyl-2-methyl)propyl-4-trifluoromethyl-2(1H)-quin olinone;
   (Z)-6-(1-isobutyl-3-methyl-1-)butenyl-4-trifluoromethyl-2(1 H)-quinolinone;
   (E)-6-(1-isobutyl-3-methyl-1-)butenyl-4-trifluoromethyl-2(1 -quinolinone;
   6-(1-isobutyl-3-methyl)butyl-4-trifluoromethyl-2(1H)-quinol inone;
   6-(1-propyl)butyl-4-trifluoromethyl-2(1H)-thioquinolinone;
   6-(3-oxo-1-)cyclopentenyl-4-trifluoromethyl-2(1H)-quinolino ne;
   6-(3-oxo-1-)cyclohexenyl-4-trifluoromethyl-2(1H)-quinolinon e;
   6-(3-oxo-1-)cyclopentenyl-3-methyl-4-difluoromethyl-2(1H)-q uinolinone;
6-(3-oxo-1-)cyclohexenyl-3-methyl-4-difluoromethyl-2(1H)-qu inolinone;
   (±)-6-(3-hydroxy-1-)cyclohexenyl-4-trifluoromethyl-2(1H)-qu inolinone;
   6-(1-hydroxy-1,1-diphenyl)methyl-4-trifluoromethyl-2(1H)-qu inolinone;
   6-(3-hydroxy-3-methyl-1-)butynyl-4-trifluoromethyl-2(1H)-qu inolinone;
   6-(1-hydroxy)cyclopentyl-1,4-dihydro-4,4-dimethyl-1,3-benzo [d]oxazin-2-one;
   6-(cyclopentenyl)-1,4-dihydro-4,4-dimethyl-1,3-benzo[d]oxaz in-2-one;
   6-cyclopentyl-1,4-dihydro-4,4-dimethyl-1,3-benzo[d]oxazin-2 -one;
   6-(1-hydroxy)cyclohexyl-1,4-dihydro-4,4-dimethyl-1,3-benzo[ d]oxazin-2-one;
   6-(1-cyclohexenyl)-1,4-dihydro-4,4-dimethyl-1,3-benzo[d]oxa zin-2-one;
   6-cyclohexyl-1,4-dihydro-4,4-dimethyl-1,3-benzo[d]oxazin-2-one;
   6-(1-hydroxy)cycloheptyl-1,4-dihydro-4,4-dimethyl-1,3-benzo [d]oxazin-2-one;
   6-(1-cycloheptenyl)-1,4-dihydro-4,4-dimethyl-1,3-benzo[d]ox azin-2-one;
   6-(1-cycloheptyl)-1,4-dihydro-4,4-dimethyl-1,3-benzo[d]oxaz in-2-one;
   6-(2,6,6-trimethyl-1-)cyclohexenyl-1,4-dihydro-4,4-dimethyl -1,3-benzo[d]oxazin-2-one;
   (±)-6-(3,3,5-trimethyl-1-)cyclohexenyl-1,4-dihydro-4,4-dime thyl-1,3-benzo[d]oxazin-2-one;
   (±)-6-(3,5,5-trimethyl-1-)cyclohexenyl-1,4-dihydro-4,4-dime thyl-1,3-benzo[d]oxazin-2-one;
   (±)-6-(5-methyl-1-)cyclohexenyl-1,4-dihydro-4,4-dimethyl-1, 3-benzo[d]oxazin-2-one;
   (±)-6-(3-methyl-1-)cyclohexenyl-1,4-dihydro-4,4-dimethyl-1, 3-benzo[d]oxazin-2-one;
   (±)-6-(2,6-dimethyl-1-)cyclohexenyl-1,4-dihydro-4,4-dimethy 1-1,3-benzo[d]oxazin-2one;
   (±)-6-(2-bicyclo[2.2.1]heptenyl)-1,4-dihydro-4,4-dimethyl-1 ,3-benzo[d]oxazin-2-one;
   (±)-6-(4,5-trans-dimethyl-1-)cyclohexenyl-1,4-dihydro-4,4-d imethyl-1,3-benzo[d]oxazin-2-one;
   (±)-6-(3,4-trans-dimethyl-1-)cyclohexenyl-1,4-dihydro-4,4-d imethyl-1,3-benzo[d]oxazin-2-one;
   6-(6,6-dimethyl-1-)cyclohexenyl-1,4-dihydro-4,4-dimethyl-1, 3-benzo[d]oxazin-2-one;
   6-(5,5-dimethyl-1-)cyclopentenyl-1,4-dihydro-4,4-dimethyl-1 ,3-benzo[d]oxazin-2-one;
   (±)-6-(3,3,5-cis-trimethyl)cyclohexyl-1,4-dihydro-4,4-dimet hyl-1,3-benzo[d]oxazin-2one;
   (±)-6-(3,3,5-trans-trimethyl)cyclohexyl-1,4-dihydro-4,4-dim ethyl-1,3-benzo[d]oxazin-2one;
   (±)-6-(3-cis-methyl)cyclohexyl-1,4-dihydro-4,4-dimethyl-1,3 -benzo[d]oxazin-2,one;
   (±)-6-(3-trans-methyl)cyclohexyl-1,4-dihydro-4,4-dimethyl-1 ,3-benzo[d]oxazin-2-one;
   (±)-6-(2,6-cis,cis-dimethyl)cyclohexyl-1,4-dihydro-4,4-dime thyl-1,3-benzo[d]oxazin-2-one;
   (E)-6-(1,4-dimethyl-1-)pentenyl-1,4-dihydro-4,4-dimethyl-1, 3-benzo[d]oxazin-2-one;
   6-(1-cyclohexenyl)-1,4-dihydro-4,4-dimethyl-1,3-benzo[d]oxa zin-2-thione;
   6-(3-oxo-1-)cyclopentenyl-1,4-dihydro-4,4-dimethyl-1,3-benz o[d]oxazin-2-one;
   6-(3-oxo-1-)cyclohexenyl-1,4-dihydro-4,4-dimethyl-1,3-benzo [d]oxazin-2-one;
   (±)-6-(3-hydroxy-1-)cyclohexenyl-1,4-dihydro-4,4-dimethyl-1 ,3-benzo[d]oxazin-2-one;
   (±)-6-(3-cis-hydroxy)cyclohexyl-1,4-dihydro-4,4-dimethyl-1, 3-benzo[d]oxazin-2-one;
   (±)-6-(3-butyl-3-hydroxy-1-)cyclohexenyl-1,4-dihydro-4,4-di methyl-1,3-benzo[d]oxazin-2-one;
   6-(3-oxo-1-)cyclohexenyl-1,4-dihydro-4,4-dimethyl-1,3-benzo [d]oxazin-2-thione;
   (±)-6-(3-hydroxy-1-)cyclohexenyl-1,4-dihydro-4,4-dimethyl-1 ,3-benzo[d]oxazin-2-thione;
   (±)-6-(1-cyclohexenyl)-1,4-dihydro-4-methyl-1,3-benzo[d]oxa zin-2-one;
   6-(1-cyclohexenyl)-1,4-dihydro-4,4,5-trimethyl-1,3-benzo[d] oxazin-2-one;
   6-(1-cyclohexenyl)-3,4-dihydro-4,4-dimethyl-2(1H)-quinolino ne;
   6-cyclohexyl-3,4-dihydro-4,4-dimethyl-2(1H)-quinolinone;
   (±)-6-bromo-6-(1-cyclohexenyl)-1,4-dihydro-4-trifluoromethy 1-1,3-benzo[d]oxazin-2one;
   5-(3-oxo-1-)cyclohexenyl-3,3-dimethyl-2-indolone;
   (±)-5-(3-hydroxy-1-)cyclohexenyl-3,3-dimethyl-2-indolone;
   (±)-5-(3-oxocyclohexyl)-3,3-dimethyl-2-indolone;
   5-cyclohexyl-3,3-spirocyclohexyl-2-indolone;
   5-cyclopentyl-3,3-spirocyclohexyl-2-indolone;
   6-(1-hydroxycyclohexyl)-2(3H)-benzothiozolone;
   6-cyclohexenyl-2(3H)-benzothiozolone;
   1-benzyl-6-cyclohexyl-3,4-dihydro-3-methyl-2(1H)-quinazolin one;
   6-(2,3-difluoro)phenyl-4-trifluoromethyl-2(1H)-quinolinone;
   4-trifluoromethyl-6-(3-nitro)phenyl-2(1H)-quinolinone;
   4-trifluoromethyl-6-(3,5-dichloro)phenyl-2(1H)-quinolinone;
   4-trifluoromethyl-6-(3-fluoro-5-N-hydroxyliminomethyl)pheny 1-2(1H)-quinolinone;
   4-trifluoromethyl-6-(3-fluoro-5-formylmethylphenyl)-2(1H)-q uinolinone;
   4-trifluoromethyl-6-(3-fluoro-5-cyano)phenyl-2(1H)-quinolin one;
   4-trifluoromethyl-6-(3-fluoro-5-chloro)phenyl-2(1H)-quinoli none;
   4-trifluoromethyl-6-(4-hydroxymethyl)phenyl-2(1H)-quinolino ne;
   4-trifluoromethyl-6-(3-acetylphenyl)-2(1H)-quinolinone;
   4-trifluoromethyl-6-(4-ethylphenyl)-2(1H)-quinolinone;
   4-trifluoromethyl-6-(3-ethoxylphenyl)-2(1H)-quinolinone;
   4-trifluoromethyl-6-(3-methylphenyl)-2(1H)-quinolinone;
   4-trifluoromethyl-6-(3-trifluoromethylphenyl)-2(1H)-quinoli none;
   4-trifluoromethyl-6-(3-chlorophenyl)-2(1H)-quinolinone;
   4-trifluoromethyl-6-(3-fluorophenyl)-2(1H)-quinolinone;
   4-trifluoromethyl-6-(2-methylphenyl)-2(1H)-quinolinone;
   4-trifluoromethyl-6-(4-formyl)phenyl-2(1H)-quinolinone;
   4-trifluoromethyl-6-(4-tert-butylphenyl)-2(1H)-quinolinone;
   4-trifluoromethyl-6-(2-methoxyphenyl)-2(1H)-quinolinone;
   4-trifluoromethyl-6-(2-fluorophenyl)-2(1H)-quinolinone;
   4-trifluoromethyl-6-(4-acetylphenyl)-2(1H)-quinolinone;
   4-trifluoromethyl-6-(4-methylphenyl)-2(1H)-quinolinone;
   4-trifluoromethyl-6-(4-fluorophenyl)-2(1H)-quinolinone;
   4-trifluoromethyl-6-(4-methoxyphenyl)-2(1H)-quinolinone;
   4-trifluoromethyl-6-(3,5-bis-trifluoromethyl)phenyl-2(1H)-q uinolinone;
   4-trifluoromethyl-6-(4-trifluoromethoxyphenyl)-2(1H)-quinol inone;
   4-trifluoromethyl-6-(2,4-dichlorophenyl)-2(1H)-quinolinone;
   3-fluoro-4-trifluoromethyl-6-(2-fluorophenyl)-2(1H)-quinoli none;
   3-fluoro-4-trifluoromethyl-6-(2,4-dichlorophenyl)-2(1H)-qui nolinone;
   4-trifluoromethyl-6-(4-hydroxyphenyl)-2(1H)-quinolinone;
   4-trifluoromethyl-6-(4,4,4-trifluoro-1(E)-butenyl)-2(1H)-qu inolinone;
   4-trifluoromethyl-6-(4,4,4-trifluorobutyro)-2-isopropyloxyq uinoline;
   4-trifluoromethyl-6-(1-hydroxy-4,4,4-trifluorobutyl)-2-isop ropyloxyquinoline;
   4-trifluoromethyl-6-(1-(3,3,3-trifluoropropyl)-1(E)-propeny 1)-2(1H)-quinolinone;
   4-trifluoromethyl-6-(1-ethyl-1-hydroxy-4,4,4-trifiuorobutyl )-2-isopropyloxyquinoline;
   4-trifluoromethyl-6-(1-ethyl-4,4,4-trifluoro-1(E)-butenyl)-2(1H)-quinolinone;
   4-trifluoromethyl-6-(1-ethyl-4,4,4-trifluoro-1(Z)-butenyl)-2(1H)-quinolinone;
   2-chloro-4-trifluoromethyl-6-(bis-N,N-2,2,2-trifluoroethyl) aminoquinoline;
   2-methoxy-4-trifluoromethyl-6-(bis-2,2,2-trifluoroethyl)ami noquinoline;
   2-isopropyloxy-4-trifluoromethyl-6-(bis-2,2,2-trifluoroethy 1)aminoquinoline;
   2-ethoxy-4-trifluoromethyl-6-(bis-2,2,2-trifluoroethyl)amin oquinoline;
   2-acetyloxy-4-trifluoromethyl-6-(bis-2,2,2-trifluoroethyl)a minoquinoline;
   2-(2-dimethylamino)ethoxy-4-trifluoromethyl-6-(bis-2,2,2-tr ifluoroethyl)aminoquinoline;
   2-isobutyryloxy-4-trifluoromethyl-6-(bis-2,2,2-trifluoroeth yl)aminoquinoline;
   2-(2,2-dimethyl)propyryloxy-4-trifluoromethyl-6-(bis-2,2,2-trifluoroethyl)-aminoquinoline;
   2-N,N-dimethylcarbamyloxy-4-trifluoromethyl-6-(bis-2,2,2-tr ifluoroethyl)-aminoquinoline;
   2-cyano-4-trifluoromethyl-6-(bis-2,2,2-trifluoroethyl)amino quinoline; and
   4-trifiuoromethyl-6-(bis-2,2,2-trifluoroethyl)amino-2(1H)-q uinolinone oxime,
   as the present compounds (1) to (5),
   (R/S)-6,7,7a,11-tetrahydro-7a-methyl-4-trifluoromethyl-2-py ridono[5,6-g]pyrrolidino[1,2-a]quinoline;
   (R/S)-3-fluoro-6,7,7a,11-tetrahydro-7a-methyl-4-trifluorome thyl-2-pyridono[5,6-g]pyrrolidino[1,2-a] quinoline;
   (R/S)-6,7,7a,11-tetrahydro-1,7a-dimethyl-4-trifluoromethyl-2-pyridono[5,6-g]pyrrolidino[1,2-a]quinoline;
   (R/S)-3-fluoro-6,7,7a,11-tetrahydro-1,7a-dimethyl-4-trifluo romethyl-2-pyridono[5,6-g]pyrrolidino[1,2-a]quinoline;
   11-(trifluoromethyl)-9-pyridon[6,5-i]julolidine;
   8-methyl-11-(trifluoromethyl)-9-pyridon[6,5-i]julolidine;
   7-fluoro-1,2,3,4-tetrahydro-2,2-dimethyl-6-trifluoromethyl-8-pyridono[5,6-g]quinoline;
   6-difluoromethyl-7-fluoro-1,2,3,4-tetrahydro-2,2-dimethyl-8 -pyridono[5,6-g]quinoline;
   7-fluoro-1,2,3,4-tetrahydro-2,2,9-trimethyl-6-trifluorometh yl-8-pyridono[5,6-g]quinoline;
   6-difluoromethyl-7-fluoro,1,2,3,4-tetrahydro-2,2,9-trimethy 1-8-pyridono[5,6-g]quinoline;
   7-fluoro-1,2,3,4-tetrahydro-1,2,2,9-tetramethyl-6-trifluoro methyl-8-pyridono[5,6-g]quinoline;
   6-difluoromethyl-7-fluoro-1,2,3,4-tetrahydro-1,2,2,9-tetram ethyl-8-pyridono[5,6-g]quinoline;
   7-fluoro-1,2-dihydro-2,2,4-trimethyl-6-trifluoromethyl-8-py ridono[5,6-g]quinoline;
   7-fluoro-1,2,3,4-tetrahydro-2,2,4-trimethyl-6-trifluorometh yl-8-pyridono[5,6-g]quinoline;
   1,10-[1,3-dihydro-3-oxo-(2,1-isoxazolyl)]-1,2,3,4-tetrahydr o-2,2,4,10-tetramethyl-6-trifluoromethyl-8-pyridono[5,6-g]q uinoline;
   7-fluoro-1,2-dihydro-2,2,4,10-tetramethyl-6-trifluoromethyl -8-pyridono[5,6-g]quinoline;
   7-fluoro-1,2,3,4-tetrahydro-2,2,4,10-tetramethyl-6-trifluor omethyl-8-pyridono[5,6-g]quinoline;
   7-fluoro-1,2,3,4-tetrahydro-2,2,4,9,10-pentamethyl-6-triflu oromethyl-8-pyridono[5,6-g]quinoline;
   7-fluoro-1,2,3,4-tetrahydro-1,2,2,4,10-pentamethyl-6-triflu oromethyl-8-pyridono[5,6-g]guinoline;
   1,2,3,4-tetrahydro-1-hydroxy-2,2-dimethyl-6-trifluoromethyl -8-pyridono[5,6-g]quinoline;
   1,2,3,4-tetrahydro-1-hydroxy-2,2,9-trimethyl-6-trifluoromet hyl-8-pyridono[5,6-g]quinoline;
   2,2-diethyl-7-fluoro-1,2,3,4-tetrahydro-6-trifluoromethyl-8 -pyridono[5,6-g]quinoline;
   (R/S)-4-Ethyl-1-formyl-1,2,3,4-tetrahydro-6-(trifluoromethy 1)-8-pyridono[5,6-g]quinoline;
   (R/S)-4-Ethyl-1,2,3,4-tetrahydro-1-(trifluoroacetyl)-6-(tri fluoromethyl)-8-pyridono[5,6-g]quinoline;
   (R/S)-1-acetyl-4-ethyl-1,2,3,4-tetrahydro-6-(trifluoromethy 1)-8-pyridono[5,6-g]quinoline;
   (R/S)-4-ethyl-1,2,3,4-tetrahydro-10-nitro-6-(trifluoromethy 1)-8-pyridono[5,6-g]quinoline;
   1,2,3,4-tetrahydro-2,2-dimethyl-10-nitro-6-(trifluoromethyl )-8-pyridono[5,6-g]quinoline;
   1,2,3,4-tetrahydro-2,2-dimethyl-7,10-dinitro-6-(trifluorome thyl)-8-pyridono[5,6-g]quinoline; and
   (R/S)4-Ethyl-1,2,3,4-tetrahydro-1-nitro-6-(trifluoromethyl) -8-pyridono[5,6-g]quinoline quinoline,
   as the present compounds (A) to (F),
   1,2,3,6-tetrahydro-1-methyl-9-(trifluoromethyl)-7H-[1,4]oxa zino[3,2-g]quinolin-7-one;
   1,2,3,6-tetrahydro-1,6-dimethyl-9-(trifluoromethyl)-7H-[1,4 ]oxazino[3,2-g]quinolin-7-one;
   1-ethyl-1,2,3,6-tetrahydro-9-(trifluoromethyl)-7H-[1,4]oxaz ino[3,2-g]quinolin-7-one;
   1-ethyl-1,2,3,6rtetrahydro-6-methyl-9-(trifluoromethyl)-7H-[1,4]oxazino[3,2-g]quinolin-7-one;
   1,2,3,6-tetrahydro-1-(2,2,2-trifluoroethyl)-9-(trifluoromet hyl)-7H-[1,4]oxazino[3,2-g]quinolin-7-one;
   8-fluoro-1,2,3,6-tetreahydro-1-(2,2,2-trifluoroethyl)-9-(tr ifluoroemthyl)-7H-[1,9]oxaaino[3,2-g]quinolin-7-one;
   8-chloro-1,2,3,6-tetrahydro-1-(2,2,2-trifluoroethyl)-9-(tri fluoromethyl)-7H-[1,4]oxazzno[3,2-g]quinolin-7-one;
   9-(difluoromethyl)-1,2,3,6-tetrahydro-1-(2,2,2-trifluoroeth yl)-7H-[1,4]oxazino[3,2-g]quinolin-7-one;
   1,2,3,6-tetrahydro-6-methyl-1-(2,2,2-trifluoroethyl)-9-(tri fluoromethyl)-7H-[1,4]oxazino[3,2-g]quinolin-7-one;
   7-chloro-2,3-dihydro-1-(2,2,2-trifluoroethyl)-9-(trifluorom ethyl)-1H-[1,4]oxazino[3,2-g]quinoline;
   1,2,3,6-tetrahydro-1-(2,2,2-trifluoroethyl)-9-(trifluoromet hyl)-7H-[1,4]oxazino[3,2-g]quinolin-7-thione;
   1,2,3,6-tetrahydro-1-propyl-9-(trifluoromethyl)-7H-[1,4]oxa zino[3,2-g]quinolin-7-one;
   1,2,3,6-tetrahydro-1-isobutyl-9-(trifluoromethyl)-7H-[1,4]o xazino[3,2-g]quinolin-7-one;
   1,2,3,6-tetrahydro-1-isobutyl-6-methyl-9-(trifluoromethyl)-7H-[1,4]oxazino[3,2-g]quinolin-7-one;
   (+)-1,2,3,6-tetrahydro-3-methyl-1-(2,2,2-trifluoroethyl)-9-(trifluoromethyl)-7H-[1,4]oxazino[3,2-g]quinolin-7-one;

   (-)-1,2,3,6-tetrahydro-3-methyl-1-(2,2,2-trifluoroethyl)-9-(trifluoromethyl)-7H-[1,4]oxazino[3,2-g]quinolin-7-one;
   (+)-1,2,3,6-tetrahydro-3-methyl-1-(2,2,2-trifluoroethyl)-9-(trifluoromethyl)-7H-[1,4]oxazino[3,2-g]quinolin-7-one;
   (+)-1,2,3,6-tetrahydro-1,3-dimethyl-9-(trifluoromethyl)-7H-[1,4]oxazino[3,2-g]quinolin-7-one;
   (+)-3-ethyl-1,2,3,6-tetrahydro-1-(2,2,2-trifluoroethyl)-9-( trifluoromethyl)-7H-[1,4]oxazino[3,2-g]quinolin-7-one;
   (+)-3-ethyl-1,2,3,6-tetrahydro-1-methyl-9-(trifluoromethyl) -7H-[1,4]oxazino[3,2-g]quinolin-7-one;
   1,2,3,6-tetrahydro-9-(trifluoromethyl)-7H-[1,4]oxazino[3,2-g]quinolin-7-one;
   1-cyclopropylmethyl-1,2,3,6-tetrahydro-9-(trifluoromethyl)-7H-[1,4]oxazino[3,2-g]quinolin-7-one;
   1,2,3,6-tetrahydro-1-(pyridylmethyl)-9-(trifluoromethyl)-7H -[1,4]oxazino[3,2-g]quinolin-7-one;
   (+)-1,2,3,6-tetrahydro-2-methyl-1-(2,2,2-trifluoroethyl)-9-(trifluoromethyl)-7H-[1,4]oxazino[3,2-g]quinolin-7-one;
   (+)-1,2,3,6-tetrahydro-2-methyl-1-(2,2,2-trifluoroethyl)-9-(trifluoromethyl)-7H-[1,4]oxazino[3,2-g]quinolin-7-one;
   (-)-1,2,3,6-tetrahydro-2-methyl-1-(2,2,2-trifluoroethyl)-9-(trifluoromethyl)-7H-[1,4]oxazino[3,2-g]quinolin-7-one;
   (+)-trans-1,2,3,6-tetrahydro-2,3-dimethyl-1-(2,2,2-trifluor oethyl)-9-(trifluoromethyl)-7H-[1,4]oxazino[3,2-g]quinolin-7-one;
   (+)-cis-1,2,3,6-tetrahydro-2,3-dimethyl-1-(2,2,2-trifluoroe thyl)-9-(trifluoromethyl)-7H-[1,4]oxazino[3,2-g]quinolin-7-one;
   (+)-trans-3-ethyl-1,2,3,6-tetrahydro-2-methyl-1-(2,2,2-trif luoroethyl)-9-(trifluoromethyl)-7H-[1,4]oxazino[3,2-g]quino lin-7-one;
   (+)-cis-3-ethyl-1,2,3,6-tetrahydro-2-methyl-1-(2,2,2-triflu oroethyl)-9-(trifluoromethyl)-7H-[1,4]oxazino[3,2-g]quinoli n-7-one;
   (+)-1,2,3,6-tetrahydro-2-(hydroxymethyl)-1-(2,2,2-trifluoro ethyl)-9-(trifluoromethyl)-7H-[1,4]oxazino[3,2-g]quinolin-7 -one;
   (+)-1,2,3,6-tetrahydro-2-(acetoxymethyl)-1-(2,2,2-trifluoro ethyl)-9-(trifluoromethyl)-7H-[1,4]oxazino[3,2-g]quinolin-7 -one;
   (+)-1,2,3,6-tetrahydro-2-(methoxymethyl)-1-(2,2,2-trifluoro ethyl)-9-(trifluoromethyl)-7H-[1,4]oxazino[3,2-g]quinolin-7 -one;
   (+)-1,2,3,6-tetrahydro-2-(methoxymethyl)-1-(2,2,2-trifluoro ethyl)-9-(trifluoromethyl)-7H-[1,4]oxazino[3,2-g]quinolin-7 -one;
   (-)-1,2,3,6-tetrahydro-2-(methoxymethyl)-1-(2,2,2-trifluoro ethyl)-9-(trifluoromethyl)-7H-[1,4]oxamino[3,2-glquinolin-7 -one;
   (+)-2-(ethoxymethyl)-1,2,3,6-tetrahydro-1-(2,2,2-trifluoroe thyl)-9-(trifluoromethyl)-7H-[1,4]oxazino[3,2-g]quinolin-7-one;
   (+)-1,2,3,6-tetrahydro-2-(propoxymethyl)-1-(2,2,2-trifluoro ethyl)-9-(trifluoromethyl)-7H-[1,4]oxazino[3,2-g]quinolin-7 -one;
   1,2-dihydro-1-(2,2,2-trifluoroethyl)-9-(trifluoromethyl)-3H -[1,4]oxazino[3,2-g]quinolin-2,7-dione;
   (+)-1,2,3,6-tetrahydro-2-hydroxy-2-methyl-1-(2,2,2-trifluor oethyl)-9-(trifluoromethyl)-7H-[1,4]oxazino[3,2-g]quinolin-7-one;
   1,2-dihydro-3-methyl-1-(2,2,2-trifluoroethyl)-9-(trifluorom ethyl)-3H-[1,4]oxazino[3,2-g]-quinolin-2,7-dione;
   1,2,3,6-tetrahydro-1-(2,2,2-trifluoroethyl)-9-(trifluoromet hyl)-2-thioxo-7H-[1,4]oxazino[3,2-g]quinolin-7-one;
   (+)-1,2,3,6-tetrahydro-2-methyl-9-(trifluoromethyl)-7H-[1,4 ]oxazino[3,2-g]quinolin-7-one;
   1-cyclopropylmethyl-1,2,3,6-tetrahydro-2-methyl-9-(trifluor omethyl)-7H-[1,4]oxazino[3,2-g]quinolin-7-one;
   (+)-2-ethyl-1,2,3,6-tetrahydro-9-(trifluoromethyl)-7H-[1,4] oxazino[3,2-g]quinolin-7-one;
   1-cyclopropylmethyl-2-ethyl-1,2,3,6-tetrahydro-9-(trifluoro methyl)-7H-[1,4]oxazino[3,2-g]quinolin-7-one;
   1,2,3,6-tetrahydro-1-isopropyl-9-(trifluoromethyl)-7H-[1,4] oxazino[3,2-g]quinolin-7-one;
   (+)-2-ethyl-1,2,3,6-tetrahydro-1-(2,2,2-trifluoroethyl)-9-( trifluoromethyl)-1H-[1,4]oxazino[3,2-g]quinolin-7-one;
   (+)-1,2-diethyl-1,2,3,6-tetrahydro-9-(trifluoromethyl)-7H-[ 1,4]oxazino[3,2-g]quinolin-7-one;
   (+)-1,2,3,6-tetrahydro-1-(2,2,2-trifluoroethyl)-2,9-bis(tri fluoromethyl)-7H-[1,4]oxazino[3,2-g]quinolin-7-one;
   (+)-1,2,3,6-tetrahydro-1-(2,2,2-trifluoroethyl)-2,9-bis(tri fluoromethyl)-7H-[1,4]oxazino[3,2-g]quinolin-7-one;
   (-)-1,2,3,6-tetrahydro-1-(2,2,2-trifluoroethyl)-2,9-bis(tri fluoromethyl)-7H-[1,4]oxazino[3,2-g]quinolin-7-one;
   (+)-1-ethyl-1,2,3,6-tetrahydro-2-methyl-9-(trifluoromethyl) -7H-[1,4]oxazino[3,2-g]quinolin-7-one;
   (2R)-(-)-1,2,3,6-tetrahydro-2-methyl-1-(2,2,2-trifluormethy 1)-9-(trifluromethyl)-7H-[1,4]oxazino[3,2-g]quinolin-7-one;
   (2R)-2-ethyl-1,2,3,6-tetrahydro-1-(2,2,2-trifluoroethyl)-9-(trifluoromethyl)-7H-[1,4]oxazino[3,2-g]quinolin-7-one;
   (2R)-1,2,3,6-tetrahydro-2-isobityl-1-(2,2,2-trifluoroethyl) -9-(trifluoromethyl)-7H-[1,4]oxazino[3,2-g]quinolin-7-one;
   (2R)-1,2,3,6-tetrahydro-2-isopropyl-1-(2,2,2-trifluoroethyl )-9-(trifluoromethyl)-7H-[1,4]oxazino[3,2-g]quinolin-7-one;
   (+)-1,2,3,4,4a,5-Hexahydro-11-(trifiuoromethyl)pyrido[1',2' :4,5][1,4]oxazino[3,2-g]quinolin-9(8H)-one;
   (R)-2,3,3a,4-tetrahydro-10-(trifluoromethyl)-pyrrolo[1',2' :4,5][1,4]oxazino[3,2-g]quinolin-8(7H)-one;
   1,3,4,6-tetrahydro-1,3,3-trimethyl-9-(trifluoromethyl)-pyra zino[3,2-g]quinolin-2,7-dione;
   1,2,3,4-tetrahydro-1,3,3-trimethyl-9-(trifluoromethyl)-pyra zino[3,2-g]quinolin-7(6H)-one;
   9-(trifluoromethyl)-1,2,3,6-tetrahydro-7H-[1,4]thiazino[3,2 -g]quinolin-7-one;
   1-methyl-9-(trifluoromethyl)-1,2,3,6-tetrahydro-7H-[1,4]thi azino[3,2-g]quinolin-7-one; and
   1-(2,2,2-trifluoroethyl)-9-(trifluoromethyl)-1,2,3,6-tetrah ydro-7H-[1,4]thiazino[3,2-g]quinolin-7-one, and
   as the present compound (α),
   (R)-6-(2-(2,2,2-trifluoroethyl)-1-pyrrolidinyl)-4-trifluoro methyl-2(1H) quinolinone;
   (R)-6-(2-phenylthiomethyl-1-pyrrolidinyl)-4-trifluoromethyl -2(1H)-quinolinone;
   (R)-6-(2-(2,2,2-trifluoroethyl)-1-piperidinyl)-4-trifluorom ethyl-2(1H)-quinolinone;
   (R)-6-(2-Benzyloxymethyl)-1-piperidinyl)-4-trifluoromethyl-2(1H)-guinolinone;
   (R)-6-(2-diethylaminomethyl-1-pyrrolidinyl)-4-trifluorometh yl-2(1H)-quinolinone;
   6-(2(R)-hydroxymethyl-5(S)-methyl-1-pyrrolidinyl)-4-trifluo romethyl-2(1H)-quinolinone;
   6-(2(R)-fluoromethyl-5(R)-methyl-1-pyrrolidinyl)-4-trifluor omethyl-2(1H)-quinolinone;
   6-(2(R)-fluoromethyl-5(S)-methyl-l-pyrrolidinyl)-4-trifluor omethyl-2(1H)-quinolinone;
   6-(2(R)-difluoromethyl-5(R)-methyl-1-pyrrolidinyl)-4-triflu oromethyl-2(1H)-quinolinone;
   6-(2(R)-fluoromethyl-5(S)-methyl-1-pyrrolidinyl)-4-trifluor omethyl-2(1H)-quinolinone;
   6-(2(R)-(1(R)-hydroxy-2,2,2-trifluoroethyl)-5(S)-methyl-1-p yrrolidinyl)-4-trifluoromethyl-2(1H)-quinolinone;
   6-(2(R)-(1(S)-hydroxy-2,2,2-trifluoroethyl)-5(S)-methyl-1-p yrrolidinyl)-4-trifluoromethyl-2(1H)-quinolinone;
   6-(2(R)-(1(S)-hydroxy-2,2,2-trifluoroethyl)-5(R)-methyl-1-p yrrolidinyl)-4-trifluoromethyl-2(1H)-quinolinone;
   6-(2(R)-(1(R)-hydroxy-2,2,2-trifluoroethyl)-5(R)-methyl-1-p yrrolidinyl)-4-trifluoromethyl-2(1H)-quinolinone;
   6-(2(R)-(2,2,2-trifluoroethyl)-5(R)-methyl-1-pyrrolidinyl)-4-trifluoromethyl-2(1H)-quinolinone;
   6-(2(R)-(1(S)-hydroxy-2,2,2-trifluoroethyl)-4(R)-hydroxy-1-pyrrolidinyl)-4-trifluoromethyl-2(1H)-quinolinone;
   6-(2(R)-(1(R)-hydroxy-2,2,2-trifluoroethyl)-4(R)-hydroxy-1-pyrrolidinyl)-4-trifluoromethyl-2(1H)-quinolinone;
   6-(2(R)-(1(S)-fluoro-2,2,2-trifluoroethyl)-4(S)-fluoro-1-py rrolidinyl)-4-trifluoromethyl-2(1H)-quinolinone;
   6-(2(R)-(1(R)-fluoro-2,2,2-trifluoroethyl)-4(S)-fluoro-1-py rrolidinyl)-4-trifluoromethyl-2(1H)-quinolinone;
   6-(2(R)-(1(S)-hydroxy-2,2,2-trifluoroethyl)-4(R)-methyl-1-p yrrolidinyl)-4-trifluoromethyl-2(1H)-quinolinone;
   6-(2(R)-(1(R)-hydroxy-2,2,2-trifluoroethyl)-4(R)-methyl-1-p yrrolidinyl)-4-trifluoromethyl-2(1H)-quinolinone;
   6-(2(R)-(1(S)-hydroxy-2,2,2-trifluoroethyl)-4(S)-methyl-1-p yrrolidinyl)-4-trifluoromethyl-2(1H)-quinolinone;
   6-(2(R)-(1(R)-hydroxy-2,2,2-trifluoroethyl)-4(S)-methyl-1-p yrrolidinyl)-4-trifluoromethyl-2(1H)-quinolinone;
   6-(2(R)-(1(S)-hydroxy-2,2,2-trifluoroethyl)-4(R)-methoxy-1-pyrrolidinyl)-4-trifluoromethyl-2(1H)-quinolone;

   6-(2(R)-(1(R)-hydroxy-2,2,2-trifluoroethyl)-4(R)-methoxy-1-pyrrolidinyl)-4-trifluoromethyl-2(1H)-quinolinone;
   6-(2(R)-(1(R)-hydroxy-2,2,2-trifluoroethyl)-4(S)-methoxy-1-pyrrolidinyl)-4-trifluoromethyl-2(1H)-quinolinone;
   6-(2(R)-(1(S)-hydroxy-2,2,2-trifluoroethyl)-4(S)-methoxy-1-pyrrolidinyl)-4-trifluoromethyl-2(1H)-quinolinone;
   4-chloro-6-(2(R)-(1(S)-hydroxy-2,2,2-trifluoroethyl)-5(R)-m ethyl-1-pyrrolidinyl)-2(1H)-quinolinone;
   4-chloro-6-(2(R)-(1(R)-hydroxy-2,2,2-trifluoroethyl)-5(R)-m ethyl-1-pyrrolidinyl)-2(1H)-quinolinone;
   4-chloro-6-(2(R)-(1(S)-hydroxy-2,2,2-trifluoroethyl)-1-pyrr olidinyl)-2(1H)-quinolinone;
   4-chloro-6-(2(R)-(1(R)-hydroxy-2,2,2-trifluoroethyl)-1-pyrr olidinyl)-2(1H)-quinolinone;
   6-(2(R)-(1(R)-hydroxy-1-methyl-2,2,2-trifluoroethyl)-5(R)-m ethyl-1-pyrrolidinyl)-4-trifluoromethyl-2(1H)-quinolinone;
   6-(2(R)-(1(S)-hydroxy-1-methyl-2,2,2-trifluoroethyl)-5(R)-m ethyl-1-pyrrolidinyl)-4-trifluoromethyl-2(1H)-quinolinone;
   6-(2(R)-(1-hydroxy-1-trifluoromethyl-2,2,2-trifluoroethyl)-5(R)-methyl-1-pyrrolidinyl)-4-trifluoromethyl-2(1H)-quinoli none;
   6-(2(R)-(1(R)-ethoxy-2,2,2-trifluoroethyl)-1-pyrrolidinyl)-4-trifluoromethyl-2(1H)-quinolinone;
   6-(2(R)-(1(R)-hydroxy-2,2,2-trifluoroethyl)-1-pyrrolidinyl) -4-propyl-2(1H)-quinolinone;
   6-(2(R)-(1(S)-hydroxy-2,2,2-trifluoroethyl)-1-pyrrolidinyl) -4-propyl-2(1H)-quinolinone;
   6-(2(R)-(1(S)-hydroxy-2,2,2-trifluoroethyl)-1-pyrrolidinyl) -4-ethyl-2(1H)-quinolinone; 6-(2(R)-(1(R)-hydroxy-2,2,2-trifluoroethyl)-1-pyrrolidinyl) -4-ethyl-2(1H)-quinolinone;
   6-(2(R)-chloromethyl-5-(R)-methyl-1-pyrrolidinyl)-4-trifluo romethyl-2(1H)-quinolinone;
   6-(2(R)-chloromethyl-5-(S)-methyl-1-pyrrolidinyl)-4-trifluo romethyl-2(1H)-quinolinone;
   6-(2(R)-(1(S)-hydroxy-2,2,2-trifluoroethyl)-5(S)-phenyl-1-p yrrolidinyl)-4-trifluoromethyl-2(1H)-quinolinone;
   6-(2(R)-(1(R)-hydroxy-2,2,2-trifluoroethyl)-5(S)-phenyl-1-p yrrolidinyl)-4-trifluoromethyl-2(1H)-quinolinone;
   6-(2(R)-(1(S)-hydroxy-2,2,2-trifluoroethyl)-5,5-dimethyl-1-pyrrolidinyl)-4-trifluoromethyl-2(1H)-quinolinone;
   6-(2(R)-(1(R)-hydroxy-2,2,2-trifluoroethyl)-5,5-dimethyl-1-pyrrolidinyl)-4-trifluoromethyl-2(1H)-quinolinone;
   6-(2(R)-(1(S)-hydroxy-2,2,2-trifluoroethyl)-5(R)-phenyl-1-p yrrolidinyl)-4-trifluoromethyl-2(1H)-quinolinone;
   6-(2(R)-(1(R)-hydroxy-2,2,2-trifluoroethyl)-5(R)-phenyl-1-p yrrolidinyl)-4-trifluoromethyl-2(1H)-quinolinone;
   6-(2(R)-(1(R),2-dihydroxyethyl)-1-pyrrolidinyl)-4-trifluoro methyl-2(1H)-quinolinone;
   6-(2(R)-(1(S),2-dihydroxyethyl)-1-pyrrolidinyl)-4-trifluoro methyl-2(1H)-quinollnone;
   6-(2(R)-(1(R)-hydroxybenzyl)-5(S)-methyl-1-pyrrolidinyl)-4-trifluoromethyl-2(1H)-quinolinone;
   6-(2(R)-(1(S)-hydroxybenzyl)-5(R)-methyl-1-pyrrolidinyl)-4-trifluoromethyl-2(1H)-quinolinone;
   6-(2(R)-(1(R)-hydroxybenzyl)-5(R)-methyl-1-pyrrolidinyl)-4-trifluoromethyl-2(1H)-quinolinone;
   6-(2(R)-((2-1,3-dithianyl)-1(R)-hydroxymethyl)-5(R)-methyl-1-pyrrolidinyl)-4-trifluoromethyl-2(1H)-quinolinone;
   6-(2(R)-((2-1,3-dithianyl)-1(59-hydroxymethyl)-5(R)-methyl-1-pyrrolidinyl)-4-trifluoromethyl-2(1H)-quinolinone;
   6-(2(R)-difluoromethyl-5,5-dimethyl-1-pyrrolidinyl)-4-trifl uoromethyl-2(1H)-quinolinone;
   6-(2(R)-fluoromethyl-5,5-dimethyl-1-pyrrolidinyl)-4-trifluo romethyl-2(1H)-quinolinone;
   6-(2(R)-hydroxymethyl-5,5-dimethyl-1-pyrrolidinyl)-4-triflu oromethyl-2(1H)-quinolinone;
   6-(2(R)-hydroxymethyl-1-piperidinyl)-4-trifluoromethyl-2(1H )-quinolinone;
   6-(2(R)-(1(S)-hydroxyethyl-1-piperidinyl)-4-trifluoromethyl -2(1H)-quinolinone:
   6-(2(R)-(1(R)-hydroxyethyl-1-piperidinyl)-4-trifluoromethyl -2(1H)-quinolinone;
   6-(2(R)-trifluoroacetyl-1-piperidinyl)-4-trifluoromethyl-2( 1H)-quinolinone;
   6-(2(R)-(1(S)-hydroxypentyl-1-piperidinyl)-4-trifluoromethy 1-2(1H)-quinolinone;
   6-(2(R)-(1(R)-hydroxypentyl-1-piperidinyl)-4-trifluoromethy 1-2(1H)-quinolinone:
   6-(2(R)-(1(R)-hydroxyethyl)-5(R)-methyl-1-pyrrolidinyl)-4-t rifluoromethyl-2(1H)-quinolinone;
   6-(2(R)-(1-hydroxy-1-methylethyl)-5(R)-methyl-1-pyrrolidiny 1)-4-trifluoromethyl-2(1H)-quinolinone;
   6-(2(R)-(1(S)-hydroxy-1-cyclopropylmethyl)-5(R)-methyl-1-py rrolidinyl)-4-trifluoromethyl-2(1H)-quinolinone;
   6-(2(R)-(1(R)-hydroxy-1-cyclopropylmethyl)-5(R)-methyl-1-py rrolidinyl)-4-trifluoromethyl-2(1r-quinolinone;
   6-(2(R)-(1(S)-hydroxypropyl)-5(R)-methyl-1-pyrrolidinyl)-4-trifluoromethyl-2(1H)-quinolinone,
   6-(2(R)-(1(R)-hydroxypropyl)-5(S)-methyl-1-pyrrolidinyl)-4-trifluoromethyl-2(1H)-quinolinone;
   6-(2(R)-(1(R)-hydroxypropyl)-5(R)-methyl-1-pyrrolidinyl)-4-trifluoromethyl-2(1H)-quinolinone;
   6-(2(R)-(1(S)-hydroxypropyl)-5(S)-methyl-1-pyrrolidinyl)-4-trifluoromethyl-2(1H)-quinolinone;
   6-(2(R)-(1(R)-hydroxy-2-methylpropyl)-5(R)-methyl-1-pyrroli dinyl)-4-trifluoromethyl-2(1H)-quinolinone;
   6-(2(R)-(1(R)-hydroxy-2-acetoxyethyl)-1-pyrrolidinyl)-4-tri fluoromethyl-2(1H)-quinolinone;
   6-(2(R)-(1(R)-hydroxy-2-chloroethyl)-1-pyrrolidinyl)-4-trif luoromethyl-2(1H)-quinolinone:
   6-(2(R)-(2-hydroxyethyl)-1-pyrrolidinyl)-4-trifluoromethyl-2(1H)-quinolinone;
   6-(R)-(2-oxoethyl)-1-pyrrolidinyl)-4-trifluoromethyl-2(1H)-quinolinone;
   6-(2(R)-acetyloxymethyl-6(R)-methyl-1-piperidinyl)-4-triflu oromethyl-2(1H)-quinolinone;
   6-(2(R)-(1(R)-chloro-2-hydroxymethyl)-1-pyrrolidinyl)-4-tri fluoromethyl-2(1H)-quinolinone;
   6-(2(R)-hydroxymethyl-6(R)-methyl-1-piperidinyl)-4-trifluor omethyl-2(1H)-quinolinone;
   6-(2(R)-(1(R)-hydroxy-2,2,2-trifluoroethyl)-6(R)-methyl-1-p iperidinyl)-4-trifluoromethyl-2(1H)-quinolinone; 6-(2(R)-(1(S)-hydroxy-2,2,2-trifluoroethyl)-5(R)-methyl-1-p yrrolidinyl)-4-chlorodifluoromethyl-2(1H)-quinolinone;
   6-(2(R)-(1(R)-hydroxy-2,2,2-trifluoroethyl)-5(R)-methyl-1-p yrrolidinyl)-4-chlorodifluoromethyl-2(1H)-quinolinone;
   6-(2(R)-(1(S)-hydroxy-2,2,2-trifluoroethyl)-5(S)-methyl-1-p yrrolidinyl)-4-chlorodifluoromethyl-2(1H)-quinolinone;
   6-(2(R)-(2(S)-hydroxy-3,3,3-trifluoropropyl)-1-pyrrolidinyl )-4-trifluoromethyl-2(1H)-quinolinone;
   6-(2(R)-(2(R)-hydroxy-3,3,3-trifluoropropyl)-1-pyrrolidinyl )-4-trifluoromethyl-2(1H)-quinolinone;
   6-(2(R)-acetyloxymethyl-6(R)-methyl-1-piperidinyl)-4-triflu oromethyl-2(1H)-quinolinone;
   6-(2(R)-hydroxyethyl-5(R)-methyl-1-pyrrolidinyl)-4-trifluor omethyl-2(1H)-quinolinone:
   6-(2(R)-hydroxyethyl-5(S)-methyl-1-pyrrolidinyl)-4-trifluor omethyl-2(1H)-quinolinone:
   6-(2(R)-acetyloxyethyl-5(R)-methyl-1-pyrrolidinyl)-4-triflu oromethyl-2(1H)-quinolinone;
   6-(2(R)-(1(S)-hydroxyethyl-2,2,2-trifluoroethyl)-4(S)-fluor o-1-pyrrolidinyl)-4-trifluoromethyl-2(1H)-quinolinone; and
   6-(2(R)-(1(R)-hydroxyethyl-2,2,2-trifluoroethyl)-4(S)-fluor o-1-pyrrolidinyl)-4-trifluoromethyl-2(1H)-quinolinone.

More preferred embodiments of the present compounds include:
as the present compounds (I) to (IX), compounds represented by the formulas (a) and (a'): 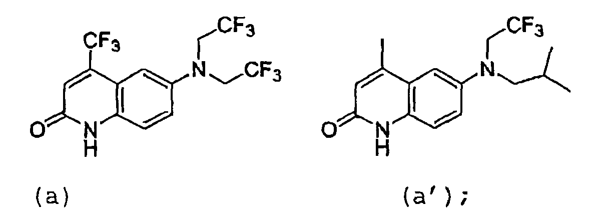 as the present compounds (1) to (5), a compound represented by the formula (b): 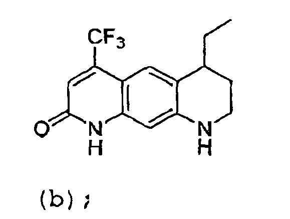 as the present compounds (A) to (F), a compound represented by the formula (c): 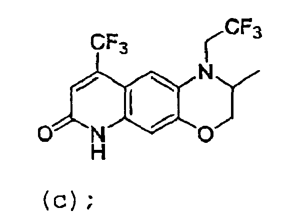 and
as the present compound (α), a compound represented by the formula (d):

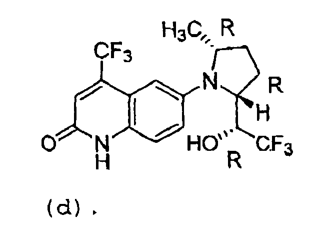

The present pharmaceutical composition contains the present compound or a pharmaceutically acceptable salt thereof.

Examples of the pharmaceutically acceptable salt include a salt with a physiologically acceptable acid (an inorganic acid, an organic acid, and so on) or base (an alkaline metal and so on), and a physiologically acceptable acid addition salt is particularly preferred. Examples of such acid addition salt include a salt with an inorganic acid (hydrochloric acid, phosphoric acid, hydrobromic acid, sulfuric acid, and so on) and a salt with an organic acid (acetic acid, formic acid, propionic acid, fumaric acid, maleic acid, succinic acid, tartaric acid, citric acid, malic acid, oxalic acid, benzoic acid, methanesulfonic acid, benzenesulfonic acid, and so on).

The present pharmaceutical composition may be used to inhibit Aβ accumulation as an Aβ accumulation-inhibitory agent (hereinafter, sometimes referred to as the present Aβ accumulation-inhibitory agent).

Also, it is possible to inhibit Aβ accumulation by (step of) administering an effective amount of the present pharmaceutical composition to a mammal which may be diagnosed with an Aβ-related disease. For example, as such a step, by performing a step of administering orally or parenterally an effective amount of the present Aβ accumulation-inhibitory agent to a mammal such as a human which may be diagnosed with an Aβ-related disease, it is possible to inhibit Aβ accumulation in the mammal.

In the case of orally administering the present Aβ accumulation-inhibitory agent to a mammal which may be diagnosed with an Aβ-related disease, the present Aβ accumulation-inhibitory agent may be used in an ordinary dosage form such as a tablet (including a sugar-coated tablet and a film-coated tablet), a pill, a granule, a powder, a capsule (including a soft capsule), a syrup, an emulsion, and a suspension.

In the case of parenteral administration, the present Aβ accumulation-inhibitory agent may be used in an ordinary liquid dosage form such as an emulsion and a suspension. Examples of a method of the parenteral administration include a method of injection and a method of administering a suppository into the rectum.

For a method for administering the present Aβ accumulation-inhibitory agent, a conventional method of administration such as the aforementioned oral administration and the parenteral administration may be appropriately selected without any particular limitation, and it may be used singly or in combination.

An appropriate dosage form of the present Aβ accumulation-inhibitory agent may be produced by mixing the present compound or a pharmaceutically acceptable salt thereof with an acceptable ordinary carrier, excipient, binder, stabilizer, diluent, and so on. Further, when the present Aβ accumulation-inhibitory agent is used in the form of an injection, an acceptable buffer, solubilizer, isotonic agent, and so on may be added.

Because the present Aβ accumulation-inhibitory agent thus obtained is low-toxic, and thus highly safe, it may be used (administered) to, for example, a mammal (for example, a human, a rat, a mouse, a guinea pig, a rabbit, a sheep, a pig, a cow, a horse, a cat, a dog and a monkey). The dosage varies depending on age, sex, weight and severity of a disease of a mammal to be administered, a kind and an administration form (dosage form) of the present Aβ accumulation-inhibitory agent, and so on. Usually, for example, in the case of orally administering to a human, it may be administered at, as the amount of an active ingredient, 0.01 mg to 1000 mg, preferably 0.1 mg to 1000 mg, more preferably 1.0 mg to 200 mg, and even more preferably 1.0 mg to 50 mg per day, to an adult (for example, weighing 60 kg). The daily dose may also be administered in several divided dosage.

Also, in the case of parenterally administering to a human, it may be administered by an intravenous injection at, as the amount of an active ingredient, 0.01 mg to 30 mg, preferably 0.1 mg to 20 mg, and more preferably 1.0 mg to 10 mg per day, to an adult (for example, weighing 60 kg).

Examples of a disease to which the present Aβ accumulation-inhibitory agent may be applicable include such a disease as an Aβ-related disease (amyloidosis).

The Aβ-related disease is classified into "localized amyloidosis" in which accumulation (deposition) is caused in only some organs and tissues in a living body, and "systemic amyloidosis" in which accumulation is caused in various organs and tissues throughout the body.

Examples of localized amyloidosis include Alzheimer's disease, Down's syndrome, cerebrovascular amyloidosis, hereditary amyloidotic cerebral hemorrhage, mad cow disease (bovine spongiform encephalopathy, BSE), variant Creutzfeldt-Jakob disease (vCJD), Gerstmann-Straussler-Scheinker syndrome, transmissible spongiform encephalopathy, scrapie, heart disease (senile cardiac amyloidosis), endocrine tumor (thyroid cancer), adult-onset diabetes, cutaneous amyloidosis and localized nodular amyloidosis.

Examples of systemic amyloidosis include familial amyloid polyneuropathy (FAP), tuberculosis, Hansen's disease, familial Mediterranean fever, bronchiectasis, Muckle-wells syndrome, reactive AA amyloidosis, immunocytic amyloidosis, osteomyelitis and cardiac failure. Other examples include senile amyloidosis which develops non-hereditarily at an advanced age, dialysis amyloidosis caused by amyloid that results from alteration in protein irremovable by a dialysis membrane used in treatment of dialysis patients, and secondary amyloidosis caused by amyloid that is generated by cleavage of a protein expressed in rheumatism.

### Examples

The present invention will be described below in further detail by example, however, the present invention is not limited by the example.

### Example 1 Measurement of fluctuation in the amount of Aβ accumulation in cultured nerve cells

Using a phenol red-free DMEM medium (manufactured by Invitrogen Corporation) to which charcoal dextran-treated FBS has been added so that its final concentration can be 10%, cultured nerve cells (human neuroblastoma CHP212, ATCC) were inoculated in a six-well plate (manufactured by BD Falcon) at approximately 2 x 10⁵ cells/well, and then cultured overnight. after which DMSO or DMSO containing a test substance of the formula (a) dissolved therein was added to the cells. The cells were then cultured, and a protease inhibitor (p1860, manufactured by Sigma-Aldrich Corporation) and DMSO were added to each well 48 hours after the addition of the test substance, followed by measuring the amount of Aβ in the medium by ELISA method (Human/Rat Amyloid β1-42 measuring kit, Code. No. 290-62601, Wako Pure Chemical Industries, Ltd.) The results were shown in Figure 1.

It was observed that the amount of Aβ accumulation was markedly decreased in a culture that had been treated with the test substance of the formula (a).

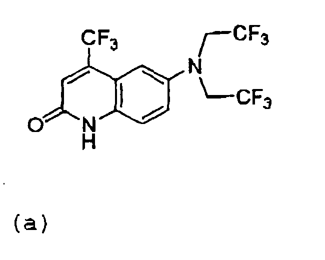

### Industrial Applicability

According to the present invention, it becomes possible to provide a compound that inhibits Aβ accumulation.

## Claims

1. A pharmaceutical composition for inhibiting amyloid-β protein accumulation comprising a compound represented by the following formulas: 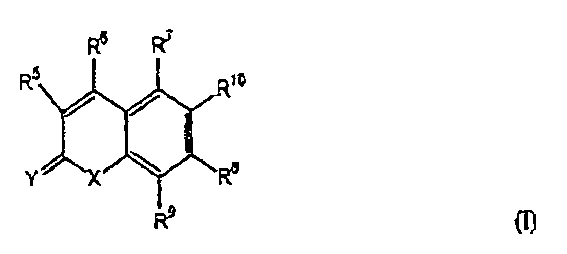 or 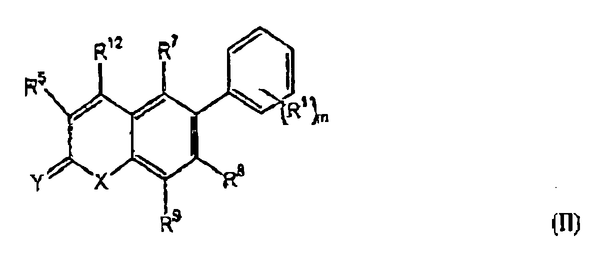 or 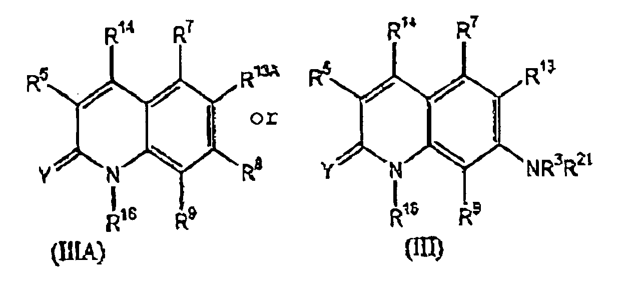 or 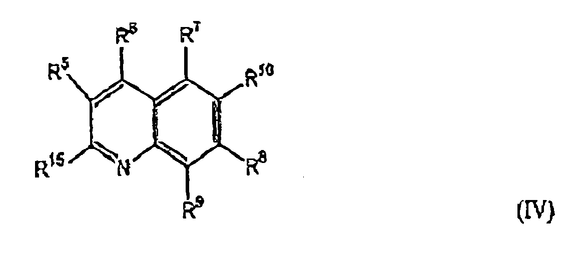 or 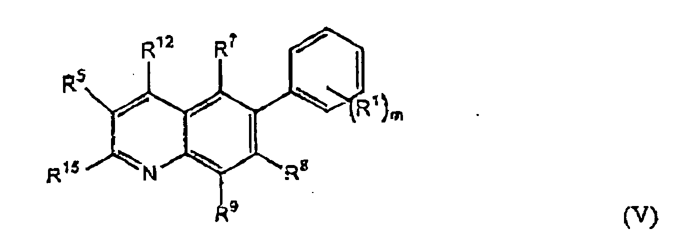 or 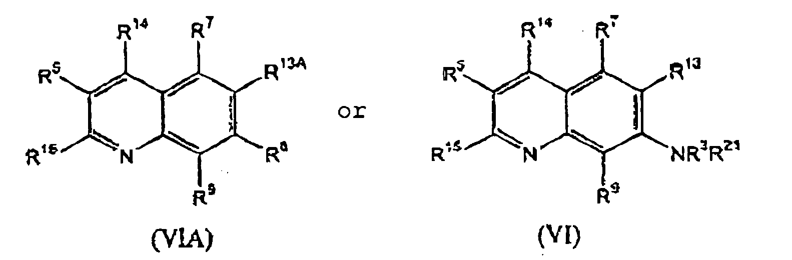 or 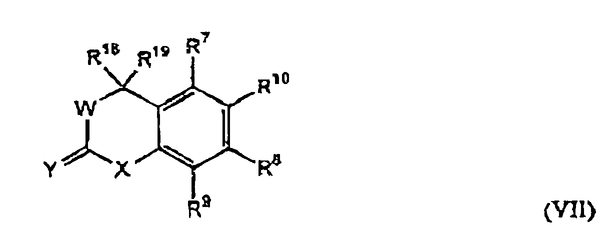 or 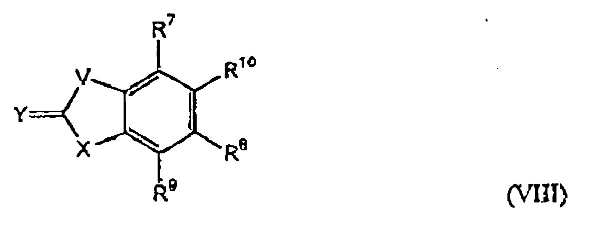 or 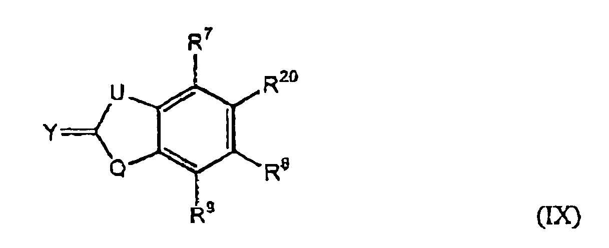 or 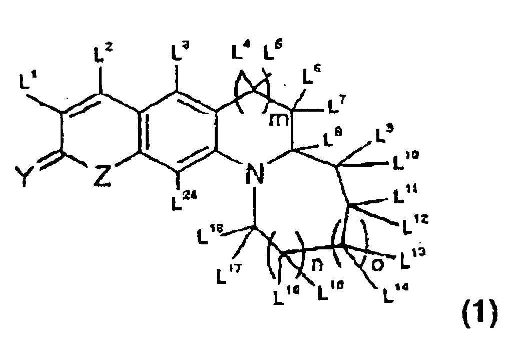 or 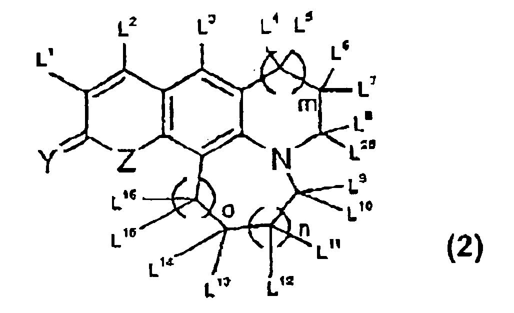 or 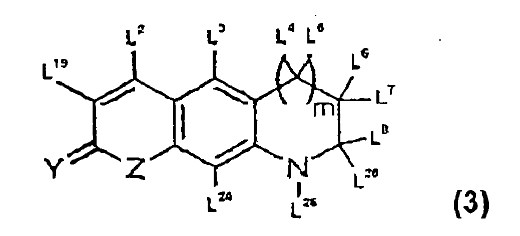 or 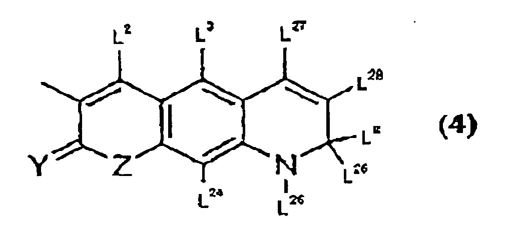 or 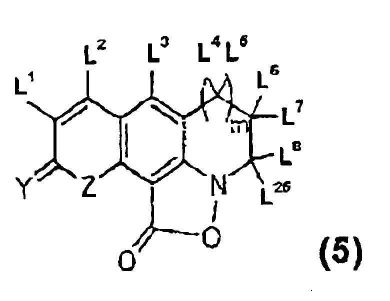 or 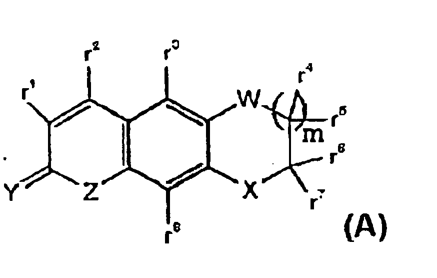 or 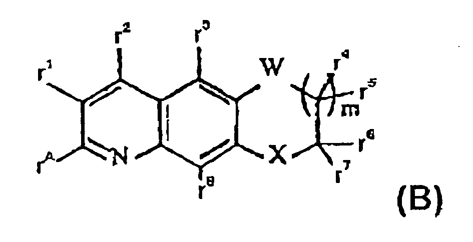 or 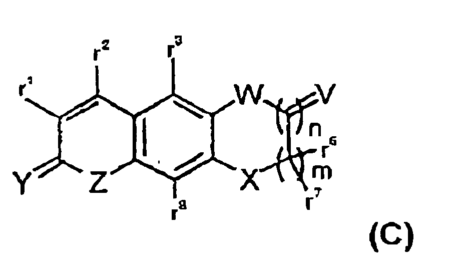 or 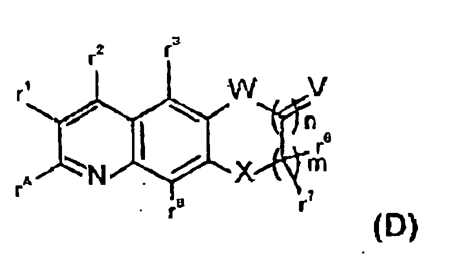 or 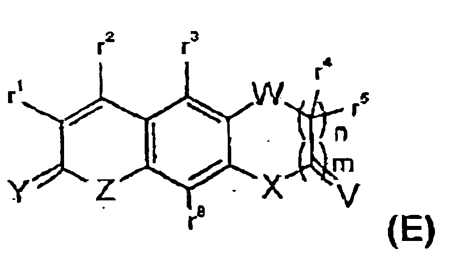 or 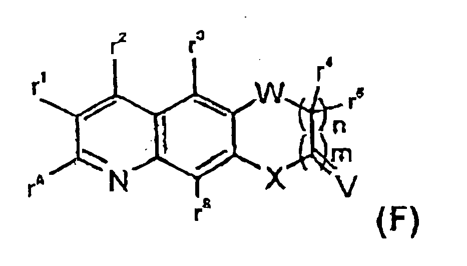 or 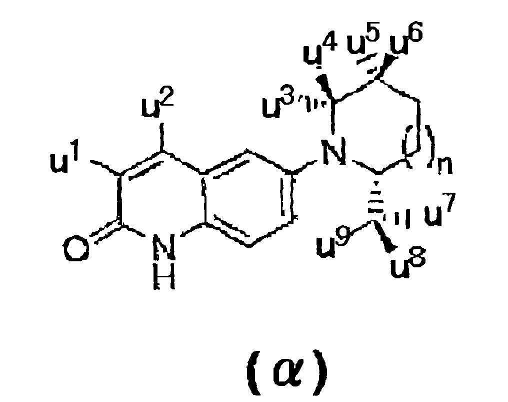 or a pharmaceutically acceptable salt thereof,
wherein, in the formulas (I) to (IX),
R¹ and R² are each independently selected from the group of COR³, CSR³, SO₂R³, NO, NR³R⁴, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₈ heteroalkyl, C₂-C₈ heteroalkenyl, C₂-C₈ heteroalkynyl, (CH₂)ₙR^{3A}, aryl, and heteroaryl, wherein the alkyl, alkenyl, alkynyl, haloalkyl, haloalkenyl, haloalkynyl, heteroalkyl, heteroalkenyl, heteroalkynyl, aryl, and heteroaryl are optionally substituted with F, Cl, Br, I, OR³, NR³R⁴, CN, NO₂, SR³, SOR³, SO₂R³, C₁-C₄ alkyl, C₁-C₄ haloalkyl or C₁-C₄ heteroalkyl; or
R¹ and R² are taken together to form a three- to nine-membered alkyl, alkenyl, heteroalkyl, or heteroalkenyl ring, wherein the alkyl, alkenyl, heteroalkyl, or heteroalkenyl ring are optionally substituted with F, Cl, Br, I, OR³, NR³R⁴, C₁-C₄ alkyl, C₁-C₄ haloalkyl or C₁-C₄ heteroalkyl; or
R¹ and R² are taken together to form one of: 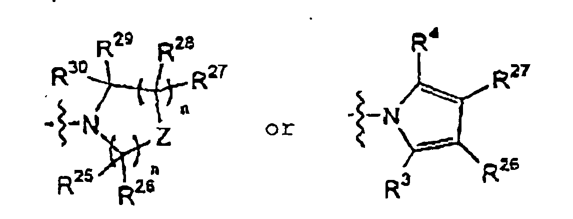 R³ and R⁴ are each independently selected from the group of hydrogen, C₁-C₈ alkyl, C₂-C₈ alkenyl, C₂-C₈ alkynyl, C₁-C₈ haloalkyl, C₁-C₈ heteroalkyl, heteroaryl, and aryl, wherein the alkyl, alkenyl, alkynyl, haloalkyl, heteroalkyl, heteroaryl, and aryl are optionally substituted with halogen, C₁-C₄ alkyl, C₁-C₄ haloalkyl or C₁-C₄ heteroalkyl;
R^{3A} is aryl or heteroaryl, wherein the aryl and heteroaryl is optionally substituted with halogen, CN, NO₂, C₁-C₄ alkyl, C₁-C₄ haloalkyl or C₁-C₄ heteroalkyl;
R⁵ is selected from the group of hydrogen, F, Cl, Br, I, OR³, SR³, NR³R⁴, C₁-C₄ alkyl, C₁-C₄ haloalkyl or C₁-C₄ heteroalkyl;
R⁶ is selected from the group of F, Cl, Br, I, CH₃, CF₃, CHF₂, CFH₂, CN, CF₂Cl, CF₂OR³, OR³, SR³, SOR³, SO₂R³, CO₂R³, NR³R⁴, C₁-C₄ alkyl, C₂-C₄ alkenyl, C₂-C₄ alkynyl, C₁-C₄ haloalkyl, C₂-C₄ haloalkenyl, C₂-C₄ haloalkynyl, C₁-C₄ heteroalkyl, C₂-C₄ heteroalkenyl, and C₂-C₄ heteroalkynyl, wherein the alkyl, alkenyl, alkynyl, haloalkyl, haloalkenyl, haloalkynyl, heteroalkyl, heteroalkenyl, and heteroalkynyl are optionally substituted with F, Cl, Br, C₁-C₄ alkyl, C₁-C₄ haloalkyl or C₁-C₄ heteroalkyl;
R⁷ and R⁸ are each independently selected from the group of hydrogen, F, Cl, Br, I, CN, OR³, NR³R⁴, NR³CR³R⁹CONR³R⁴, Cₙ(R³)₂ₙOR³, SR³, SOR³, SO₂R³, NR³COR⁴, C₁-C₈ alkyl, C₁-C₈ haloalkyl, and C₁-C₈ heteroalkyl ;
R⁹ is selected from the group of hydrogen, F, Br, Cl, I, OR³, NR³R⁴, SR³, SOR³, SO₂R³, C₁-C₄ alkyl, C₁-C₄ haloalkyl and C₁-C₄ heteroalkyl;,
R¹⁰ is selected from the group represented by the following formulas: 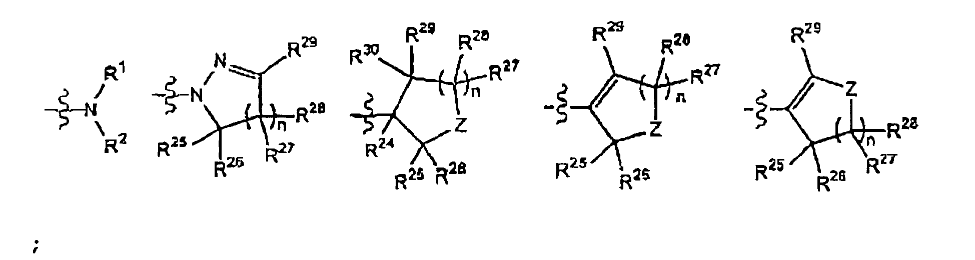 R¹¹ is selected from the group of F, Br, Cl, I, C₁-C₆ alkyl, C₁-C₆ haloalkyl, C₁-C₆ heteroalkyl, NO₂, CN, CF₃, OR³, NR3R⁴, SR³, SOR³, and SO₂R³;
R¹² is selected from the group of F, Br, Cl, I, CN, OR³, SR³, SOR³, SO₂R³, NR³R⁴, and C₁-C₄ haloalkyl;
R¹³ is selected from the group of hydrogen, F, Cl, Br, I, CN, OR³, NR³R⁴, COR³ CO₂R³, SR³, SOR³, SO₂R³, C₁-C₈ alkyl, C₂-C₈ alkenyl, C₂-C₈ alkynyl, C₁-C₈ haloalkyl, C₂-C₈ haloalkenyl, C₂-C₈ haloalkynyl, C₁-C₆ heteroalkyl, C₂-C₈ heteroalkenyl, C₂-C₈ heteroalkynyl, and (CH₂)ₙR^{3A}, wherein the alkyl, alkenyl, alkynyl, haloalkyl, haloalkenyl, haloalkynyl, heteroalkyl, heteroalkenyl and heteroalkynyl are optionally substituted with F, Cl, Br, I, CN, NO₂, NR³R⁴, SR³, C₁-C₄ alkyl, C₁-C₄ haloalkyl or C₁-C₄ heteroalkyl;
R^{13A} is NHR¹ or heteroaryl, wherein the heteroaryl is optionally substituted with F, Cl, Br, I, CN, NO₂, NR³R⁴, SR³, SOR³, SO₂R³, C₁-C₄ alkyl, C₁-C₄ haloalkyl or C₁-C₄ heteroalkyl;
R¹⁴ is selected from the group of F, Br, Cl, I, CF₃, CHF₂, CH₂F, CF₂Cl, and CF₂OR³;
R¹⁵ is selected from the group of F, Br, Cl, I, CN, C₁-C₄ alkyl, C₁-C₄ haloalkyl, C₁-C₄ heteroalkyl, OR¹⁶, NR¹⁶R⁴, SR¹⁶, CH₂R¹⁶, COR³, CO₂R³, CONR³R⁴, SOR³, and SO₂R³;
R¹⁶ is selected from the group of hydrogen, C₁-C₈ alkyl, C₁-C₈ haloalkyl, C₁-C₈ heteroalkyl, CH₃R^{3A}, aryl, heteroaryl COR¹⁷, CO₂R¹⁷, and CONR¹⁷R¹⁷;
R¹⁷ is selected from the group of hydrogen, C₁-C₄ alkyl, C₁-C₄ haloalkyl and C₁-C₄ heteroalkyl;
R¹⁸ and R¹⁹ are each independently selected from the group of hydrogen, C₁-C₆ alkyl, C₁-C₆ haloalkyl and C₁-C₆ heteroalkyl; or
R¹⁸ and R¹⁹ are taken together to form a three- to seven-membered ring;
R²⁰ is aryl or heteroaryl, wherein the aryl or heteroaryl are optionally substituted with F, Cl, Br, CN, OR³, SR³, SOR³, SO₂R³, NO₂, NR³R³, C₁-C₄ alkyl, C₁-C₄ haloalkyl or C₁-C₄ heteroalkyl;
R²¹ is selected from the group of CR³R⁴CONR³R⁴, Cₙ(R³)₂ₙOR³, SOR³, SO₂R³, C₂-C₈ alkyl, C₂-C₈ haloalkyl, and C₂-C₈ heteroalkyl;
R²² and R²³ are each independently selected from the group of hydrogen, C₁-C₆ alkyl, C₁-C₆ haloalkyl and C₁-C₆ heteroalkyl; or
R²² and R²³ are taken together to form a three- to seven-membered ring;
R²⁴ is hydrogen or OR³;
R²⁵ through R³⁰ are each independently selected from the group of hydrogen, F, Cl, Br, I, OR³, NR³R⁴, SR³, SOR³, SO₂R³, C₁-C₆ alkyl, C₁-C₆ haloalkyl, C₁-C₆ heteroalkyl, C₂-C₆ alkynyl and C₂-C₆ alkenyl, wherein the alkyl, haloalkyl, heteroalkyl, alkynyl, and alkenyl are optionally substituted with F, Cl, Br, I, OR³, NR³R⁴, C₁-C₄ alkyl, C₁-C₄ haloalkyl, C₁-C₄ heteroalkyl, aryl or heteroaryl, and wherein the aryl and heteroaryl are optionally substituted with F, Cl, Br, I, CN, NO₂, OH, OCH₃, CF₃ or C₁-C₆ alkyl; or
Any two of R²⁵ R²⁶, R²⁷, R²⁸, R²⁹ and R³⁰ are taken together to form a three to seven-membered alkyl or alkenyl or heteroalkyl ring; or
any four of R²⁵ R²⁶, R²⁷, R²⁸, R²⁹ and R³⁰ are taken together to form a fused aromatic ring;
Q is O or S;
U is selected from the group of V, OCR²²R²³, SCR²²R²³, NR³CR²²R²³, and CR³R⁴CR²²R²³;
V is selected from the group of 0, NR³, CR²²R²³, CR³R⁴O, and CR³R⁴S ;
W is selected from the group of O, S, NR³, and CR³R⁴; X is selected from the group of O, S and NR¹⁶;
Y is selected from the group of O, S, NR³, NOR³ and CR³R⁴; Z is selected from the group of O, S, NR³, C=O, and CR²⁵R²⁶;
or
Z is two hydrogens;
n is 1, 2 or 3; and
m is 1 to 5,
and wherein, in the formulas (1) to (5),
L¹ is hydrogen, F, Cl, Br, I, NO₂, OL²⁰, NL²¹L²², SL²⁰, a C₁-C₄ alkyl or perhaloalkyl, or is an optionally substituted allyl, arylmethyl, alkynyl, alkenyl, aryl or heteroaryl, wherein L²¹ is hydrogen, a C₁-C₆ alkyl or perfluoroalkyl, aryl, heteroaryl, optionally substituted allyl or arylmethyl, SO₂L²³ or S(O)L²³, wherein L²³ is hydrogen, a C₁-C₆ alkyl or perfluoroalkyl, aryl, heteroaryl, or optionally substituted allyl or arylmethyl, L²⁰ is hydrogen, a C₁-C₆ alkyl or perfluoroalkyl, aryl, heteroaryl, optionally substituted allyl or arylmethyl, and L²² is hydrogen, a C₁-C₄ alkyl or perfluoroalkyl, aryl, heteroaryl, optionally substituted allyl or arylmethyl, OL²⁰ or NHL²¹;
L² is hydrogen, F, Br, Cl, a C₁-C₄ alkyl or perhaloalkyl, aryl, heteroaryl, CF₃, CF₂H, CFH₂, CF₂OL²⁰, CH₂OL²⁰, or OL²⁰, wherein L²⁰ has the same definition given above;
L³ is hydrogen, a C₁-C₄ alkyl, F, Cl, Br, I, OL²⁰, NL²¹L²² or SL²⁰, wherein L²⁰ through L²² have the definition given above;
L⁴ and L⁵ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁴ and L⁵ taken together can form a three- to seven-membered ring optionally substituted with hydrogen, F, Cl, Br, OL²⁰ or NL²¹L²², wherein L²⁰ through L²² have the definitions given above;
L⁶ and L⁷ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L⁶ and L' taken together can form a three- to seven-membered ring optionally substituted with hydrogen, F, Cl, Br, OL²⁰ or NL²¹L²², wherein L²⁰ through L²² have the definitions given above;
L⁸ is hydrogen, a C₁-C₁₂ alkyl or perfluoroalkyl, hydroxymethyl, aryl, heteroaryl or optionally substituted allyl, arylmethyl, alkynyl or alkenyl;
L⁹ through L¹⁸ each independently are hydrogen, a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or any two of L⁹ through L¹⁸ taken together can form a three- to seven-membered ring optionally substituted with hydrogen, F, Cl, Br, OL²⁰ or NL²¹L²², wherein L²⁰ through L²² have the definitions given above;
L¹⁹ is F, NO₂ or SL²⁰, wherein L²⁰ has the definition given above;
L²⁴ is hydrogen, a C₁-C₄ alkyl, F, Cl, Br, I, NO₂, OL²⁰, NL²¹L²² or SL²⁰, wherein L²⁰ through L²² have the definitions given above;
L²⁵ is hydrogen, a C₁-C₁₂ alkyl or perfluoroalkyl, hydroxymethyl, aryl, heteroaryl or optionally substituted allyl, arylmethyl, alkynyl or alkenyl, or L²⁵ and L⁸ taken together can form a three- to seven-membered ring optionally substituted with hydrogen, F, Cl, Br, OL²⁰ or NL²¹L²², wherein L²⁰ through L²² have the definitions given above;
L²⁶ is hydrogen, a C₁-C₆ alkyl or perfluoroalkyl, NO₂, OL²⁰, C(O)L²⁰, C(O)OL²⁰, C(O)NL²¹, L²², or an optionally substituted aryl, heteroaryl, allyl or arylmethyl, wherein L²⁰ through L²² have the definitions given above;
L²⁷ and L²⁸ each independently are hydrogen, F, Cl, Br,
I, OL²⁰, NL²¹L²², a C₁-C₄ alkyl or perfluoroalkyl, heteroaryl, optionally substituted allyl, arylmethyl, alkynyl or alkenyl, or an aryl optionally substituted with hydrogen, F, Cl, Br, OL²⁰ or NL²¹L²², or L²⁷ and L²⁸ taken together can form a three- to seven-membered ring optionally substituted with hydrogen, F, Cl, Br, OL²⁰ or NL²¹L²², wherein L²⁰ through L²² have the definitions given above;
m is 0 or 1;
n is 0 or 1;
o is 0 or 1;
Y is 0 or S;
Z is O, S, NH, NL²² or NCOL²², wherein L²² has the definition given above; and
any two of L⁴ through L⁸, L²⁵ and L²⁸ taken together can form a three- to seven-membered ring optionally substituted with hydrogen, F, Cl, Br, OL²⁰ or NL²¹L²², wherein L²⁰ through L²² have the definitions given above,
and wherein, in the formulas (A) to (F),
r¹ is hydrogen, F, Cl, Br, I, NO₂, Or⁹, Nr¹⁰r¹¹, S(O)mr⁹, C₁-C₈ alkyl, C₃-C₈ cycloalkyl, C₁-C₈ heteroalkyl, C₁-C₈ haloalkyl, C₁-C₈ aryl, C₁-C₈ arylalkyl, C₁-C₈ heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r² is hydrogen, F, Cl, Br, I, CF₃, CF₂Cl, CF₂H, CFH₂, CF₂Or⁹, CH₂Or⁹, Or⁹, S(O)mr⁹, Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted;
r³ is hydrogen, F, Cl, Br, I, Or⁹, S(O)mr⁹, Nr¹⁰r¹¹, or C₁-C₆ alkyl, C₁-C₆ heteroalkyl, or C₁-C₆ haloalkyl, wherein the alkyl, heteroalkyl, and haloalkyl groups may be optionally substituted;
r⁴ and r⁵ each independently are hydrogen, or⁹, S(O)mr⁹, Nr¹⁰r¹¹, C(Y)Or¹¹, C(Y)Nr¹⁰r¹¹, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁴ and r⁵ taken together can form a saturated or unsaturated three- to seven-membered ring that may be optionally substituted;
r⁶ and r⁷ each independently are hydrogen, C₁-C₈ alkyl, C₃-C₈ cycloalkyl, C₁-C₈ heteroalkyl, C₁-C₈ haloalkyl, aryl, arylalkyl, heteroaryl, C₂-C₈ alkynyl, or C₂-C₈ alkenyl, wherein the alkyl, cycloalkyl, heteroalkyl, haloalkyl, aryl, arylalkyl, heteroaryl, alkynyl, and alkenyl groups may be optionally substituted; or
r⁶ and r⁷ taken together can form a saturated or unsaturated three- to seven-membered ring that may be optionally substituted; or
r⁶ and r⁵ taken together can form a saturated or unsaturated three- to seven-membered ring that may be optionally substituted;
r⁸ is hydrogen, C₁-C₄ alkyl, C₁-C₄ heteroalkyl, C₁-C₄ haloalkyl, F, Cl, Br, I, NO₂, Or⁹, Nr¹⁰r¹¹ or S(O)mr⁹, wherein the alkyl, heteroalkyl, and haloalkyl groups may be optionally substituted;
r⁹ is hydrogen, C₁-C₆ alkyl, C₁-C₆ heteroalkyl, C₁-C₆ haloalkyl, aryl, heteroaryl, arylalkyl, C₂-C₄ alkynyl or C₂-C₈ alkenyl, wherein the alkyl, heteroalkyl, haloalkyl, aryl, arylalkyl, heteroaryl, alkenyl and alkynyl groups may be optionally substituted;
r¹⁰ is hydrogen, C₁-C₆ alkyl, C₁-C₆ heteroalkyl, C₁-C₆ haloalkyl, C(Y)r¹², C(Y)Or¹², aryl, heteroaryl, C₂-C₄ alkynyl, C₂-C₄ alkenyl, arylalkyl, SO₂r¹² or S(O)r¹², wherein the alkyl, heteroalkyl, haloalkyl, aryl, arylalkyl, heteroaryl, alkynyl, and alkenyl groups may be optionally substituted;
r¹¹ is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, arylalkyl, heteroaryl, alkynyl and alkenyl groups may be optionally substituted;
r¹² is hydrogen, C₁-C₆ alkyl, C₁-C₆ heteroalkyl, C₁-C₆ haloalkyl, aryl, heteroaryl, arylalkyl, C₂-C₄ alkynyl, or C₂-C₈ alkenyl, wherein the alkyl, heteroalkyl, haloalkyl, aryl, heteroaryl, arylalkyl, alkynyl and alkenyl groups may be optionally substituted;
r¹³ is hydrogen, C₁-C₈ alkyl, C₃-C₈ cycloalkyl, C₁-C₈ heteroalkyl, C₁-C₈ haloalkyl, aryl, heteroaryl, arylalkyl, heteroarylalkyl, C₂-C₄ alkynyl, or C₂-C₈ alkenyl, wherein the alkyl, cycloalkyl, heteroalkyl, haloalkyl, aryl, arylalkyl, heteroaryl, heteroarylalkyl, alkynyl and alkenyl groups may be optionally substituted; or
r¹³ and r⁴ taken together can form a saturated or unsaturated three- to seven-membered ring that may be optionally substituted;
r¹³ and any two of r⁴ through r⁷ taken together can form a saturated or unsaturated three- to seven-membered ring that may be optionally substituted;
r¹⁴ and r¹⁵ each independently are hydrogen, C₁-C₈ alkyl, C₃-C₈ cycloalkyl, C₁-C₈ heteroalkyl, C₁-C₈ haloalkyl, aryl, heteroaryl, arylalkyl, C₂-C₈ alkynyl or C₂-C₈ alkenyl, wherein the alkyl, cycloalkyl, heteroalkyl, haloalkyl, aryl, heteroaryl, arylalkyl, alkynyl and alkenyl groups may be optionally substituted;
r^{A} is hydrogen, F, Br, Cl, I, CN, C₁-C₆ alkyl, C₁-C₆ haloalkyl, C₁-C₆ heteroalkyl, Or¹⁶, Nr¹⁶r¹⁷, Sr¹⁶, CH₂r¹⁶, cor¹⁷, CO₂r¹⁷, CONr¹⁷r¹⁷, SOr¹⁷ or SO₂r¹⁷, wherein the alkyl, haloalkyl, and heteroalkyl groups may be optionally substituted;
r¹⁶ is hydrogen, C₁-C₈ alkyl, C₁-C₈ haloalkyl, C₁-C₈ heteroalkyl, COr¹⁷, CO₂r¹⁷, CONr¹⁷r¹⁷, C₂-C₈ alkynyl, C₂-C₈ alkenyl, aryl or heteroaryl, wherein the alkyl, heteroalkyl, haloalkyl, aryl, heteroaryl, alkynyl and alkenyl groups may be optionally substituted;
r¹⁷ is hydrogen, C₁-C₄alkyl, C₁-C₄haloalkyl or C₁-C₄heteroalkyl, wherein the alkyl, heteroalkyl, and haloalkyl groups may be optionally substituted;
m is 0, 1 or 2;
n is 1 or 2;
V is O, S or Cr¹⁴r¹⁵;
W is O, S, NH, Nr¹³, NC(Y)r¹¹, or NSO₂r¹¹;
X and Z each independently are O, S(O)m, NH, Nr¹¹, NC(Y)r¹¹, NSO₂r¹² or NS (O)r¹²; and
Y is O or S, and
wherein, in the formula (α),
u¹ is hydrogen, F, Cl, or C₁-C₃ aliphatic;
u² is selected from the group of hydrogen, F, Cl, Br, C₁-C₄ aliphatic, C₁-C₄ haloaliphatic, and C₁-C₄ heteroaliphatic;
u³ and u⁴ each independently is selected from the group of hydrogen, C₁-C₄ aliphatic, C₁-C₄ haloaliphatic, C₁-C₄ heteroaliphatic, optionally substituted aryl and heteroaryl;
u⁵ and u⁶ each independently is selected from the group of hydrogen, F, Cl, Ou¹⁰, C₁-C₄ aliphatic, C₁-C₄ haloaliphatic, and C₁-C₄ heteroaliphatic;
u⁷ and u⁸ each independently is selected from the group of hydrogen, F, Cl, C₁-C₄ aliphatic, C₁-C₄ haloaliphatic, and C₁-C₄ heteroaliphatic; or
u⁷ and u⁸ taken together form a carbonyl group;
u⁹ is selected from the group of halogen, Ou¹⁰, Su¹⁰, Nu¹⁰, u¹¹, C₁-C₄ haloaliphatic, C₁-C₄ heteroaliphatic, and C₁-C₄ heterohaloaliphatic; and
u¹⁰ and u¹¹ each independently is selected from the group of hydrogen, C₁-C₄ aliphatic, phenyl, and benzyl; and
n is 0 or 1.

2. An amyloid-β protein accumulation-inhibitory agent for inhibiting amyloid-β protein accumulation comprising the compound according to Claim 1 or a pharmaceutically acceptable salt thereof.

3. Use of the compound according to Claim 1 or a pharmaceutically acceptable salt thereof for inhibiting amyloid-β protein accumulation.

4. A method for inhibiting amyloid-β protein accumulation, comprising a step of administering an effective amount of the compound according to Claim 1 or a pharmaceutically acceptable salt thereof to a mammal which may be diagnosed with an amyloid-β protein-related disease.
